(19) Europäisches Patentamt
European Patent Office
Office européen des brevets

(11) **EP 4 071 172 A1**

(12) **EUROPEAN PATENT APPLICATION**
published in accordance with Art. 153(4) EPC

(43) Date of publication:
**12.10.2022 Bulletin 2022/41**

(51) International Patent Classification (IPC):
**C07K 16/28** $^{(2006.01)}$   **A61P 35/00** $^{(2006.01)}$
**A61K 39/00** $^{(2006.01)}$

(21) Application number: **20907118.2**

(52) Cooperative Patent Classification (CPC):
**A61K 39/00; A61P 35/00; C07K 16/28**

(22) Date of filing: **22.12.2020**

(86) International application number:
**PCT/KR2020/018931**

(87) International publication number:
**WO 2021/133036 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.12.2019 KR 20190173414**
**22.05.2020 KR 20200061907**

(71) Applicant: **Lg Chem, Ltd.**
**Seoul 07336 (KR)**

(72) Inventors:
• **CHOI, Yoon Aa**
**Daejeon 34122 (KR)**
• **KIM, Jung A**
**Daejeon 34122 (KR)**
• **JUNG, Saem**
**Daejeon 34122 (KR)**
• **LEE, Ji Hyun**
**Daejeon 34122 (KR)**
• **NA, Kyubong**
**Daejeon 34122 (KR)**
• **KIM, Yeonchul**
**Daejeon 34122 (KR)**
• **KIM, Han Byul**
**Daejeon 34122 (KR)**

(74) Representative: **Hoffmann Eitle**
**Patent- und Rechtsanwälte PartmbB**
**Arabellastraße 30**
**81925 München (DE)**

(54) **ANTI-LILRB1 ANTIBODY AND USES THEREOF**

(57)    The present invention relates to an anti-LILRB1 antibody having increased specificity for LILRB1, and to uses thereof. Specifically, provided are an anti-LILRB1 antibody or antigen-binding fragment thereof, and uses thereof in treating cancer.

【Figure 6】

HCT-116 cell / THP 1 cell derived macrophage / NOG Mouse / anti-LILRB-1 / Intraperitoneal Administration for 3-weeks

Data are presented as mean and error SEM of 6 mice per group.*P < 0.05, **P < 0.01

EP 4 071 172 A1

**Description**

[Technical Field]

Cross-Reference to Related Applications

This application claims the benefits of KR 10-2019-0173414 filed on December 23, 2019 and KR 10-2020-0061907 filed on May 22, 2020 with the Korean Intellectual Property Office, the entire disclosure of which is herein incorporated by reference.

The disclosure relates to an anti-LILRB1 antibody and uses thereof. More specifically, an anti-LILRB1 antibody or an antigen-binding fragment thereof, and a use thereof for cancer therapy are provided.

[Background Art]

Leukocyte immunoglobulin-like receptor subfamily B member 1 (LILRB1; also known as ILT2, CD85j, or LIR-1) is an inhibitory receptor, which is expressed in cells such as B cells, T cells, NK cells, dendritic cells, macrophages, and other immune cells. LILRB1 participates in a signal transduction mechanism of inhibiting activities of immune cells by binding classical and non-classical MHC class I.

Meanwhile, it has been reported that various cancer cells overexpress MHC class I such as HLA-G for immune evasion. It has been expected that blocking the binding of LILRB1 to MHC Class I allows recovery of the inhibited activities of immune cells, thereby exhibiting anti-cancer effects.

Therefore, it is required to develop novel agent binding to LILRB1 and blocking the binding of LILRB1 to MHC Class I and/or the interaction between LILRB1 and MHC Class I.

[Disclosure]

[Technical Problem]

This disclosure provides antibodies, which bind to LILRB1, act on LILRB1-expressing immune cells, regulate activities of the immune cells, and exhibit anti-cancer effects, and uses thereof for cancer therapies.

An embodiment provides an anti-LILRB1 antibody, which binds to LILRB1, or an antigen-binding fragment thereof. The anti-LILRB1 antibody or an antigen-binding fragment thereof may have an activity to block the binding of LILRB1 to MHC Class I and/or blocking the interaction between LILRB1 and MHC Class I. In addition, the anti-LILRB1 antibody or an antigen-binding fragment thereof may have an activity to inhibit immune evasion of cancer cells. Furthermore, the anti-LILRB1 antibody or an antigen-binding fragment thereof may have an anti-cancer effect. The anti-cancer effect may be against a cancer cell expressing or overexpressing MHC Class I on its cell surface.

Another embodiment provides a pharmaceutical composition for treatment and/or prevention of a cancer, the composition comprising the anti-LILRB1 antibody or an antigen-binding fragment thereof as an active ingredient.

Another embodiment provides a pharmaceutical composition for inhibition of binding of LILRB1 to MHC Class I and/or blocking the interaction between LILRB1 and MHC Class I, the composition comprising the anti-LILRB1 antibody or an antigen-binding fragment thereof as an active ingredient.

Another embodiment provides a pharmaceutical composition for inhibiting immune evasion of cancer cell, the composition comprising the anti-LILRB1 antibody or an antigen-binding fragment thereof as an active ingredient.

[Technical Solution]

An embodiment provides an anti-LILRB1 antibody, which binds to LILRB1, or an antigen-binding fragment thereof. The anti-LILRB1 antibody or an antigen-binding fragment thereof may have an activity to block the binding of LILRB1 to MHC Class I and/or blocking the interaction between LILRB1 and MHC Class I. In addition, the anti-LILRB1 antibody or an antigen-binding fragment thereof may have an activity to inhibit immune evasion of cancer cells. In addition, the anti-LILRB1 antibody or an antigen-binding fragment thereof may have an anti-cancer effect.

The anti-LILRB1 antibody or an antigen-binding fragment thereof may comprise the following complementarity determining regions (CDRs):

(1) based on the CDR definition according to Kabat numbering (Kabat, E.A., Wu, T.T., Perry, H., Gottesman, K. and Foeller, C. (1991) Sequences of Proteins of Immunological Interest, Fifth Edition. NIH Publication No. 91-3242; http://www.abysis.org/),

a CDR-L1 comprising an amino acid sequence of SEQ ID NO: 1, 7, 13, 19, 25, 31, 37, 43, 49, 55, 61, 67, 73, 79, 85, 91, 97, 103, 109, or 115,
a CDR-L2 comprising an amino acid sequence of SEQ ID NO: 2, 8, 14, 20, 26, 32, 38, 44, 50, 56, 62, 68, 74, 80, 86, 92, 98, 104, 110, or 116,
a CDR-L3 comprising an amino acid sequence of SEQ ID NO: 3, 9, 15, 21, 27, 33, 39, 45, 51, 57, 63, 69, 75, 81, 87, 93, 99, 105, 111, or 117,
a CDR-H1 comprising an amino acid sequence of SEQ ID NO: 4, 10, 16, 22, 28, 34, 40, 46, 52, 58, 64, 70, 76, 82, 88, 94, 100, 106, 112, or 118,
a CDR-H2 comprising an amino acid sequence of SEQ ID NO: 5, 11, 17, 23, 29, 35, 41, 47, 53, 59, 65, 71, 77, 83, 89, 95, 101, 107, 113, or 119, and
a CDR-H3 comprising an amino acid sequence of SEQ ID NO: 6, 12, 18, 24, 30, 36, 42, 48, 54, 60, 66, 72, 78, 84, 90, 96, 102, 108, 114, or 120; or

(2) based on the CDR definition according to IMGT numbering (http://www.imgt.org/),

a CDR-L1 comprising an amino acid sequence of SEQ ID NO: 121, 126, 131, 136, 141, 146, 151, 156, 161, 166, 171, 176, 181, 186, 191, 196, 201, 206, 211, or 216,
a CDR-L2 comprising an amino acid sequence of SEQ ID NO: 122, 127, 132, 137, 142, 147, 152, 157, 162, 167, 172, 177, 182, 187, 192, 197, 202, 207, 212, or 217,
a CDR-L3 comprising an amino acid sequence of SEQ ID NO: 3, 9, 15, 21, 27, 33, 39, 45, 51, 57, 63, 69, 75, 81, 87, 93, 99, 105, 111, or 117,
a CDR-H1 comprising an amino acid sequence of SEQ ID NO: 123, 128, 133, 138, 143, 148, 153, 158, 163, 168, 173, 178, 183, 188, 193, 198, 203, 208, 213, or 218,
a CDR-H2 comprising an amino acid sequence of SEQ ID NO: 124, 129, 134, 139, 144, 149, 154, 159, 164, 169, 174, 179, 184, 189, 194, 199, 204, 209, 214, or 219, and
a CDR-H3 comprising an amino acid sequence of SEQ ID NO: 125, 130, 135, 140, 145, 150, 155, 160, 165, 170, 175, 180, 185, 190, 195, 200, 205, 210, 215, or 220.

In a specific embodiment, combinations of 6 CDRs (CDR-L1, CDR-L2, CDR-L3, CDR-H1, CDR-H2, and CDR-H3) that can be comprised in the anti-LILRB1 antibody or an antigen-binding fragment thereof provided in this disclosure are illustrated in Table 1:

[Table 1]

|  | CDR | Amino Acid Sequence (N→C) (Kabat) | SEQ ID NO | Amino Acid Sequence (N→C) (IMGT) | SEQ ID NO |
|---|---|---|---|---|---|
| E3/E3.1 | CDR-L1 | QGDSLRNFYAS | 1 | SLRNFY | 121 |
|  | CDR-L2 | GKNNRPS | 2 | GKN | 122 |
|  | CDR-L3 | NSRDSSGSHLTGV | 3 | NSRDSSGSHLTGV | 3 |
|  | CDR-H1 | SYAMS | 4 | GFTFSSYA | 123 |
|  | CDR-H2 | AISGSGGSTYYADSVKG | 5 | ISGSGGST | 124 |
|  | CDR-H3 | DTYYYGSGRSNAFDI | 6 | ARDTYYYGSGRSNAFDI | 125 |

(continued)

| | CDR | Amino Acid Sequence (N→C) (Kabat) | SEQ ID NO | Amino Acid Sequence (N→C) (IMGT) | SEQ ID NO |
|---|---|---|---|---|---|
| B3 | CDR-L1 | QASQDISNYLN | 7 | QDISNY | 126 |
| | CDR-L2 | DASNLET | 8 | DAS | 127 |
| | CDR-L3 | QQYDNLP | 9 | QQYDNLP | 9 |
| | CDR-H1 | DYAMH | 10 | GFTFDDYA | 128 |
| | CDR-H2 | GISWNSGSIGYADSVKG | 11 | ISWNSGSI | 129 |
| | CDR-H3 | VGDSSGWSDAFDI | 12 | ARVGDSSGWSDAFDI | 130 |
| A10 | CDR-L1 | RASQSVSSNLA | 13 | QSVSSN | 131 |
| | CDR-L2 | GASTRAT | 14 | GAS | 132 |
| | CDR-L3 | QQYGSSPRMYT | 15 | QQYGSSPRMYT | 15 |
| | CDR-H1 | SYAIS | 16 | GGTFSSYA | 133 |
| | CDR-H2 | GIIPIFGTANYAQKFQG | 17 | IIPIFGTA | 134 |
| | CDR-H3 | GGLGELDNWFDP | 18 | ARGGLGELDNWFDP | 135 |
| G1 | CDR-L1 | SGYKLGDRYVS | 19 | KLGDRY | 136 |
| | CDR-L2 | KDSQRPS | 20 | KDS | 137 |
| | CDR-L3 | QAWDSGTGV | 21 | QAWDSGTGV | 21 |
| | CDR-H1 | SYGIS | 22 | GGTFSSYG | 138 |
| | CDR-H2 | WISAYNGNTNYAQELQG | 23 | ISAYNGNT | 139 |
| | CDR-H3 | VGVAGKLDY | 24 | ARVGVAGKLDY | 140 |

(continued)

| | CDR | Amino Acid Sequence (N→C) (Kabat) | SEQ ID NO | Amino Acid Sequence (N→C) (IMGT) | SEQ ID NO |
|---|---|---|---|---|---|
| G9 | CDR-L1 | TGSSSDVGGYNYVS | 25 | SSDVGGYNY | 141 |
| | CDR-L2 | DVSNRPS | 26 | DVS | 142 |
| | CDR-L3 | SSYTGSSTLDVL | 27 | SSYTGSSTLDVL | 27 |
| | CDR-H1 | SYWIG | 28 | GYSFTSYW | 143 |
| | CDR-H2 | IIYPGDSDTRYSPSFQG | 29 | IYPGDSDT | 144 |
| | CDR-H3 | QYYDGGYYMDV | 30 | ASQYYDGGYYMDV | 145 |
| H2 | CDR-L1 | QGDSLRNYYAS | 31 | SLRNYY | 146 |
| | CDR-L2 | GNNKRPS | 32 | GNN | 147 |
| | CDR-L3 | NSLDSTYNHPI | 33 | NSLDSTYNHPI | 33 |
| | CDR-H1 | SYDIH | 34 | GYTFTSYD | 148 |
| | CDR-H2 | WISAYNGNTNYAQKLQG | 35 | ISAYNGNT | 149 |
| | CDR-H3 | DGGDAFDI | 36 | ARDGGDAFDI | 150 |
| H11 | CDR-L1 | QGDSLRSYYAS | 37 | SLRSYY | 151 |
| | CDR-L2 | GRNNRPS | 38 | GRN | 152 |
| | CDR-L3 | KSRDSSGNHYV | 39 | KSRDSSGNHYV | 39 |
| | CDR-H1 | SYYMH | 40 | GYTFTSYY | 153 |
| | CDR-H2 | IINPSGGSTSYAQKFQG | 41 | INPSGGST | 154 |
| | CDR-H3 | DAGSSSDY | 42 | ARDAGSSSDY | 155 |

(continued)

| | CDR | Amino Acid Sequence (N→C) (Kabat) | SEQ ID NO | Amino Acid Sequence (N→C) (IMGT) | SEQ ID NO |
|---|---|---|---|---|---|
| F12 | CDR-L1 | AGTSSDIGDYDYVS | 43 | SSDIGDYDY | 156 |
| | CDR-L2 | DVSRRPS | 44 | DVS | 157 |
| | CDR-L3 | ASYTSSSVVV | 45 | ASYTSSSVVV | 45 |
| | CDR-H1 | SYWIG | 46 | GYSFTSYW | 158 |
| | CDR-H2 | IIYPGDSDTRYSPSFQG | 47 | IYPGDSDT | 159 |
| | CDR-H3 | QYYDGGYYMDV | 48 | ASQYYDGGYYMDV | 160 |
| B9 | CDR-L1 | RASQSISRYLN | 49 | QSISRY | 161 |
| | CDR-L2 | GASSLQS | 50 | GAS | 162 |
| | CDR-L3 | QQAYGFPLT | 51 | QQAYGFPLT | 51 |
| | CDR-H1 | SYAIS | 52 | GGTFSSYA | 163 |
| | CDR-H2 | GIIPIFGTANYAQKFQG | 53 | IIPIFGTA | 164 |
| | CDR-H3 | GEIAVAQNWDYYGMDV | 54 | ARGEIAVAQNWDYYGMDV | 165 |
| G11 | CDR-L1 | TGTSSDVGGYNYVS | 55 | SSDVGGYNY | 166 |
| | CDR-L2 | DVSKRPS | 56 | DVS | 167 |
| | CDR-L3 | SSYSSSSTLVV | 57 | SSYSSSSTLVV | 57 |
| | CDR-H1 | SYWIG | 58 | GYSFTSYW | 168 |
| | CDR-H2 | IIYPGDSDTRYSPSFQG | 59 | IYPGDSDT | 169 |
| | CDR-H3 | QYYDGGYYMDV | 60 | ASQYYDGGYYMDV | 170 |

(continued)

| | CDR | Amino Acid Sequence (N→C) (Kabat) | SEQ ID NO | Amino Acid Sequence (N→C) (IMGT) | SEQ ID NO |
|---|---|---|---|---|---|
| G6 | CDR-L1 | QGDSLRRYYAT | 61 | SLRRYY | 171 |
| | CDR-L2 | GQNYRPS | 62 | GQN | 172 |
| | CDR-L3 | NSRDSSGNHVV | 63 | NSRDSSGNHVV | 63 |
| | CDR-H1 | SYYMH | 64 | GYTFTSYY | 173 |
| | CDR-H2 | GIIPIFGTANYAQKFQG | 65 | IIPIFGTA | 174 |
| | CDR-H3 | GWGYSSSFDY | 66 | ARGWGYSSSFDY | 175 |
| F11 | CDR-L1 | SGSSSNIGTNTVN | 67 | SSNIGTNT | 176 |
| | CDR-L2 | SNDQRPS | 68 | SND | 177 |
| | CDR-L3 | ETWDDSLKGPV | 69 | ETWDDSLKGPV | 69 |
| | CDR-H1 | SYAMS | 70 | GFTFSSYA | 178 |
| | CDR-H2 | TISGSGDSTYYADSVKG | 71 | ISGSGDST | 179 |
| | CDR-H3 | EWELGDAFDI | 72 | AREWELGDAFDI | 180 |
| D3 | CDR-L1 | RASQSISSYLN | 73 | QSISSY | 181 |
| | CDR-L2 | AASSLQS | 74 | AAS | 182 |
| | CDR-L3 | QQSYSTRWT | 75 | QQSYSTRWT | 75 |
| | CDR-H1 | SYAMS | 76 | GSTFSSYA | 183 |
| | CDR-H2 | AISGSGGSTYYADSVKG | 77 | ISGSGGST | 184 |
| | CDR-H3 | DRGSYGYYYGMDV | 78 | AKDRGSYGYYYGMDV | 185 |

(continued)

|  | CDR | Amino Acid Sequence (N→C) (Kabat) | SEQ ID NO | Amino Acid Sequence (N→C) (IMGT) | SEQ ID NO |
|---|---|---|---|---|---|
| B12 | CDR-L1 | RASQSISSYLN | 79 | QSISSY | 186 |
|  | CDR-L2 | AASSLQS | 80 | AAS | 187 |
|  | CDR-L3 | QQSYSTLRT | 81 | QQSYSTLRT | 81 |
|  | CDR-H1 | GYYMH | 82 | GYTFTGYY | 188 |
|  | CDR-H2 | WINPNSGGTNYAQKFQG | 83 | INPNSGGT | 189 |
|  | CDR-H3 | AGASIVGATALDY | 84 | TRAGASIVGATALDY | 190 |
| E4 | CDR-L1 | TRSSGSIASNYVQ | 85 | SGSIASNY | 191 |
|  | CDR-L2 | EDNQRPS | 86 | EDN | 192 |
|  | CDR-L3 | QSYDTGNRNYV | 87 | QSYDTGNRNYV | 87 |
|  | CDR-H1 | SYTIS | 88 | GGTFSSYT | 193 |
|  | CDR-H2 | RIIPILGIANYAQKFQG | 89 | IIPILGIA | 194 |
|  | CDR-H3 | GPSLNYAGYFDN | 90 | VRGPSLNYAGYFDN | 195 |
| E12 | CDR-L1 | QGDSLRSYYAS | 91 | SLRSYY | 196 |
|  | CDR-L2 | GKEKRPS | 92 | GKE | 197 |
|  | CDR-L3 | NSRGSTTDYMV | 93 | NSRGSTTDYMV | 93 |
|  | CDR-H1 | SYAMH | 94 | GFTFSSYA | 198 |
|  | CDR-H2 | VISYDGSNKYYADSVKG | 95 | ISYDGSNK | 199 |
|  | CDR-H3 | ERGSGMDV | 96 | ARERGSGMDV | 200 |

(continued)

| | CDR | Amino Acid Sequence (N→C) (Kabat) | SEQ ID NO | Amino Acid Sequence (N→C) (IMGT) | SEQ ID NO |
|---|---|---|---|---|---|
| D1 | CDR-L1 | KASQDIDDDMN | 97 | QDIDDD | 201 |
| | CDR-L2 | EASTLVP | 98 | EAS | 202 |
| | CDR-L3 | LQHDKFPYT | 99 | LQHDKFPYT | 99 |
| | CDR-H1 | SYGIS | 100 | GYTFTSYG | 203 |
| | CDR-H2 | WINPNSGGTNYAQKFQG | 101 | INPNSGGT | 204 |
| | CDR-H3 | RGVDEGDY | 102 | ASRGVDEGDY | 205 |
| E6 | CDR-L1 | TGSSGNIASNYVQ | 103 | SGNIASNY | 206 |
| | CDR-L2 | RDDQRPS | 104 | RDD | 207 |
| | CDR-L3 | QSYDSSSWV | 105 | QSYDSSSWV | 105 |
| | CDR-H1 | TYDIT | 106 | GYTFTTYD | 208 |
| | CDR-H2 | WMNPNSGNSRSAQKFQG | 107 | MNPNSGNS | 209 |
| | CDR-H3 | GDYSGVVLTATALDY | 108 | ATGDYSGVVLTATALDY | 210 |
| E9 | CDR-L1 | SGSSSNIGNNYVY | 109 | SSNIGNNY | 211 |
| | CDR-L2 | RNNQRPS | 110 | RNN | 212 |
| | CDR-L3 | AAWDDSLSGWV | 111 | AAWDDSLSGWV | 111 |
| | CDR-H1 | SYGMH | 112 | GFTFSSYG | 213 |
| | CDR-H2 | NIKQDGSEKYYVDSVKG | 113 | IKQDGSEK | 214 |
| | CDR-H3 | EDRIAAAGMRELDY | 114 | AREDRIAAAGMRELDY | 215 |

(continued)

| | CDR | Amino Acid Sequence (N→C) (Kabat) | SEQ ID NO | Amino Acid Sequence (N→C) (IMGT) | SEQ ID NO |
|---|---|---|---|---|---|
| A11 | CDR-L1 | RSSQSLLHSNGYNYLD | 115 | QSLLHSNGYNY | 216 |
| | CDR-L2 | LGSNRAS | 116 | LGS | 217 |
| | CDR-L3 | MQGTHWPPYT | 117 | MQGTHWPPYT | 117 |
| | CDR-H1 | SYAMT | 118 | GFSFTSYA | 218 |
| | CDR-H2 | GISSDGTTTTYADSVRG | 119 | ISSDGTTT | 219 |
| | CDR-H3 | DQLLGWDALNV | 120 | ARDQLLGWDALNV | 220 |

In an embodiment, the anti-LILRB1 antibody or an antigen-binding fragment thereof may comprise:

a light chain variable region comprising a CDR-L1, a CDR-L2, and CDR-L3, and
a heavy chain variable region comprising a CDR-H1, a CDR-H2, and a CDR-H3, wherein the CDRs are as described above.

More specifically, the anti-LILRB1 antibody or an antigen-binding fragment thereof may comprise:

a light chain variable region comprising an amino acid sequence of SEQ ID NO: 221, 223, 225, 227, 229, 231, 233, 235, 237, 239, 241, 243, 245, 247, 249, 251, 253, 255, 257, 259, or 345, and
a heavy chain variable region comprising an amino acid sequence of SEQ ID NO: 222, 224, 226, 228, 230, 232, 234, 236, 238, 240, 242, 244, 246, 248, 250, 252, 254, 256, 258, or 260.

In a specific embodiment, combinations of a light chain variable region and a heavy chain variable region that can be comprised in the anti-LILRB1 antibody or an antigen-binding fragment thereof provided in this disclosure are illustrated in Table 2:

[Table 2]

| | variable region | Amino acid sequence(N→C) | SEQ ID NO |
|---|---|---|---|
| E3 | light chain variable region | SYELTQDPAVSVALGQTVRITCQGDSLRNFYASWYQQKS GQAPVLVMYGKNNRPSGIPDRFSGSTSGNTASLTITGAQ AEDEADYYCNSRDSSGSHLTGVFGGGTKVTVLGQPAAA | 221 |
| | heavy chain variable region | QVQLVESGGGLVQPGGSLRLSCAASGFTFSSYAMSWVR QAPGKGLEWVSAISGSGGSTYYADSVKGRFTISRDNSKN TLYLQMISLRAEDTAVYYCARDTYYYGSGRSNAFDIWGQ GTLVTVSS | 222 |

(continued)

| | variable region | Amino acid sequence(N→C) | SEQ ID NO |
|---|---|---|---|
| B3 | light chain variable region | DIQMTQSPSSLSASVGDRVTITCQASQDISNYLNWYQQK PGKAPKLLIYDASNLETGVPSRFSGSGSGTDFTFTISSLQ PEDIATYYCQQYDNLPFGGGTKVDIKRTAAA | 223 |
| | heavy chain variable region | EVQLLESGGGLVQPGRSLRLSCAASGFTFDDYAMHWVR QAPGKGLEWVSGISWNSGSIGYADSVKGRFTISRDNSKN TLYLQMNSLRAEDTAVYYCARVGDSSGWSDAFDIWGQG TMVTVSS | 224 |
| A10 | light chain variable region | DIVMTQSPATLSVSPGERATLSCRASQSVSSNLAWYQQK PGQAPRLLIYGASTRATGIPARFSGSGSGTEFTLTISSLQS EDFAVYYCQQYGSSPRMYTFGQGTKVDIKRTAAA | 225 |
| | heavy chain variable region | QMQLVQSGAEVKKPGSSVKVSCKASGGTFSSYAISWVR QAPGQGLEWMGGIIPIFGTANYAQKFQGRVTITADKSIST AYMELSSLRSEDTAVYYCARGGLGELDNWFDPWGQGTL VTVSS | 226 |
| G1 | light chain variable region | SYELTQPPSLSVSPGQTASITCSGYKLGDRYVSWYQQKT GQSPVVVIYKDSQRPSGVPERFSGSNSGNTATLTISGTQ AMDEADYYCQAWDSGTGVFGGGTKLTVLGQPAAA | 227 |
| | heavy chain variable region | EVQLVQSGAEVKKPGSSVKVSCKASGGTFSSYGISWVR QAPGQGLEWMGWISAYNGNTNYAQELQGRVTMTTDTS TSTAYMELRSLRSDDTAVYYCARVGVAGKLDYWGQGTLV TVSS | 228 |
| G9 | light chain variable region | QSALTQPASVSGSPGQSITISCTGSSSDVGGYNYVSWYQ QHPGKAPKLMIYDVSNRPSGVSDRFSGSKSGNMASLTIS GLQAEDEADYYCSSYTGSSTLDVLFGGGTKLTVLGQPAA A | 233 |
| | heavy chain variable region | QVQLVQPGAEVKKPGESLKISCKGSGYSFTSYWIGWVR QMPGKGLEWMGIIYPGDSDTRYSPSFQGQVTISADKSIS TAYLQWSSLKASDTAMYYCASQYYDGGYYMDVWGQGT LVTVSS | 234 |
| H2 | light chain variable region | SYELTQDPAVSVALGQTVRITCQGDSLRNYYASWYQQKP GQAPILVISGNNKRPSGIPDRFSGSSSGDTASLTISGAQA EDEADYYCNSLDSTYNHPIFGGGTKVTVLGQPAAA | 235 |
| | heavy chain variable region | QVQLVQSGAEVKKPGASVKVSCKASGYTFTSYDIHWVR QATGQGLEWMGWISAYNGNTNYAQKLQGRVTMTTDTST STAYMELRSLRSDDTAVYYCARDGGDAFDIWGQGTLVTV SS | 236 |

(continued)

| | variable region | Amino acid sequence(N→C) | SEQ ID NO |
|---|---|---|---|
| H11 | light chain variable region | SYELTQDPAASVALGQTVRITCQGDSLRSYYASWYQQKPGQAPVVVIYGRNNRPSGIPDRFSGSSSGDTASLTITGAQAEDEADYYCKSRDSSGNHYVFGTGTKLTVLGQPAAA | 231 |
| | heavy chain variable region | QVQLVQSGAEVKKPGASVKVSCKASGYTFTSYYMHWVRQAPGQGLEWMGIINPSGGSTSYAQKFQGRVTMTRDTSTSTVYMELSSLRSEDTAVYYCARDAGSSSDYWGRGTLVTVSS | 232 |
| F12 | light chain variable region | QSVLTQPASVSGSPGQSITISCAGTSSDIGDYDYVSWYQQHPGKTPKLMIYDVSRRPSGVPDRFSGSKSGNTASLTISGLQTEDEADYYCASYTSSSVVVFGGGTKLTVLGQPAAA | 237 |
| | heavy chain variable region | QVQLVQSGAEVKKPGESLKISCKGSGYSFTSYWIGWVRQMPGKGLEWMGIIYPGDSDTRYSPSFQGQVTISADKSISTAYLQWSSLKASDTAMYYCASQYYDGGYYMDVWGQGTLVTVSS | 238 |
| B9 | light chain variable region | DIQMTQSPSSLSASVGDRVTITCRASQSISRYLNWYQQKPGKAPKLLIYGASSLQSGVPSRFSGSGSGTDFTLTISSLQPEDFATYHCQQAYGFPLTLGGGTKVEIKRTAAA | 229 |
| | heavy chain variable region | QVQLVESGAEVKKPGSSVKVSCKASGGTFSSYAISWVRQAPGQGLEWMGGIIPIFGTANYAQKFQGRVTITADESTSTAYMELSSLRSEDTAVYYCARGEIAVAQNWDYYGMDVWGQGTLVTVSS | 230 |
| G11 | light chain variable region | QSALTQPRSVSGSPGQSVTISCTGTSSDVGGYNYVSWYQQHPGKAPKLMIYDVSKRPSGVPDRFSGSKSGNTASLTISGLQAEDEADYYCSSYSSSSTLVVFGGGTKLTVLGQPAAA | 239 |
| | heavy chain variable region | QVQLVQSGAEVKKPGESLKISCKGSGYSFTSYWIGWVRQMPGKGLEWMGIIYPGDSDTRYSPSFQGQVTISADKSISTAYLQWSSLKASDTAMYYCASQYYDGGYYMDVWGQGTLVTVSS | 240 |
| G6 | light chain variable region | SYELTQDPAVSVALGQTVTITCQGDSLRRYYATWYQQKPGQAPVLVIYGQNYRPSGIPDRFSGSNSGTTASLTITGAQAEDEADYYCNSRDSSGNHVVFGGGTKLTVLGQPAAA | 241 |
| | heavy chain variable region | EVQLVESGAEVKKPGASVKVSCKASGYTFTSYYMHWVRQAPGQGLEWMGGIIPIFGTANYAQKFQGRVTITADESTSTAYMELSSLRSEDTAVYYCARGWGYSSSFDYWGQGTTVTVSS | 242 |

(continued)

| | variable region | Amino acid sequence(N→C) | SEQ ID NO |
|---|---|---|---|
| F11 | light chain variable region | QSVLTQPPSTSGTPGQTFSIFCSGSSSNIGTNTVNWYQQ LPGTAPKLLIYSNDQRPSGVPDRFSGSKSGTSASLAISGL QSEDEADYYCETWDDSLKGPVFGGGTKVTVLGQPAAA | 243 |
| | heavy chain variable region | EVQLVESGGGLVQPGGSLKLSCAASGFTFSSYAMSWVR RAPGKGLEWVSTISGSGDSTYYADSVKGRFTISRDNSKN TLYLQMNNLRAEDTAVYYCAREWELGDAFDIWGRGTLVT VSS | 244 |
| D3 | light chain variable region | DIQMTQSPSSLSASVGDRVTITCRASQSISSYLNWYQQK PGKAPKLLIYAASSLQSGVPSRFSGSGSGTDFTLTISSLQ PEDFATYYCQQSYSTRWTFGQGTKVEIKRTAAA | 245 |
| | heavy chain variable region | EVQLLESGGGVVQPGRSLRLSCAASGSTFSSYAMSWVR QAPGKGLEWVSAISGSGGSTYYADSVKGRFTISRDNSKN TLYLQMNSLRAEDTAVYYCAKDRGSYGYYYGMDVWGQ GTMVTVSS | 246 |
| B12 | light chain variable region | DIQMTQSPSSLSASVGDRVTITCRASQSISSYLNWYQQK PGKAPKLLIYAASSLQSGVPSRFSGSGSGTDFTLTISSLQ PEDFATYYCQQSYSTLRTFGQGTKVEIKRTAAA | 247 |
| | heavy chain variable region | QVQLVQSGAEVKKPGASVKVSCKASGYTFTGYYMHWV RQAPGQGLEWMGWINPNSGGTNYAQKFQGRVTITADES TSTAYMELSSLRSEDTAVYYCTRAGASIVGATALDYWGQ GTLVTVSS | 248 |
| E4 | light chain variable region | NFMLTQPHSVSESPGKTVTISCTRSSGSIASNYVQWYQQ RPGSSPTTVIYEDNQRPSGVPDRFSGSIDSSSNSASLTIS GLKTEDEADYYCQSYDTGNRNYVFGTGTQLTVLGQPAA A | 249 |
| | heavy chain variable region | QVQLVQSGAEVKKPGSSVKVSCKASGGTFSSYTISWVR QAPGQGLEWMGRIIPILGIANYAQKFQGRVTMTRDMSTD TAYMELSSLTYDDTAVYFCVRGPSLNYAGYFDNWGQGT LVTVSS | 250 |
| E12 | light chain variable region | SYELTQDPAVSVALGQTVRITCQGDSLRSYYASWYQQKS GQAPVLVIYGKEKRPSGIPDRFSGSSSGNTASLTITGARA EDEADYYCNSRGSTTDYMVFGGGTQLTVLGQPAAA | 251 |
| | heavy chain variable region | QVQLVESGGGLVQPGGSLRLSCAASGFTFSSYAMHWVR QAPGKGLEWVAVISYDGSNKYYADSVKGRFTISRDNSKN TLYLQMNSLRAEDTAVYYCARERGSGMDVWGQGTLVTV SS | 252 |

(continued)

| | variable region | Amino acid sequence(N→C) | SEQ ID NO |
|---|---|---|---|
| D1 | light chain variable region | ETTLTQSPAFMSATPGDKVNISCKASQDIDDDMNWYQQK PGEAAISIIQEASTLVPGIPPRFSGSGYGTDFTLTINNIESE DAAYYFCLQHDKFPYTFGQGTKLEIKRTAAA | 253 |
| | heavy chain variable region | EVQLVQSGAEVKKPGASVKVSCKASGYTFTSYGISWVR QAPGQGLEWMGWINPNSGGTNYAQKFQGRVTMTRDTS ISTAYMELSRLRSDDTAVYYCASRGVDEGDYWGQGTMV TVSS | 254 |
| E6 | light chain variable region | NFMLTQPHSVSESPGKTVTLSCTGSSGNIASNYVQWYQ HRPGSAPTTVIYRDDQRPSGVPDRFSGSIDSSSNSASLTI SGLRPEDEADYYCQSYDSSSWVFGGGTKLTVLGQPAAA | 255 |
| | heavy chain variable region | QVQLVQSGAEVKKPGASVKVSCKASGYTFTTYDITWVR QAPGQGLEWMGWMNPNSGNSRSAQKFQGRVSMTSDS SISTAYMELSSLRSEDTAVYYCATGDYSGVVLTATALDY WGQGTLVTVSS | 256 |
| E9 | light chain variable region | QSELTQLPSASETPGQRVTISCSGSSSNIGNNYVYWYQQ LPGTAPKLLIYRNNQRPSGVPDRFSGSKSGTSASLAISGL RSEDEADYYCAAWDDSLSGWVFGGGTKLTVLGQPAAA | 257 |
| | heavy chain variable region | QVQLVESGGGLVQPGRSLRLSCAASGFTFSSYGMHWV RQAPGKGLEWVANIKQDGSEKYYVDSVKGRFTISRDNA KNTLYLQMNSLRAEDTAVYYCAREDRIAAAGMRELDYW GQGTLVTVSS | 258 |
| A11 | light chain variable region | DIVMTQSPLSLPVTPGEPASISCRSSQSLLHSNGYNYLD WYLQKPGQSPQLLIYLGSNRASGVPDRFSGSGSGTDFT LKISRVEAEDVGVYYCMQGTHWPPYTFGQGTKVEIKRTA AA | 259 |
| | heavy chain variable region | EVQLLESGGGLEQPGGFLRLSCAASGFSFTSYAMTWVR QAPGKGLEWVSGISSDGTTTTYADSVRGRFTISRDNAKN TVYLQMNSLRDEDTAVYYCARDQLLGWDALNVWGQGT MVTVSS | 260 |
| E3.1 | light chain variable region | SYELTQDPAVSVALGQTVRITCQGDSLRNFYASWYQQKS GQAPVLVMYGKNNRPSGIPDRFSGSTSGNTASLTITGAQ AEDEADYYCNSRDSSGSHLTGVFGGGTKVTVL | 345 |
| | heavy chain variable region | QVQLVESGGGLVQPGGSLRLSCAASGFTFSSYAMSWVR QAPGKGLEWVSAISGSGGSTYYADSVKGRFTISRDNSKN TLYLQMISLRAEDTAVYYCARDTYYYGSGRSNAFDIWGQ GTLVTVSS | 222 |

In this disclosure, the expression "an antibody or an antigen-binding fragment (for example, CDR, variable region, or heavy chain /light chain) comprising, consists of, or represented by a certain amino acid sequence" may refer to an antigen-binding fragment that consists essentially of (1) the certain amino acid sequence or (2) an amino acid sequence wherein an insignificant mutation (for example, substitution, deletion, and/or addition of an amino acid residue(s); leading to no impact on the activity of the antibody) is introduced in the amino acid sequence (1).

The anti-LILRB1 antibody or an antigen-binding fragment thereof provided in this disclosure may have a binding affinity ($K_D$) to LILRB1 (for example, human LILRB1) of 10mM or less, 5 mM or less, 1mM or less, 0.5mM or less, 0.2mM, or 0.15mM or less, for example, 0.001 nM to 10mM, 0.005nM to 10mM, 0.01nM to 10mM, 0.05nM to 10mM, 0.1nM to 10mM, 0.5nM to 10mM, 1nM to 10mM, 0.001nM to 5mM, 0.005nM to 5mM, 0.01nM to 5mM, 0.05nM to 5mM, 0.1nM to 5mM, 0.5nM to 5mM, 1nM to 5mM, 0.001 nM to 1mM, 0.005nM to 1mM, 0.01nM to 1mM, 0.05nM to 1mM, 0.1nM to 1mM, 0.5nM to 1mM, 1nM to 1mM, 0.001 nM to 0.5mM, 0.005nM to 0.5mM, 0.01nM to 0.5mM, 0.05nM to 0.5mM, 0.1nM to 0.5mM, 0.5nM to 0.5mM, 1nM to 0.5mM, 0.001 nM to 0.2mM, 0.005nM to 0.2mM, 0.01nM to 0.2mM, 0.05nM to 0.2mM, 0.1nM to 0.2mM, 0.5nM to 0.2mM, 1nM to 0.2mM, 0.001nM to 0.15mM, 0.005nM to 0.15mM, 0.01nM to 0.15mM, 0.05nM to 0.15mM, 0.1nM to 0.15mM, 0.5nM to 0.15mM, or 1nM to 0.15mM, when measured by surface plasmon resonance (SPR).

Another embodiment provides a pharmaceutical composition comprising the anti-LILRB1 antibody or an antigen-binding fragment thereof as an active ingredient. For example, the pharmaceutical composition may be a pharmaceutical composition for treating and/or preventing a cancer. The pharmaceutical composition may have an activity to inhibit the binding of LILRB1 to MHC Class I and/or the interaction between LILRB1 and MHC Class I. The cancer may be a cancer associated with the interaction between LILRB1 and MHC Class I. In an embodiment, the pharmaceutical composition may have an activity to inhibit immune evasion of a cancer cell. The cancer cell may be a cell expressing or overexpressing MHC Class I on cell surface.

Another embodiment provides a composition for blocking the binding of LILRB1 to MHC Class I and/or the interaction between LILRB1 and MHC Class I, the composition comprising the anti-LILRB1 antibody or an antigen-binding fragment thereof as an active ingredient.

Another embodiment provides a composition for inhibiting immune evasion of a cancer cell, the composition comprising the anti-LILRB1 antibody or an antigen-binding fragment thereof as an active ingredient.

Another embodiment provides a method of treating and/or preventing a cancer, comprising administering (orally or parenterally) a pharmaceutically effective amount of the anti-LILRB1 antibody or an antigen-binding fragment thereof to a subject (e.g., a mammal including human) in need of treating and/or preventing the cancer.

Another embodiment provides a method of blocking the binding of LILRB1 to MHC Class I and/or a method of blocking the interaction between LILRB1 and MHC Class I, comprising administering (orally or parenterally) a pharmaceutically effective amount of the anti-LILRB1 antibody or an antigen-binding fragment thereof to a subject (e.g., a mammal including human) in need of inhibiting the binding of LILRB1 to MHC Class I and/or the interaction between LILRB1 and MHC Class I.

Another embodiment provides a method of inhibiting immune evasion of a cancer cell, comprising administering (orally or parenterally) a pharmaceutically effective amount of the anti-LILRB1 antibody or an antigen-binding fragment thereof to a subject (e.g., a mammal including human) in need of inhibiting immune evasion of the cancer cell.

The methods provided in this disclosure may further comprise a step of identifying the subject in need of treating and/or preventing the cancer, inhibiting the binding of LILRB1 to MHC Class I and/or the interaction between LILRB1 and MHC Class I, and/or inhibiting immune evasion of the cancer cell, prior to the step of administering.

Another embodiment provides a nucleic acid molecule (polynucleotide) encoding at least one polypeptide selected from the group consisting of CDR (CDR-L1, CDR-L2, CDR-L3, CDR-H1, CDR-H2, CDR-H3, a combination of CDR-L1, CDR-L2, and CDR-L3, or a combination of CDR-H1, CDR-H2, and CDR-H3), a light chain variable region comprising CDR-L1, CDR-L2, and CDR-L3, a heavy chain variable region comprising CDR-H1, CDR-H2, and CDR-H3; a light chain comprising the light chain variable region, and a heavy chain comprising the heavy chain variable region, of the anti-LILRB1 antibody described above.

Another embodiment provides a recombinant vector comprising the nucleic acid molecule. In an embodiment, the recombinant vector may comprise a nucleic acid molecule encoding the light chain variable region or light chain, and a nucleic acid molecule encoding the heavy chain variable region or heavy chain, respectively (e.g., in two separate vectors) or all together (e.g., in one vector). The recombinant vector may be used as an expression vector.

Another embodiment provides a recombinant cell comprising the nucleic acid molecule or the recombinant vector.

Another embodiment provides a method of preparing an anti-LILRB1 antibody or an antigen-binding fragment thereof, comprising expressing the nucleic acid molecule in a cell. The step of expressing the nucleic acid molecule may comprise culturing the recombinant cell.

As described herein, the antigen-binding fragment of an anti-LILRB1 antibody may refer to a fragment which is derived from an anti-LILRB1 antibody and retain antigen (LILRB1) binding affinity of the antibody. In an embodiment, the antigen-binding fragment may be an polypeptide comprising the 6 CDRs of an anti-LILRB1 antibody as described above, and, for example, may be scFv, scFv-Fc, scFv-Ck(kappa constant region), scFv-Cλ(lambda constant region), (scFv)$_2$, Fab, Fab', or a F(ab')$_2$, but not be limited thereto. In an embodiment, the antigen-binding fragment may be scFv, a fusion polypeptide (scFv-Fc) wherein scFv is fused with a Fc region of an immunoglobulin (e.g., IgA, IgD, IgE, IgG (IgG1, IgG2, IgG3, IgG4), IgM, etc.), or a fusion polypeptide (scFv-Ck or scFv-Cλ) wherein scFv is fused with a constant region (e.g., kappa or lambda) of a light chain.

The anti-LILRB1 antibody or an antigen-binding fragment thereof may have a regulatory activity, for example, an antagonistic or agonistic activity, on LILRB1 protein. In addition, the anti-LILRB1 antibody or an antigen-binding fragment thereof may have an activity of blocking the binding of LILRB1 to MHC Class I and/or the interaction between LILRB1 and MHC Class I. In addition, the anti-LILRB1 antibody or an antigen-binding fragment thereof may have an activity of inhibiting immune evasion of a cancer cell. Furthermore, the anti-LILRB1 antibody or an antigen-binding fragment thereof may have an anti-cancer effect.

A protein LILRB1, which is an antigen of an anti-LILRB1 antibody or an antigen-binding fragment thereof provided in this disclosure, may be derived from mammal. For example, LILRB1 as an antigen may be a human LILRB1 (e.g., GenBank accession numbers AAH15731.1 (SEQ ID NO: 348), NP_001265328.2, NP_001265327.2, NP_001075108.2, NP_001075107.2, NP_001075106.2, NP_006660.4, NM_001081637.2, NM_001081638.3, NM_001081639.3, NM_001278398.2, NM_001278399.2, etc.), but not be limited thereto.

MHC Class I may be one of classes of major histocompatibility complex (MHC) molecules. In an embodiment, the MHC Class I may be a human MHC Class I and may be at least one selected from the group consisting of HLA(human leukocyte antigen)-A, HLA-B, HLA-C, HLA-E, HLA-F, and HLA-G, but not be limited thereto.

As described herein, the term "antibody" may refer to a protein that specifically binds to a specific antigen, and may be a protein produced by stimulation of an antigen in the immune system, or a protein produced by chemical synthesis or recombinant production, with no specific limitation. The antibody may be non-naturally occurring, for example, produced by recombinant or synthetic production. The antibody may be an animal antibody (e.g., a mouse antibody, etc.), a chimeric antibody, a humanized antibody, or a human antibody. The antibody may be a monoclonal or polyclonal antibody.

In the anti-LILRB1 antibody or an antigen-binding fragment thereof provided herein, the portion, except for the heavy-chain CDR and light-chain CDR portions or the heavy-chain variable and light-chain variable regions as defined above, may be derived from any subtype of immunoglobulin (e.g., IgA, IgD, IgE, IgG (IgG1, IgG2, IgG3, IgG4), IgM, and the like), and, for example, derived from the framework portions, and/or light-chain constant region and/or heavy-chain constant region. In an embodiment, the anti-LILRB1 antibody provided in this disclosure may be an antibody in a form of human IgG, for example, IgG1, IgG2, IgG3, or IgG4, but not be limited thereto.

An intact antibody (e.g., IgG type) has a structure with two full-length light chains and two full-length heavy chains, in which each light chain is linked to a corresponding heavy chain via a disulfide bond. The constant region of an antibody is divided into a heavy-chain constant region and a light-chain constant region. The heavy-chain constant region is of a gamma ($\gamma$), mu ($\mu$), alpha ($\alpha$), delta ($\delta$), or epsilon ($\varepsilon$) type, and has gamma1 ($\gamma$1), gamma2 ($\gamma$2), gamma3 ($\gamma$3), gamma4 ($\gamma$4), alpha1 ($\alpha$1) or alpha2 ($\alpha$2) as its subclass. The light chain constant region is of either a kappa ($\kappa$) or lambda ($\lambda$) type.

As used herein, the term "heavy chain" may be intended to encompass a full-length heavy chains and fragments thereof, wherein the full-length heavy chain may comprise a variable region VH including amino acid sequences sufficient to provide specificity to antigens, three constant regions CH1, CH2, and CH3, and a hinge. The term "light chain" may be intended to encompass full-length light chains and fragments thereof, wherein the full-length light chain may comprises a variable region VL including amino acid sequences sufficient to provide specificity to antigens, and a constant region CL.

The term "complementarity determining region (CDR)" may refer to a portion that confers antigen-binding specificity in a variable region of an antibody, and may refer to an amino acid sequence found in a hyper variable region of a heavy chain or a light chain of immunoglobulin. The heavy and light chains may respectively include three CDRs (CDRH1, CDRH2, and CDRH3; and CDRL1, CDRL2, and CDRL3). The CDR may provide contacting residues that play an important role in the binding of an antibody to its antigen or an epitope of the antigen. As used herein, the terms "specifically binding" and "specifically recognizing" may have the same general meaning as known to one of ordinary skill in the art, and indicate that an antibody and an antigen specifically interact with each other to lead to an immunological reaction.

In this disclosure, unless differently stated, the term "antibody" may encompass not only an intact antibody but also an antigen-binding fragment of the antibody possessing an antigen-binding capability.

The term "antigen-binding fragment" used herein may refer to a polypeptide in any type, which comprises a portion (e.g., 6 CDRs as described herein) capable of binding to an antigen, and, for example, may be scFv, (scFv)$_2$, scFv-Fc, Fab, Fab', or F(ab')$_2$, but is not limited thereto. In addition, as described above, the antigen-binding fragment may be scFv, a fusion polypeptide wherein scFv is fused with a Fc region of an immunoglobulin (e.g., IgA, IgD, IgE, IgG (IgG1, IgG2, IgG3, IgG4), IgM, etc.) or a constant region (e.g., kappa or lambda).

Among the antigen-binding fragments, Fab includes light chain and heavy chain variable regions, a light chain constant region, and a first heavy chain constant region CH1.

Fab' is different from Fab in that Fab' comprises a hinge region having at least one cysteine residue at the C-terminal of CH1.

F(ab')$_2$ antibody is formed through disulfide bridging of the cysteine residues in the hinge region of Fab'.

Fv is a minimal antibody fragment composed of only a heavy chain variable region and a light chain variable region. Recombination techniques of generating an Fv fragment are widely known in the art.

Two-chain Fv comprises a heavy chain variable region and a light chain variable region which are linked to each other by a non-covalent bond. Single-chain Fv generally comprises a heavy-chain variable region and a light-chain variable region which are linked to each other by a covalent bond via a peptide linker or directly linked at the C-terminals to have a dimer structure like two-chain Fv.

The antigen-binding fragments may be obtained using protease (for example, Fab may be obtained by restrictively cleaving a whole antibody with papain, and an F(ab')$_2$ fragment may be obtained by cleaving with pepsin), or may be prepared by using a genetic recombination technique.

The term "hinge region" may refer to a region between CH1 and CH2 domains within heavy chain of an antibody, which functions to provide flexibility for the antigen-binding site in the antibody.

The anti-LILRB1 antibody may be a monoclonal or polyclonal antibody and, for example, a monoclonal antibody. A monoclonal antibody can be prepared using a method widely known in the art, for example, using a phage display technique. Alternatively, the anti-LILRB1 antibody may be constructed in the form of a mouse-derived monoclonal antibody by a conventional method.

Meanwhile, individual monoclonal antibodies can be screened using a typical ELISA (Enzyme-Linked ImmunoSorbent Assay) format, based on the binding potential against LILRB1. Inhibitory activities can be verified through functional analysis such as competitive ELISA for verifying the molecular interaction of binding assemblies or functional analysis such as a cell-based assay. Then, with regard to monoclonal antibody members selected on the basis of their strong inhibitory activities, their affinities (Kd values) to LILRB1 may be each verified.

The pharmaceutical composition may further comprise a pharmaceutically acceptable carrier, in addition to the active ingredient (the anti-LILRB1 antibody or an antigen-binding fragment thereof). The pharmaceutically acceptable carrier may be anyone selected from those commonly used for the formulation of antibodies. For example, the pharmaceutically acceptable carrier may be one or more selected from the group consisting of lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, mineral oil, and the like, but are not limited thereto. The pharmaceutical composition may further comprise one or more selected from the group consisting of a diluent, an excipient, a lubricant, a wetting agent, a sweetener, a flavor enhancer, an emulsifying agent, a suspension agent, preservative, and the like, which can be commonly used for manufacturing pharmaceutical composition.

The pharmaceutical composition, or the antibody or an antigen-binding fragment thereof may be administered orally or parenterally in a pharmaceutically effective amount. The parenteral administration may be intravenous injection, subcutaneous injection, muscular injection, intraperitoneal injection, endothelial administration, intranasal administration, intrapulmonary administration, rectal administration or intralesional local administration. Since proteins or peptides are digested when administered orally, the active ingredient in the compositions for oral administration may be coated or formulated to prevent digestion in stomach. In addition, the antibody or the compositions may be administered using an optional device that enables the active ingredient to be delivered to target cells (e.g., cancer cells).

The anti-LILRB1 antibody or an antigen-binding fragment thereof may be comprised in the pharmaceutical composition or administered to a subject in a pharmaceutically effective amount. As used herein, the term "pharmaceutically effective amount" may refer to an amount of an active ingredient (the antibody or fragment thereof) at which the active ingredient can exert desired effects (e.g., anti-cancer effect). The pharmaceutically effective amount may be prescribed in a variety of ways, depending on various factors, such as age, body weight, gender, pathologic conditions, diets, excretion speed, and/or reaction sensitivity of a subject, formulation types, administration time, administration interval, administration route, administration manner, and the like. For example, anti-LILRB1 antibody or an antigen-binding fragment thereof may be administered at the amount of 0.005 ug/kg to 1000mg/kg, 0.005 ug/kg to 500mg/kg, 0.005 ug/kg to 250mg/kg, 0.005 ug/kg to 100mg/kg, 0.005 ug/kg to 75mg/kg, 0.005 ug/kg to 50mg/kg, 0.01 ug/kg to 1000mg/kg, 0.01 ug/kg to 500mg/kg, 0.01 ug/kg to 250mg/kg, 0.01 ug/kg to 100mg/kg, 0.01 ug/kg to 75mg/kg, 0.01 ug/kg to 50mg/kg, 0.05 ug/kg to 1000mg/kg, 0.05 ug/kg to 500mg/kg, 0.05 ug/kg to 250mg/kg, 0.05 ug/kg to 100mg/kg, 0.05 ug/kg to 75mg/kg, or 0.05 ug/kg to 50mg/kg per day, but not be limited thereto. The daily dosage may be formulated into a single formulation in a unit dosage form or formulated in suitably divided dosage forms, or it may be manufactured to be contained in a multiple dosage container.

The pharmaceutical compositions may be formulated into a form of a solution in oil or an aqueous medium, a suspension, syrup, an emulsifying solution, an extract, powder, granules, a tablet, or a capsule, and may further comprise a dispersing or a stabilizing agent for the formulation.

The subject, to whom the antibody, pharmaceutical composition, or method provided in this disclosure is applied, may be selected from mammals including a mammal including primates such as humans and monkeys, rodents such as rats and mice, and the like.

The cancer may be a solid cancer or blood cancer. The cancer may be, but not limited to, one or more selected from the group consisting of lung cancer (e.g., squamous cell carcinoma of the lung, small-cell lung cancer, non-small-cell lung cancer, adenocarcinoma of the lung), peritoneal carcinoma, skin cancer, squamous cell carcinoma, melanoma in the skin or eyeball, rectal cancer, cancer near the anus, esophagus cancer, small intestinal tumor, endocrine gland cancer, parathyroid cancer, adrenal cancer, soft-tissue sarcoma, urethral cancer, leukemia (e.g., chronic or acute leukemia), lymphocytic lymphoma, hepatoma, gastric cancer, pancreatic cancer, glioblastoma, cervical cancer, ovarian cancer, liver cancer, bladder cancer, hepatocellular adenoma, breast cancer, colon cancer, large intestine cancer, endometrial carcinoma or uterine carcinoma, salivary gland tumor, renal cell carcinoma, kidney cancer, prostate cancer, vulvar cancer, thyroid cancer, head and neck cancer, brain cancer, biliary tract cancer, gallbladder cancer, bone osteosarcoma, and the like. The cancer may be a primary cancer or a metastatic cancer. The cancer may be a cancer characterized by expression or overexpression of MHC Class I on a surface of cancer cell, and, for example, may be colon adenocarcinoma, small cell lung carcinoma, breast cancer, pancreatic cancer, malignant melanoma, bone osteosarcoma, renal cell carcinoma, or gastric cancer. The overexpression of MHC Class I may refer to an overexpression compared to that of a normal cell or a cancer cell which is non-responsive or resistant to the immunotherapy, for example, T-cell (e.g., cytotoxic T-cell) mediated immunotherapy.

As used herein, the term "treatment of cancer" may refer to all anti-cancer actions that prevent, alleviate or ameliorate the symptoms of cancer, or partially or completely remove a cancer, such as, cancer cell death, inhibition of cancer cell proliferation, inhibition of cancer metastasis, and the like.

The anti-LILRB1 antibody or an antigen-binding fragment thereof provided in this disclosure may be co-administered with another drug, for example, at least one selected from the group consisting of conventionally used agents for immunotherapy, anti-cancer agents, cytotoxic agents, and the like. Accordingly, an embodiment provides a pharmaceutical composition of combined administration for treating and/or preventing a cancer, comprising (1) an anti-LILRB1 antibody or an antigen-binding fragment thereof, and (2) at least one selected from the group consisting of agents for immunotherapy, anti-cancer agents, cytotoxic agents, and the like. Another embodiment provides a method of treating and/or preventing a cancer, comprising administering (1) an anti-LILRB1 antibody or an antigen-binding fragment thereof, and (2) at least one selected from the group consisting of agents for immunotherapy, anti-cancer agents, cytotoxic agents, and the like, to a subject in need of treating and/or preventing the cancer. The agents for immunotherapy, anti-cancer agents, and cytotoxic agents may include any drugs which are conventionally used for cancer therapy, and/or have cytotoxic activity, and for example, they may be at least one selected from the group consisting of proteins such as antibodies, nucleic acid molecules such as siRNA, and/or small molecular chemicals such as paclitaxel, docetaxel, and the like, but not limited thereto.

Another embodiment provides a polypeptide molecule comprising a heavy chain complementarity determining region (CDR-H1, CDR-H2, CDR-H3, or a combination thereof), a light chain complementarity determining region (CDR-L1, CDR-L2, CDR-L3, or a combination thereof), a combination thereof; or heavy chain variable region, light chain variable region, or a combination thereof, of the anti-LILRB1 antibody as described above. The polypeptide molecule may be used in preparing an antibody as a precursor of antibody, or comprised in a protein scaffold having an antibody-like structure (e.g., peptibody), a bispecific antibody, or a multispecific antibody, as a component thereof. In another embodiment, the polypeptide molecule may be used as a target (antigen) recognition domain or a secreted antibody, in cell therapeutics for target therapy, such as CAR-T. In another embodiment, the polypeptide molecule may be used for constructing anti-LILRB1 antibody-secreting cells as cell therapeutics.

Another embodiment provides a nucleic acid molecule encoding a heavy chain complementarity determining region (CDR-H1, CDR-H2, CDR-H3, or a combination thereof), a heavy chain variable region, or a heavy chain, of the anti-LILRB1 antibody.

Another embodiment provides a nucleic acid molecule encoding a light chain complementarity determining region (CDR-L1, CDR-L2, CDR-L3, or a combination thereof), a light chain variable region, or a light chain, of the anti-LILRB1 antibody.

Another embodiment provides a recombinant vector comprising a nucleic acid molecule encoding a heavy chain variable region or a heavy chain of the anti-LILRB1 antibody, and a light chain variable region or a light chain of the anti-LILRB1 antibody, respectively in two separate vectors or all together in one vector.

Another embodiment provides a recombinant cell comprising the nucleic acid molecule or the recombinant vector.

The term "vector" refers to a means for expressing a target gene in a host cell, as exemplified by a plasmid vector, a cosmid vector, and a viral vector such as a bacteriophage vector, a lentivirus vector, an adenovirus vector, a retrovirus vector, and an adeno-associated virus vector. The recombinant vector may be constructed from or by manipulating a plasmid (for example, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, etc.), a phage (for example, λgt4λB, λ-Charon, λΔz1, M13, etc.), or a virus vector (for example, SV40, etc.), which is commonly used in the art.

In the recombinant vector, the nucleic acid molecule may be operatively linked to a promoter. The term "operatively linked" is intended to pertain to a functional linkage between a nucleotide sequence of interest and an expression regulatory sequence (for example, a promoter sequence). When being "operatively linked", the regulatory element can control the transcription and/or translation of a polynucleotide of interest.

The recombinant vector may be constructed typically as a cloning vector or an expression vector. For recombinant expression vectors, a vector generally available in the relevant art for expressing a foreign protein in plant, animal, or microbial cells may be employed. Various methods well known in the art may be used for the construction of recombinant vectors.

For use in hosts, such as prokaryotic or eukaryotic cells, the recombinant vector may be constructed accordingly. For example, when a vector is constructed as an expression vector for use in a prokaryotic host, the vector typically includes a strong promoter for transcription (e.g., a pL$^\lambda$ promoter, a CMV promoter, a *trp* promoter, a *lac* promoter, a *tac* promoter, a T7 promoter, etc.), a ribosomal binding site for initiating translation, and transcriptional/translational termination sequences. On the other hand, an expression vector for use in a eukaryotic host includes an origin of replication operable in a eukaryotic cell, such as an f1 origin of replication, an SV40 origin of replication, a pMB1 origin of replication, an adeno origin of replication, an AAV origin of replication, and a BBV origin of replication, but is not limited thereto. In addition, the expression vector typically includes a promoter derived from genomes of mammalian cells (for example, metallothionein promoter) or from mammalian viruses (for example, adenovirus late promoter, vaccinia virus 7.5K promoter, SV40 promoter, cytomegalovirus promoter, *tk* promoter of HSV, etc.), and a polyadenylation sequence as a transcription termination sequence.

The recombinant cell may be prepared by introducing the recombinant vector into a suitable host cell. As long as it allows the sequential cloning and expression of the recombinant vector in a stable manner, any host cell known in the art may be employed in the present disclosure. Examples of the prokaryotic host cell available for the present disclosure may be selected from E. coli such as *E. coli* JM109, *E. coli* BL21, *E. coli* RR1, *E. coli* LE392, *E. coli* B, *E. coli* X 1776, *E. coli* W3110, Bacillus spp. such as *Bacillus subtilis* and *Bacillus thuringiensis,* and enterobacteriaceae strains such as *Salmonella typhimurium*, *Serratia marcescens* and various Pseudomonas species. Eukaryotic host cells that may be used for transformation may selected from, but are not limited to, *Saccharomyces cerevisiae*, insect cells, and animal cells, such as Sp2/0, CHO (Chinese hamster ovary) K1, CHO DG44, CHO S, CHO DXB11, CHO GS-KO, PER.C6, W138, BHK, COS-7, 293, HepG2, Huh7, 3T3, RIN, MDCK, etc.

The nucleic acid molecule or a recombinant vector carrying the same may be introduced (transfected) into a host cell using a method well known in the relevant art. For example, this transfection may be carried out using a CaCl$_2$ or electroporation method when the host cell is prokaryotic. For eukaryotic host cells, the genetic introduction may be achieved using, but not limited to, microinjection, calcium phosphate precipitation, electroporation, liposome-mediated transfection, or particle bombardment.

To select a transformed host cell, advantage may be taken of a phenotype associated with a selection marker according to methods well known in the art. For example, when the selection marker is a gene conferring resistance to a certain antibiotic, the host cells may be grown in the presence of the antibiotic in a medium to select a transformant of interest.

Another embodiment provides a method of preparing the anti-LILRB1 antibody or an antigen-binding fragment thereof, comprising expressing the nucleic acid molecule or a recombinant vector in a host cell. The step of expressing may be conducted by culturing the recombinant cell comprising the nucleic acid molecule (for example, in a recombinant vector) under a condition allowing the expression of the nucleic acid molecule. The method may further comprise isolating and/or purifying the antibody or its fragment from the cell culture, after the step of expressing or culturing.

[Advantageous Effects]

The anti-LILRB1 antibody or an antigen-binding fragment thereof provided in this disclosure can have high anti-cancer effect by inhibiting the immune evasion mechanism of cancer cells, allowing that the immune cells can exhibit their anti-cancer effect.

[Description of Drawings]

Fig. 1 is an electrophoresis image showing the results of SDS-PAGE gel analysis for anti-LILRB1 antibodies purified in an example.

Fig. 2 is a sensorgram showing the results of SPR (surface plasmon resonance) assay for anti-LILRB1 antibody B3 according to an example.

Fig. 3 is a sensorgram showing the results of SPR assay for anti-LILRB1 antibody E3 according to an example.

Fig. 4a is a graph showing binding ability of anti-LILRB1 antibody A10 according to an example to a human natural killer cell, KHYG-1; Fig. 4b is a graph showing binding ability of anti-LILRB1 antibody E3 according to an example to a human natural killer cell, KHYG-1; and Fig. 4c is a graph showing binding ability of human IgG4 isotype control antibody to a human natural killer cell, KHYG-1.

Fig. 5 is a graph showing the level of binding of recombinant LILRB1-Fc proteins to HLA-G overexpressing cell surface measured by iQue screener, when treated with anti-LILRB1 antibodies according to an example and human IgG4 isotype control antibody, respectively.

Fig. 6 is a graph showing in vivo antitumor effects of anti-LILRB1 antibody E3 and B3 according to an example.

Figs. 7a to 7d are flow cytometry diagrams of binding of anti-LILRB1 antibody E3.1 according to an example to cells expressing various members of human LILR family.

Figs. 8a to 8d are flow cytometry diagrams of binding of anti-LILRB1 antibody H11 according to an example to cells expressing various members of human LILR family.

Fig. 9 is a graph showing release level of granzyme B in a human natural killer cell, KHYG-1, when treated with anti-LILRB1 antibody E3.1 or H11 according to an example, comparing with that in the cell treated with a control antibody (human IgG4 isotype).

Fig. 10 is a graph showing release level of perforin in a human natural killer cell, KHYG-1, when treated with anti-LILRB1 antibody E3.1 or H11 according to an example, comparing with that in the cell treated with a control antibody (human IgG4 isotype).

Fig. 11 is a graph showing results of luciferase reporter assay for evaluating ability of anti-LILRB1 antibody E3.1 or H11 according to an example to block LILRB1 signal pathway.

Fig. 12 is a graph showing in vivo anti-tumor effects of anti-LILRB1 antibody E3.1 and H11 according to an example.

[Mode for Invention]

Hereafter, the present invention will be described in detail by examples.

The following examples are intended merely to illustrate the invention and are not construed to restrict the invention.

**Example 1: Preparation of human antibodies against LILRB1**

**1.1. Selection of human antibodies against LILRB1 using phage display**

In order to select antibodies that specifically recognize human LILRB1, a phage display screening was performed using a library composed of human scFv antibodies. As an antigen, human LILRB1-His (Cat. No. 8989-T2) and human LILRB1-Fc (Cat. No. 2017-T2) (RnD systems) were used respectively. Each antigen was conjugated with biotin by EZ-Link Sulfo-NHS-Biotin kit (ThermoFisher Scientific) for use.

The phage display screening was performed using total 4-types of LILRB1 antigens (LILRB1-His, LILRB1-Fc, LILRB1-His-Biotin, and LILRB1-Fc-Biotin) through solid-phase screening and solution-phase screening. Additional screenings were performed by gradually decreasing the concentration of the used antigen, competitively eluting with control antibodies against LILRB1, conducting negative selection to Fc when LILRB1-Fc is used as an antigen, etc. The selected products were confirmed for their binding to the antigen through polyclonal phage ELISA.

**1.2. Screening and analysis of monoclonal soluble scFvs**

Genes encoding the scFvs, which were verified to bind the antigen in Example 1.1, were amplified by PCR to prepare expression vectors. For each selection, a certain number of transformants were transferred to a 96 well culture plate for screening. Antibodies in a scFv form were expressed using Autoinduction media (Studier, F.W. (2005) Protein Expression and Purification 41, 207-34) and then analyzed for their binding to the antigen by performing DELFIA immune assay (PerkinElmer). In addition, after allowing a certain amount of each scFv antibody to be captured on the surface, DELFIA for the antigen was performed to determine the ranking for antigen-antibody binding affinity.

**1.3. Conversion of the screened scFvs into IgG antibodies**

Among the clones which were confirmed to bind to the antigen in Example 1.2, a total of 376 clones were selected, and the DNA sequences of genes encoding the selected scFvs were analyzed by a general DNA sequencing to remove duplicate clones. In addition, a total of 93 clones were selected based on the ranking of the antigen-antibody binding affinity determined in Example 1.2. Genes encoding a heavy chain variable region(VH) and a light chain variable region(VL) were respectively amplified by PCR from each of the genes encoding the selected scFvs, and inserted into an expression vector (pTRIOZ-hIgG4, InvivoGen; alternatively, any one of vectors comprising CMV promoter or CMV/CHO beta-actin fusion promoter (KR10-1038126B1) and genes encoding human IgG4 heavy chain constant region and kappa or lambda light chain constant region can be used), wherein the expression vector was designed for encoding a human IgG4 antibody(IgG4 Fc: SEQ ID NO: 341, Kappa constant region: SEQ ID NO: 342, Lambda constant region: SEQ ID NO: 343). The DNA sequence of the expression vector was confirmed by sequencing.

## 1.4. Preparation of selected antibodies

The vectors constructed in Example 1.3 were purified using Plasmid Plus Maxi kit (Qiagen). The purified vectors were used for expressing antibodies in ExpiCHO-S™ cells or Expi293™ cells.

In particular, the vectors constructed in Example 1.3 were transfected into ExpiCHO-S™ cells(Gibco) (1.5 x $10^8$ cells/Culture Volume 25 mL) by adding 80 $\mu$L of ExpiFectamine™ CHO reagent (Thermo Fisher). One day post-transfection, 150 $\mu$L of ExpiCHO™ Enhancer (Thermo Fisher) and 4 mL of ExpiCHO™ Feed (Thermo Fisher) were added to the culture. On day 5, 4 mL of ExpiCHO™ Feed was added to the culture. The transfected cells were cultured under the conditions of 32°C and 5% $CO_2$ for 7-11 days in total.

In addition, the vectors constructed in Example 1.3 were transfected into Expi293F™ cells (Gibco) (3 x $10^8$ cells/Culture Volume 100 mL) by adding 320 $\mu$L of ExpiFectamine™ 293 Reagent (Gibco) according to manufacturer's protocol. One day post-transfection, ExpiFectamine™ 293 Enhancer 1 (Thermo Fisher), ExpiFectamine™ Enhancer 2 (Thermo Fisher), and glucose were added in the amount of 0.6 mL per Culture Volume 100 mL, 6 mL per Culture Volume 100 mL, and 3.6 g per 1 liter, respectively. The transfected cells were cultured under the conditions of 36.5°C and 5% $CO_2$ for 5 days in total. The cultured cells of two types were respectively centrifuged at 4000 rpm at 4°C for 20 minutes, and then, filtrated using 0.22 um bottle-top filter system (Corning). The culture supernatant was harvested and purified using AKTA Pure L (GE healthcare). The culture supernatant was loaded into AKTA Pure L equipped with Hitrap MabSelectSure 1mL column (GE healthcare) at the flow rate of 1 mL/min,, and the column was washed with 20 column volumes (CV) of 1X PBS. Then, elutionbuffer (0.1 M sodium citrate pH 3.4 buffer) was loaded to the column, to elute a protein of interest. The eluate was concentrated using Amicon Ultra Filter Device (MWCO 10K, Merck),, centrifuged and subjected to buffer exchange with 1xPBS buffer.

The purified antibody samples were diluted with 1X PBS, to make the final concentration about 1 mg/mL. Ten (10) $\mu$L of Reducing Loading Buffer (3X) or Nonreducing Loading Buffer (3X) and 20 $\mu$L of the purified antibody sample were mixed and left in 95°C heating bath for 2 minutes, and then, brought out and cooled. The sample was injected into SDS-PAGE Gradient Gel (4-20% or 4-12%) equipped on an electrophoresis device at the amount of 10$\mu$g per well and developed on the gel. In order to analyze molecular weight of the sample, Precision Plus Protein™ Dual Color Standards (BIO-RAD) was injected to another separate well. The gel was stained with Coomassie staining solution and destained to obtain gel images.

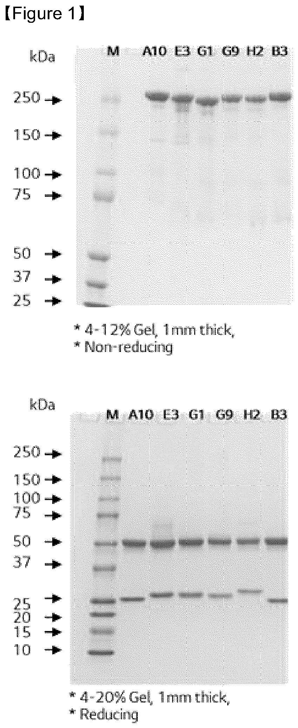

Among 93 antibodies, gel electrophoresis images for antibodies A10, B3, E3, G1, G9 and H2 were representatively shown in Fig. 1. As shown in Fig. 1, the production of antibodies having disulfide bond was confirmed.

## 1.5. Analysis of binding affinity of the selected antibodies

The binding affinities of the 93 antibodies, which were selected in Example 1.3, to the antigen, LILRB1, were measured using Biacore T200 (GE healthcare). An anti-human IgG (Fc) antibody (GE healthcare, Cat. No. BR-1008-39, final concentration of 25 $\mu$g/mL) was flowed at the flow rate of 5 $\mu$L/min for 360 seconds to be immobilized at 5000-7000 RU on Series S Sensor Chip CM5 (GE healthcare, Cat. No. BR-1005-30) using Amine Coupling Kit (GE healthcare, Cat. No. BR-1000-508). The antigen, human LILRB1 protein (LILRB1-His, RnD systems Cat. No. 8989-T2) was injected thereto in 4~9 different concentrations from 3.13 nM to 1600 nM at the flow rate of 30 $\mu$L/min to determine $k_a$ and $k_d$ values as shown in Table 3 and calculate $K_D$ value therefrom.

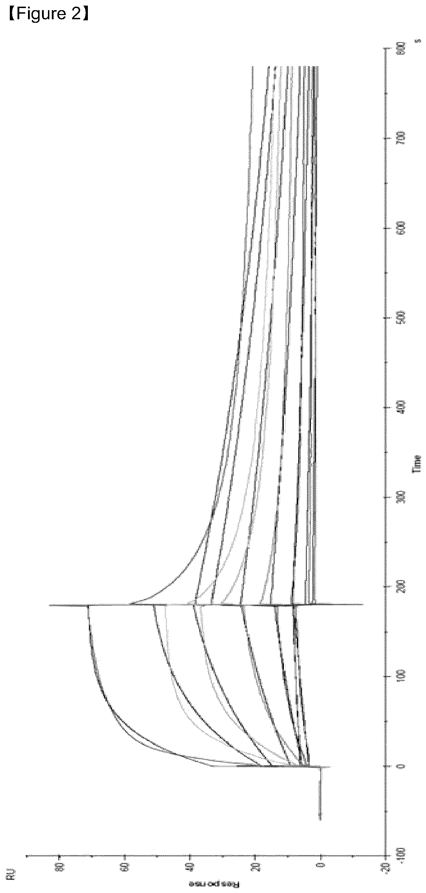

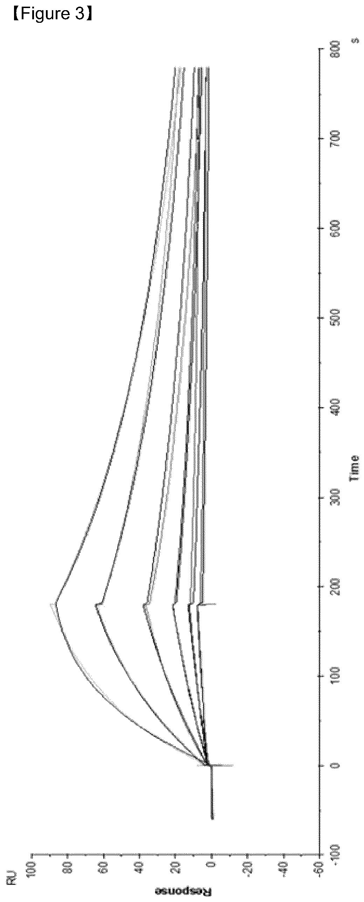

Among the 93 antibodies, 20 antibodies showing excellent binding affinities ($K_D$ values) were selected and summarized in Table 3. Among them, SPR sensorgrams for antibody B3 showing the LILRB1 binding affinity ($K_D$) of about 99.8 nM and for antibody E3 showing the LILRB1 binding affinity ($K_D$) of about 101.2 nM are shown in Figs. 2 and 3, respectively (Fig. 2: SPR sensorgram for B3, Fig. 3: SPR sensorgram for E3):

[Table 3]

| Antigen Binding Affinities ($K_D$) of Anti-LILRB1 antibodies to human LILRB1 | | | |
|---|---|---|---|
| Clone name | $k_a$ (x $10^5$) (1/Ms) | $k_d$ (x $10^{-4}$) (1/s) | $K_D$ (nM) |
| A10 | 0.504 | 76.5 | 152 |
| A11 | 0.001801 | 9.814 | 5448 |

(continued)

| Antigen Binding Affinities ($K_D$) of Anti-LILRB1 antibodies to human LILRB1 | | | |
|---|---|---|---|
| Clone name | $k_a$ (x 10^5) (1/Ms) | $k_d$ (x 10^-4) (1/s) | $K_D$ (nM) |
| B3 | 0.149 | 14.87 | 99.8 |
| B9 | 0.09324 | 6.16 | 66.1 |
| B12 | 1.84 | 14.42 | 7.84 |
| D1 | 1.165 | 57.44 | 49.33 |
| D3 | 0.0311 | 5.58 | 180 |
| E3 | 0.3460 | 35.00 | 101.2 |
| E4 | 0.1065 | 7.73 | 72.55 |
| E6 | 0.2679 | 16.27 | 60.73 |
| E9 | 0.105 | 10.48 | 99.86 |
| E12 | 2.331 | 102.6 | 44.01 |
| F11 | 2.72 | 6.15 | 2.26 |
| F12 | 2.811 | 9.731 | 3.462 |
| G1 | 4.33 | 14.19 | 3.28 |
| G6 | 2.58 | 152.4 | 59.06 |
| G9 | 1.43 | 4.36 | 3.05 |
| G11 | 0.454 | 20.53 | 45.23 |
| H2 | 5.865 | 95 | 16.20 |
| H11 | 2.962 | 22.57 | 7.621 |

**1.6. Sequence analysis of the selected antibodies**

In the 20 antibodies which are analyzed for antigen binding affinity in Example 1.5, amino acid sequences of the CDRs defined according to Kabat numbering, light chain variable region, heavy chain variable region, light chain, and heavy chain, and nucleic acid sequence encoding the light chain variable region and the heavy chain variable region were analyzed by general amino acid sequencing and DNA sequencing methods and summarized in Tables 4-23:

[Table 4]

| Antibody clone E3 | | |
|---|---|---|
| | Amino acid sequence (N→C) / Nucleic acid sequence (5'→3') | SEQ ID NO |
| CDR-L 1 | QGDSLRNFYAS | 1 |
| CDR-L2 | GKNNRPS | 2 |
| CDR-L3 | NSRDSSGSHLTGV | 3 |
| CDR-H1 | SYAMS | 4 |
| CDR-H2 | AISGSGGSTYYADSVKG | 5 |
| CDR-H3 | DTYYYGSGRSNAFDI | 6 |
| light chain variable region | SYELTQDPAVSVALGQTVRITCQGDSLRNFYASWYQQKSG QAPVLVMYGKNNRPSGIPDRFSGSTSGNTASLTITGAQAE DEADYYCNSRDSSGSHLTGVFGGGTKVTVLGQPAAA | 221 |

(continued)

| Antibody clone E3 | | |
|---|---|---|
| | Amino acid sequence (N→C) / Nucleic acid sequence (5'→3') | SEQ ID NO |
| light chain variable region coding gene | TCCTATGAGCTGACTCAGGACCCTGCTGTGTCTGTGGC CTTGGGACAGACAGTCAGGATCACATGCCAGGGAGACA GCCTCAGAAACTTTTATGCAAGCTGGTACCAGCAGAAGT CAGGACAGGCCCCAGTTCTTGTCATGTATGGTAAAAACA ACCGGCCCTCAGGGATCCCAGACCGATTCTCTGGCTCC ACCTCAGGAAACAGCTTCCTTGACCATCACTGGGGGC TCAGGCGGAAGATGAGGCTGACTATTACTGTAACTCCCG GGACAGCAGTGGTAGCCATTTGACGGGCGTATTCGGCG GAGGGACCAAGGTCACCGTCCTAGGTCAGCCCGCGGC CGCA | 261 |
| heavy chain variable region | QVQLVESGGGLVQPGGSLRLSCAASGFTFSSYAMSWVR QAPGKGLEWVSAISGSGGSTYYADSVKGRFTISRDNSKN TLYLQMISLRAEDTAVYYCARDTYYYGSGRSNAFDIWGQG TLVTVSS | 222 |
| heavy chain variable region coding gene | CAGGTGCAGCTGGTGGAGTCTGGGGGAGGCTTGGTAC AGCCTGGGGGGTCCCTGAGACTCTCCTGTGCAGCCTC TGGATTCACCTTTAGCAGCTATGCCATGAGCTGGGTCCG CCAGGCTCCAGGGAAGGGGCTGGAGTGGGTCTCAGCT ATTAGTGGTAGTGGTGGTAGCACATACTACGCAGACTCC GTGAAGGGCCGGTTCACCATCTCCAGAGACAATTCCAA GAATACGCTGTATCTGCAAATGATTAGCCTGAGAGCTGA GGACACGGCTGTGTATTACTGTGCGAGAGATACGTATTA CTATGGTTCGGGGAGAAGTAATGCTTTTGATATATGGGG CCAGGGAACCCTGGTCACCGTCTCGAGT | 262 |
| light chain (Lambda ) | SYELTQDPAVSVALGQTVRITCQGDSLRNFYASWYQQKSG QAPVLVMYGKNNRPSGIPDRFSGSTSGNTASLTITGAQAE DEADYYCNSRDSSGSHLTGVFGGGTKVTVLGQPAAAPSV TLFPPSSEELQANKATLVCLISDFYPGAVTVAWKADSSPV KAGVETTTPSKQSNNKYAASSYLSLTPEQWKSHRSYSCQ VTHEGSTVEKTVAPTECS | 301 |

(continued)

| Antibody clone E3 | | |
|---|---|---|
| | Amino acid sequence (N→C) / Nucleic acid sequence (5'→3') | SEQ ID NO |
| heavy chain | QVQLVESGGGLVQPGGSLRLSCAASGFTFSSYAMSWVR QAPGKGLEWVSAISGSGGSTYYADSVKGRFTISRDNSKN TLYLQMISLRAEDTAVYYCARDTYYYGSGRSNAFDIWGQG TLVTVSSASTKGPSVFPLAPCSRSTSESTAALGCLVKDYF PEPVTVSWNSGALTSGVHTFPAVLQSSGLYSLSSVVTVPS SSLGTKTYTCNVDHKPSNTKVDKRVESKYGPPCPPCPAP EFLGGPSVFLFPPKPKDTLMISRTPEVTCVVVDVSQEDPE VQFNWYVDGVEVHNAKTKPREEQFNSTYRVVSVLTVLHQ DWLNGKEYKCKVSNKGLPSSIEKTISKAKGQPREPQVYTL PPSQEEMTKNQVSLTCLVKGFYPSDIAVEWESNGQPENN YKTTPPVLDSDGSFFLYSRLTVDKSRWQEGNVFSCSVMH EALHNHYTQKSLSLSLGK | 302 |

[Table 5]

| Antibody clone B3 | | |
|---|---|---|
| | Amino acid sequence (N→C) / Nucleic acid sequence (5'→3') | SEQ ID NO |
| CDR-L 1 | QASQDISNYLN | 7 |
| CDR-L2 | DASNLET | 8 |
| CDR-L3 | QQYDNLP | 9 |
| CDR-H1 | DYAMH | 10 |
| CDR-H2 | GISWNSGSIGYADSVKG | 11 |
| CDR-H3 | VGDSSGWSDAFDI | 12 |
| light chain variable region | DIQMTQSPSSLSASVGDRVTITCQASQDISNYLNWYQQKP GKAPKLLIYDASNLETGVPSRFSGSGSGTDFTFTISSLQPE DIATYYCQQYDNLPFGGGTKVDIKRTAAA | 223 |
| light chain variable region coding gene | GACATCCAGATGACCCAGTCTCCATCCTCCCTGTCTGCA TCTGTAGGAGACAGAGTCACCATCACTTGCCAGGCGAGT CAGGACATTAGCAACTATTTGAATTGGTATCAGCAGAAAC CAGGGAAAGCCCCTAAGCTCCTGATCTACGATGCATCCA ATTTGGAAACAGGGGTCCCATCAAGGTTCAGTGGAAGTG GATCTGGGACAGATTTTACTTTCACCATCAGCAGCCTGCA GCCTGAAGATATTGCAACATATTACTGTCAACAGTATGATA ATCTCCCTTTCGGCGGAGGGACCAAAGTGGATATCAAAC GTACCGCGGCCGCA | 263 |

(continued)

| Antibody clone B3 | | |
|---|---|---|
| | Amino acid sequence (N→C) / Nucleic acid sequence (5'→3') | SEQ ID NO |
| heavy chain variable region | EVQLLESGGGLVQPGRSLRLSCAASGFTFDDYAMHWVRQ APGKGLEWVSGISWNSGSIGYADSVKGRFTISRDNSKNTLY LQMNSLRAEDTAVYYCARVGDSSGWSDAFDIWGQGTMVT VSS | 224 |
| heavy chain variable region coding gene | GAGGTGCAGCTGTTGGAGTCTGGGGGAGGCTTGGTACA GCCTGGCAGGTCCCTGAGACTCTCCTGTGCAGCCTCTG GATTCACCTTTGATGATTATGCCATGCACTGGGTCCGGCA AGCTCCAGGGAAGGGCCTGGAGTGGGTCTCAGGTATTA GTTGGAATAGTGGTAGCATAGGCTACGCAGACTCCGTGA AGGGCCGATTCACCATCTCCAGAGACAATTCCAAGAACA CGCTGTATCTTCAAATGAACAGTCTGAGAGCCGAGGACA CGGCCGTGTATTACTGTGCGAGAGTTGGGGATAGCAGTG GCTGGTCCGATGCTTTTGATATCTGGGGCCAAGGGACAA TGGTCACCGTCTCGAGT | 264 |
| light chain (Kappa) | DIQMTQSPSSLSASVGDRVTITCQASQDISNYLNWYQQKP GKAPKLLIYDASNLETGVPSRFSGSGSGTDFTFTISSLQPE DIATYYCQQYDNLPFGGGTKVDIKRTAAAPSVFIFPPSDEQ LKSGTASVVCLLNNFYPREAKVQWKVDNALQSGNSQESVT EQDSKDSTYSLSSTLTLSKADYEKHKLYACEVTHQGLSSPV TKSFNRGEC | 303 |
| heavy chain | EVQLLESGGGLVQPGRSLRLSCAASGFTFDDYAMHWVRQ APGKGLEWVSGISWNSGSIGYADSVKGRFTISRDNSKNTLY LQMNSLRAEDTAVYYCARVGDSSGWSDAFDIWGQGTMVT VSSASTKGPSVFPLAPCSRSTSESTAALGCLVKDYFPEPVT VSWNSGALTSGVHTFPAVLQSSGLYSLSSVVTVPSSSLGT KTYTCNVDHKPSNTKVDKRVESKYGPPCPPCPAPEFLGGP SVFLFPPKPKDTLMISRTPEVTCVVVDVSQEDPEVQFNWY VDGVEVHNAKTKPREEQFNSTYRVVSVLTVLHQDWLNGK EYKCKVSNKGLPSSIEKTISKAKGQPREPQVYTLPPSQEEM TKNQVSLTCLVKGFYPSDIAVEWESNGQPENNYKTTPPVL DSDGSFFLYSRLTVDKSRWQEGNVFSCSVMHEALHNHYT QKSLSLSLGK | 304 |

[Table 6]

| Antibody clone A10 | | |
|---|---|---|
| | Amino acid sequence (N→C) / Nucleic acid sequence (5'→3') | SEQ ID NO |
| CDR-L 1 | RASQSVSSNLA | 13 |
| CDR-L2 | GASTRAT | 14 |

(continued)

| Antibody clone A10 | | |
|---|---|---|
| | Amino acid sequence (N→C) / Nucleic acid sequence (5'→3') | SEQ ID NO |
| CDR-L3 | QQYGSSPRMYT | 15 |
| CDR-H1 | SYAIS | 16 |
| CDR-H2 | GIIPIFGTANYAQKFQG | 17 |
| CDR-H3 | GGLGELDNWFDP | 18 |
| light chain variable region | DIVMTQSPATLSVSPGERATLSCRASQSVSSNLAWYQQKPGQAPRLLIYGASTRATGIPARFSGSGSGTEFTLTISSLQSEDFAVYYCQQYGSSPRMYTFGQGTKVDIKRTAAA | 225 |
| light chain variable region coding gene | GATATTGTGATGACACAGTCTCCAGCCACCCTGTCTGTGTCTCCAGGGGAAAGAGCCACCCTCTCCTGCAGGGCCAGTCAGAGTGTTAGCAGCAACTTAGCCTGGTACCAGCAGAAACCTGGCCAGGCTCCCAGGCTCCTCATCTATGGTGCATCCACCAGGGCCACCGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATGGTAGCTCACCTCGGATGTACACTTTTGGCCAGGGGACCAAAGTGGATATCAAACGTACCGCGGCCGCA | 265 |
| heavy chain variable region | QMQLVQSGAEVKKPGSSVKVSCKASGGTFSSYAISWVRQAPGQGLEWMGGIIPIFGTANYAQKFQGRVTITADKSISTAYMELSSLRSEDTAVYYCARGGLGELDNWFDPWGQGTLVTVSS | 226 |
| heavy chain variable region coding gene | CAAATGCAGCTGGTGCAGTCTGGGGGCTGAGGTGAAGAAGCCTGGGTCCTCGGTGAAGGTCTCCTGCAAGGCTTCTGGAGGCACCTTCAGCAGCTATGCTATCAGCTGGGTGCGACAGGCCCCTGGACAAGGGCTTGAGTGGATGGGTGGGATCATCCCTATCTTTGGTACAGCAAACTACGCACAGAAGTTCCAGGGCAGAGTCACGATTACCGCGGACAAATCCATCAGCACAGCCTACATGGAGCTGAGCAGCCTGAGATCTGAGGACACGGCCGTGTATTACTGTGCGAGAGGCGGCCTCGGGGAGTTGGACAACTGGTTCGACCCCTGGGGCCAGGGAACCCTGGTCACCGTCTCGAGT | 266 |
| light chain (Kappa) | DIVMTQSPATLSVSPGERATLSCRASQSVSSNLAWYQQKPGQAPRLLIYGASTRATGIPARFSGSGSGTEFTLTISSLQSEDFAVYYCQQYGSSPRMYTFGQGTKVDIKRTAAAPSVFIFPPSDEQLKSGTASVVCLLNNFYPREAKVQWKVDNALQSGNSQESVTEQDSKDSTYSLSSTLTLSKADYEKHKLYACEVTHQGLSSPVTKSFNRGEC | 305 |

(continued)

| Antibody clone A10 | | |
|---|---|---|
| | Amino acid sequence (N→C) / Nucleic acid sequence (5'→3') | SEQ ID NO |
| heavy chain | QMQLVQSGAEVKKPGSSVKVSCKASGGTFSSYAISWVRQ APGQGLEWMGGIIPIFGTANYAQKFQGRVTITADKSISTAYM ELSSLRSEDTAVYYCARGGLGELDNWFDPWGQGTLVTVSS ASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKS LSLSLGK | 306 |

[Table 7]

| Antibody clone G1 | | |
|---|---|---|
| | Amino acid sequence (N→C) / Nucleic acid sequence (5'→3') | SEQ ID NO |
| CDR-L 1 | SGYKLGDRYVS | 19 |
| CDR-L2 | KDSQRPS | 20 |
| CDR-L3 | QAWDSGTGV | 21 |
| CDR-H1 | SYGIS | 22 |
| CDR-H2 | WISAYNGNTNYAQELQG | 23 |
| CDR-H3 | VGVAGKLDY | 24 |
| light chain variable region | SYELTQPPSLSVSPGQTASITCSGYKLGDRYVSWYQQKTG QSPVVVIYKDSQRPSGVPERFSGSNSGNTATLTISGTQAMD EADYYCQAWDSGTGVFGGGTKLTVLGQPAAA | 227 |
| light chain variable region coding gene | TCCTATGAGCTGACTCAGCCACCCTCACTGTCCGTGTCC CCAGGACAGACAGCCAGCATCACCTGCTCAGGATATAAA CTGGGAGATAGATATGTTTCCTGGTATCAGCAGAAGACAG GCCAGTCCCCTGTGGTGGTCATCTATAAAGATAGCCAGC GGCCCTCAGGGGTCCCTGAACGATTCTCTGGCTCCAAC TCTGGGAACACAGCCACTCTGACCATCAGCGGGACCCA GGCTATGGATGAGGCTGACTATTACTGTCAGGCGTGGGA CAGCGGCACTGGGGTATTCGGCGGAGGGACCAAGCTGA CCGTCCTAGGTCAGCCCGCGGCCGCA | 267 |
| heavy chain variable region | EVQLVQSGAEVKKPGSSVKVSCKASGGTFSSYGISWVRQA PGQGLEWMGWISAYNGNTNYAQELQGRVTMTTDTSTSTA YMELRSLRSDDTAVYYCARVGVAGKLDYWGQGTLVTVSS | 228 |

(continued)

| Antibody clone G1 | | |
|---|---|---|
| | Amino acid sequence (N→C) / Nucleic acid sequence (5'→3') | SEQ ID NO |
| heavy chain variable region coding gene | GAAGTGCAGCTGGTGCAGTCTGGGGCTGAGGTGAAGAA GCCTGGGTCCTCGGTGAAGGTCTCCTGCAAGGCTTCTG GAGGCACCTTCAGCAGCTATGGTATCAGCTGGGTGCGAC AGGCCCCTGGACAAGGGCTTGAGTGGATGGGATGGATC AGCGCTTACAATGGTAACACAAACTATGCACAGGAGCTC CAGGGCAGAGTCACCATGACCACAGACACATCCACGAG CACAGCCTATATGGAGCTGAGGAGCCTGAGATCTGACGA CACGGCCGTGTATTACTGTGCGAGAGTAGGGGTGGCTG GTAAACTTGACTACTGGGGCCAAGGAACCCTGGTCACCG TCTCGAGT | 268 |
| light chain (Lambd a) | SYELTQPPSLSVSPGQTASITCSGYKLGDRYVSWYQQKTG QSPVVVIYKDSQRPSGVPERFSGSNSGNTATLTISGTQAMD EADYYCQAWDSGTGVFGGGTKLTVLGQPAAAPSVTLFPPS SEELQANKATLVCLISDFYPGAVTVAWKEDSSPVKAGVETT TPSKQSNNKYAASSYLSLTPEQWKSHRSYSCQVTHEGSTV EKTVAPTECS | 307 |
| heavy chain | EVQLVQSGAEVKKPGSSVKVSCKASGGTFSSYGISWVRQA PGQGLEWMGWISAYNGNTNYAQELQGRVTMTTDTSTSTA YMELRSLRSDDTAVYYCARVGVAGKLDYWGQGTLVTVSSA STKGPSVFPLAPCSRSTSESTAALGCLVKDYFPEPVTVSW NSGALTSGVHTFPAVLQSSGLYSLSSVVTVPSSSLGTKTYT CNVDHKPSNTKVDKRVESKYGPPCPPCPAPEFLGGPSVFL FPPKPKDTLMISRTPEVTCVVVDVSQEDPEVQFNWYVDGV EVHNAKTKPREEQFNSTYRVVSVLTVLHQDWLNGKEYKCK VSNKGLPSSIEKTISKAKGQPREPQVYTLPPSQEEMTKNQV SLTCLVKGFYPSDIAVEWESNGQPENNYKTTPPVLDSDGS FFLYSRLTVDKSRWQEGNVFSCSVMHEALHNHYTQKSLSL SLGK | 308 |

[Table 8]

| Antibody clone G9 | | |
|---|---|---|
| | Amino acid sequence (N→C) / Nucleic acid sequence (5'→3') | SEQ ID NO |
| CDR-L 1 | TGSSSDVGGYNYVS | 25 |
| CDR-L2 | DVSNRPS | 26 |
| CDR-L3 | SSYTGSSTLDVL | 27 |
| CDR-H1 | SYWIG | 28 |
| CDR-H2 | IIYPGDSDTRYSPSFQG | 29 |

(continued)

| Antibody clone G9 | | |
|---|---|---|
| | Amino acid sequence (N→C) / Nucleic acid sequence (5'→3') | SEQ ID NO |
| CDR-H3 | QYYDGGYYMDV | 30 |
| light chain variable region | QSALTQPASVSGSPGQSITISCTGSSSDVGGYNYVSWYQQ HPGKAPKLMIYDVSNRPSGVSDRFSGSKSGNMASLTISGL QAEDEADYYCSSYTGSSTLDVLFGGGTKLTVLGQPAAA | 233 |
| light chain variable region coding gene | CAGTCTGCGCTGACTCAGCCTGCCTCCGTGTCTGGGTC TCCTGGACAGTCGATCACCATCTCCTGCACTGGAAGCAG CAGTGACGTTGGTGGTTATAACTATGTCTCCTGGTACCAG CAACACCCAGGCAAAGCCCCCAAACTCATGATTTATGATG TCAGTAATCGGCCCTCAGGGGTTTCTGATCGCTTCTCTG GCTCCAAGTCTGGCAACATGGCCTCCCTGACCATCTCTG GGCTCCAGGCTGAGGACGAGGCTGATTATTACTGCAGCT CATATACAGGAAGCAGCACTCTCGACGTGCTATTCGGCG GAGGGACCAAGCTGACCGTCCTAGGTCAGCCCGCGGCC GCA | 269 |
| heavy chain variable region | QVQLVQPGAEVKKPGESLKISCKGSGYSFTSYWIGWVRQ MPGKGLEWMGIIYPGDSDTRYSPSFQGQVTISADKSISTAY LQWSSLKASDTAMYYCASQYYDGGYYMDVWGQGTLVTVS S | 234 |
| heavy chain variable region coding gene | CAGGTGCAGCTGGTGCAGCCTGGAGCAGAGGTGAAAAA GCCGGGGGGAGTCTCTGAAGATCTCCTGTAAGGGTTCTG GATACAGCTTTACCAGCTACTGGATCGGCTGGGTGCGCC AGATGCCCGGGAAGGGCCTGGAGTGGATGGGGATCATC TATCCTGGTGACTCTGATACCAGATACAGCCCGTCCTTCC AAGGCCAGGTCACCATCTCAGCCGACAAGTCCATCAGCA CCGCCTACCTGCAGTGGAGCAGCCTGAAGGCCTCGGAC ACCGCCATGTATTACTGTGCGAGTCAATATTACGATGGGG GTTACTACATGGACGTCTGGGGCCAGGGAACCCTGGTC ACCGTCTCGAGT | 270 |
| light chain (Lambd a) | QSALTQPASVSGSPGQSITISCTGSSSDVGGYNYVSWYQQ HPGKAPKLMIYDVSNRPSGVSDRFSGSKSGNMASLTISGL QAEDEADYYCSSYTGSSTLDVLFGGGTKLTVLGQPAAAPS VTLFPPSSEELQANKATLVCLISDFYPGAVTVAWKADSSPV KAGVETTTPSKQSNNKYAASSYLSLTPEQWKSHRSYSCQV THEGSTVEKTVAPTECS | 309 |

(continued)

| Antibody clone G9 | | |
|---|---|---|
| | Amino acid sequence (N→C) / Nucleic acid sequence (5'→3') | SEQ ID NO |
| heavy chain | QVQLVQPGAEVKKPGESLKISCKGSGYSFTSYWIGWVRQ MPGKGLEWMGIIYPGDSDTRYSPSFQGQVTISADKSISTAY LQWSSLKASDTAMYYCASQYYDGGYYMDVWGQGTLVTVS SASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKS LSLSLGK | 310 |

[Table 9]

| Antibody clone H2 | | |
|---|---|---|
| | Amino acid sequence (N→C) / Nucleic acid sequence (5'→3') | SEQ ID NO |
| CDR-L 1 | QGDSLRNYYAS | 31 |
| CDR-L2 | GNNKRPS | 32 |
| CDR-L3 | NSLDSTYNHPI | 33 |
| CDR-H1 | SYDIH | 34 |
| CDR-H2 | WISAYNGNTNYAQKLQG | 35 |
| CDR-H3 | DGGDAFDI | 36 |
| light chain variable region | SYELTQDPAVSVALGQTVRITCQGDSLRNYYASWYQQKPG QAPILVISGNNKRPSGIPDRFSGSSSGDTASLTISGAQAEDE ADYYCNSLDSTYNHPIFGGGTKVTVLGQPAAA | 235 |
| light chain variable region coding gene | TCCTATGAGCTGACTCAGGACCCTGCTGTGTCGGTGGCC TTGGGACAGACAGTCAGGATCACATGCCAAGGAGACAG CCTCAGAAACTATTATGCAAGCTGGTACCAGCAGAAGCC AGGACAGGCCCCTATTCTTGTCATCTCTGGTAACAACAAA CGGCCCTCGGGGATCCCAGACCGATTCTCTGGCTCCAG CTCAGGAGACACAGCTTCCTTGACCATCTCTGGGGCTCA GGCGGAAGATGAGGCTGACTATTACTGTAACTCCCTAGA CAGCACTTATAACCATCCGATATTCGGCGGAGGGACCAA GGTCACCGTCCTAGGTCAGCCCGCGGCCGCA | 271 |
| heavy chain variable region | QVQLVQSGAEVKKPGASVKVSCKASGYTFTSYDIHWVRQA TGQGLEWMGWISAYNGNTNYAQKLQGRVTMTTDTSTSTA YMELRSLRSDDTAVYYCARDGGDAFDIWGQGTLVTVSS | 236 |

(continued)

| Antibody clone H2 | | |
|---|---|---|
| | Amino acid sequence (N→C) / Nucleic acid sequence (5'→3') | SEQ ID NO |
| heavy chain variable region coding gene | CAGGTCCAGCTTGTGCAGTCTGGGGCTGAGGTGAAGAA GCCTGGGGCCTCAGTGAAGGTCTCCTGCAAGGCTTCTG GATACACCTTCACCAGTTATGATATCCACTGGGTGCGACA GGCCACTGGACAAGGGCTTGAGTGGATGGGATGGATCA GCGCTTACAATGGTAACACAAACTATGCACAGAAGCTCCA GGGCAGAGTCACCATGACCACAGACACATCCACGAGCA CAGCCTACATGGAGCTGAGGAGCCTGAGATCTGACGAC ACGGCCGTGTATTACTGTGCGAGAGATGGGGGTGATGCT TTTGATATCTGGGGCCAAGGAACCCTGGTCACCGTCTCG AGT | 272 |
| light chain (Lambd a) | SYELTQDPAVSVALGQTVRITCQGDSLRNYYASWYQQKPG QAPILVISGNNKRPSGIPDRFSGSSSGDTASLTISGAQAEDE ADYYCNSLDSTYNHPIFGGGTKVTVLGQPAAAPSVTLFPPS SEELQANKATLVCLISDFYPGAVTVAWKADSSPVKAGVETT TPSKQSNNKYAASSYLSLTPEQWKSHRSYSCQVTHEGSTV EKTVAPTECS | 311 |
| heavy chain | QVQLVQSGAEVKKPGASVKVSCKASGYTFTSYDIHWVRQA TGQGLEWMGWISAYNGNTNYAQKLQGRVTMTTDTSTSTA YMELRSLRSDDTAVYYCARDGGDAFDIWGQGTLVTVSSAS TKGPSVFPLAPCSRSTSESTAALGCLVKDYFPEPVTVSWN SGALTSGVHTFPAVLQSSGLYSLSSVVTVPSSSLGTKTYTC NVDHKPSNTKVDKRVESKYGPPCPPCPAPEFLGGPSVFLF PPKPKDTLMISRTPEVTCVVVDVSQEDPEVQFNWYVDGVE VHNAKTKPREEQFNSTYRVVSVLTVLHQDWLNGKEYKCKV SNKGLPSSIEKTISKAKGQPREPQVYTLPPSQEEMTKNQVS LTCLVKGFYPSDIAVEWESNGQPENNYKTTPPVLDSDGSF FLYSRLTVDKSRWQEGNVFSCSVMHEALHNHYTQKSLSLS LGK | 312 |

[Table 10]

| Antibody clone H11 | | |
|---|---|---|
| | Amino acid sequence (N→C) / Nucleic acid sequence (5'→3') | SEQ ID NO |
| CDR-L 1 | QGDSLRSYYAS | 37 |
| CDR-L2 | GRNNRPS | 38 |
| CDR-L3 | KSRDSSGNHYV | 39 |
| CDR-H1 | SYYMH | 40 |
| CDR-H2 | IINPSGGSTSYAQKFQG | 41 |
| CDR-H3 | DAGSSSDY | 42 |

(continued)

| Antibody clone H11 | | |
|---|---|---|
| | Amino acid sequence (N→C) / Nucleic acid sequence (5'→3') | SEQ ID NO |
| light chain variable region | SYELTQDPAASVALGQTVRITCQGDSLRSYYASWYQQKPG QAPVVVIYGRNNRPSGIPDRFSGSSSGDTASLTITGAQAED EADYYCKSRDSSGNHYVFGTGTKLTVLGQPAAA | 231 |
| light chain variable region coding gene | TCCTATGAGCTGACTCAGGACCCTGCTGCGTCTGTGGCC TTGGGACAGACAGTCAGGATCACATGCCAAGGAGACAG CCTCAGAAGCTATTATGCAAGCTGGTACCAGCAGAAGCC AGGACAGGCCCCTGTAGTTGTCATCTATGGTAGAAACAA CCGGCCCTCAGGGATCCCAGACCGATTCTCTGGCTCCA GCTCAGGAGACAGCTTCCTTGACCATCACTGGGGCT CAGGCGGAAGATGAGGCTGACTATTACTGTAAGTCCCGG GACAGCAGTGGTAACCATTATGTCTTCGGAACTGGGACC AAGCTGACCGTCCTAGGTCAGCCCGCGGCCGCA | 273 |
| heavy chain variable region | QVQLVQSGAEVKKPGASVKVSCKASGYTFTSYYMHWVRQ APGQGLEWMGIINPSGGSTSYAQKFQGRVTMTRDTSTSTV YMELSSLRSEDTAVYYCARDAGSSSDYWGRGTLVTVSS | 232 |
| heavy chain variable region coding gene | CAGGTGCAGCTGGTGCAGTCTGGGGCTGAGGTGAAGAA GCCTGGGGCCTCAGTGAAGGTTTCCTGCAAGGCATCTG GATACACCTTCACCAGCTACTATATGCACTGGGTGCGACA GGCCCCTGGACAAGGGCTTGAGTGGATGGGAATAATCAA CCCTAGTGGTGGTAGCACAAGCTACGCACAGAAGTTCCA GGGCAGAGTCACCATGACCAGGGACACGTCCACGAGCA CAGTCTACATGGAGCTGAGCAGCCTGAGATCTGAGGACA CGGCCGTGTATTACTGTGCGAGAGATGCCGGCAGCTCG TCCGATTACTGGGGCCGTGGCACCCTGGTCACCGTCTC GAGT | 274 |
| light chain (Lambd a) | SYELTQDPAASVALGQTVRITCQGDSLRSYYASWYQQKPG QAPVVVIYGRNNRPSGIPDRFSGSSSGDTASLTITGAQAED EADYYCKSRDSSGNHYVFGTGTKLTVLGQPAAAPSVTLFP PSSEELQANKATLVCLISDFYPGAVTVAWKADSSPVKAGVE TTTPSKQSNNKYAASSYLSLTPEQWKSHRSYSCQVTHEGS TVEKTVAPTECS | 313 |

(continued)

| Antibody clone H11 | | |
|---|---|---|
| | Amino acid sequence (N→C) / Nucleic acid sequence (5'→3') | SEQ ID NO |
| heavy chain | QVQLVQSGAEVKKPGASVKVSCKASGYTFTSYYMHWVRQ APGQGLEVWMGIINPSGGSTSYAQKFQGRVTMTRDTSTSTV YMELSSLRSEDTAVYYCARDAGSSSDYWGRGTLVTVSSAS TKGPSVFPLAPCSRSTSESTAALGCLVKDYFPEPVTVSWN SGALTSGVHTFPAVLQSSGLYSLSSVVTVPSSSLGTKTYTC NVDHKPSNTKVDKRVESKYGPPCPPCPAPEFLGGPSVFLF PPKPKDTLMISRTPEVTCVVVDVSQEDPEVQFNWYVDGVE VHNAKTKPREEQFNSTYRVVSVLTVLHQDWLNGKEYKCKV SNKGLPSSIEKTISKAKGQPREPQVYTLPPSQEEMTKNQVS LTCLVKGFYPSDIAVEWESNGQPENNYKTTPPVLDSDGSF FLYSRLTVDKSRWQEGNVFSCSVMHEALHNHYTQKSLSLS LGK | 314 |

[Table 11]

| Antibody clone F12 | | |
|---|---|---|
| | Amino acid sequence (N→C) / Nucleic acid sequence (5'→3') | SEQ ID NO |
| CDR-L 1 | AGTSSDIGDYDYVS | 43 |
| CDR-L2 | DVSRRPS | 44 |
| CDR-L3 | ASYTSSSVVV | 45 |
| CDR-H1 | SYWIG | 46 |
| CDR-H2 | IIYPGDSDTRYSPSFQG | 47 |
| CDR-H3 | QYYDGGYYMDV | 48 |
| light chain variable region | QSVLTQPASVSGSPGQSITISCAGTSSDIGDYDYVSWYQQH PGKTPKLMIYDVSRRPSGVPDRFSGSKSGNTASLTISGLQT EDEADYYCASYTSSSVVVFGGGTKLTVLGQPAAA | 237 |
| light chain variable region coding gene | CAGTCTGTGCTGACTCAGCCTGCCTCCGTGTCTGGGTCT CCTGGACAGTCGATCACCATCTCCTGCGCTGGAACCAGC AGTGACATTGGTGATTATGACTATGTCTCCTGGTACCAAC AGCACCCAGGCAAGACTCCCAAACTCATGATTTATGATGT CAGTAGGCGGCCCTCAGGGGTCCCTGATCGCTTCTCTG GCTCCAAGTCTGGCAACACGGCCTCCCTGACCATCTCTG GGCTCCAGACTGAGGACGAGGCTGATTATTACTGCGCCT CATATACAAGCAGCAGCGTCGTGGTCTTCGGCGGAGGG ACCAAGCTGACCGTCCTAGGTCAGCCCGCGGCCGCA | 275 |

(continued)

| Antibody clone F12 | | |
|---|---|---|
| | Amino acid sequence (N→C) / Nucleic acid sequence (5'→3') | SEQ ID NO |
| heavy chain variable region | QVQLVQSGAEVKKPGESLKISCKGSGYSFTSYWIGWVRQ MPGKGLEWMGIIYPGDSDTRYSPSFQGQVTISADKSISTAY LQWSSLKASDTAMYYCASQYYDGGYYMDVWGQGTLVTVS S | 238 |
| heavy chain variable region coding gene | CAGGTGCAGCTGGTGCAGTCTGGAGCAGAGGTGAAAAA GCCCGGGGGAGTCTCTGAAGATCTCCTGTAAGGGTTCTG GATACAGCTTTACCAGCTACTGGATCGGCTGGGTGCGCC AGATGCCCGGGAAAGGCCTGGAGTGGATGGGGATCATC TATCCTGGTGACTCTGATACCAGATACAGCCCGTCCTTCC AAGGCCAGGTCACCATCTCAGCCGACAAGTCCATCAGCA CCGCCTACCTGCAGTGGAGCAGCCTGAAGGCCTCGGAC ACCGCCATGTATTACTGTGCGAGTCAATATTACGATGGGG GTTACTACATGGACGTCTGGGGCCAGGGCACCCTGGTC ACCGTCTCGAGT | 276 |
| light chain (Lambd a) | QSVLTQPASVSGSPGQSITISCAGTSSDIGDYDYVSWYQQH PGKTPKLMIYDVSRRPSGVPDRFSGSKSGNTASLTISGLQT EDEADYYCASYTSSSVVVFGGGTKLTVLGQPAAAPSVTLFP PSSEELQANKATLVCLISDFYPGAVTVAWKADSSPVKAGVE TTTPSKQSNNKYAASSYLSLTPEQWKSHRSYSCQVTHEGS TVEKTVAPTECS | 315 |
| heavy chain | QVQLVQSGAEVKKPGESLKISCKGSGYSFTSYWIGWVRQ MPGKGLEWMGIIYPGDSDTRYSPSFQGQVTISADKSISTAY LQWSSLKASDTAMYYCASQYYDGGYYMDVWGQGTLVTVS SASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKS LSLSLGK | 316 |

[Table 12]

| Antibody clone B9 | | |
|---|---|---|
| | Amino acid sequence (N→C) / Nucleic acid sequence (5'→3') | SEQ ID NO |
| CDR-L 1 | RASQSISRYLN | 49 |
| CDR-L2 | GASSLQS | 50 |

(continued)

| Antibody clone B9 | | |
|---|---|---|
| | Amino acid sequence (N→C) / Nucleic acid sequence (5'→3') | SEQ ID NO |
| CDR-L3 | QQAYGFPLT | 51 |
| CDR-H1 | SYAIS | 52 |
| CDR-H2 | GIIPIFGTANYAQKFQG | 53 |
| CDR-H3 | GEIAVAQNWDYYGMDV | 54 |
| light chain variable region | DIQMTQSPSSLSASVGDRVTITCRASQSISRYLNWYQQKPGKAPKLLIYGASSLQSGVPSRFSGSGSGTDFTLTISSLQPEDFATYHCQQAYGFPLTLGGGTKVEIKRTAAA | 229 |
| light chain variable region coding gene | GACATCCAGATGACCCAGTCTCCATCCTCCCTGTCTGCATCTGTAGGAGACAGAGTCACCATCACTTGCCGGGCAAGTCAGAGCATTAGCAGGTATTTAAATTGGTATCAGCAGAAACCAGGGAAAGCCCCCAAGCTCCTGATCTATGGTGCATCCAGTTTGCAAAGTGGGGTCCCATCAAGGTTCAGTGGCAGTGGATCTGGGACAGATTTCACTCTCACCATCAGCAGTCTGCAGCCTGAAGATTTCGCAACTTACCATTGTCAACAGGCTTACGGTTTCCCCCTCACTCTCGGCGGAGGGACCAAGGTGGAGATCAAACGTACCGCGGCCGCA | 277 |
| heavy chain variable region | QVQLVESGAEVKKPGSSVKVSCKASGGTFSSYAISWVRQAPGQGLEWMGGIIPIFGTANYAQKFQGRVTITADESTSTAYMELSSLRSEDTAVYYCARGEIAVAQNWDYYGMDVWGQGTLVTVSS | 230 |
| heavy chain variable region coding gene | CAGGTGCAGCTGGTGGAGTCTGGGGCTGAGGTGAAGAAGCCTGGGTCCTCGGTGAAGGTCTCCTGCAAGGCTTCTGGAGGCACCTTCAGCAGCTATGCTATCAGCTGGGTGCGACAGGCCCCTGGACAAGGGCTTGAGTGGATGGGAGGGATCATCCCTATCTTTGGTACAGCAAACTACGCACAGAAGTTCCAGGGCAGAGTCACGATTACCGCGGACGAATCCACGAGCACAGCCTACATGGAGCTGAGCAGCCTGAGATCTGAGGACACGGCCGTGTATTACTGTGCGAGAGGGGAAATAGCAGTGGCTCAAAACTGGGACTACTACGGTATGGACGTCTGGGGCCAGGGCACCCTGGTCACCGTCTCGAGT | 278 |
| light chain (Kappa) | DIQMTQSPSSLSASVGDRVTITCRASQSISRYLNWYQQKPGKAPKLLIYGASSLQSGVPSRFSGSGSGTDFTLTISSLQPEDFATYHCQQAYGFPLTLGGGTKVEIKRTAAAPSVFIFPPSDEQLKSGTASVVCLLNNFYPREAKVQWKVDNALQSGNSQESVTEQDSKDSTYSLSSTLTLSKADYEKHKLYACEVTHQGLSSPVTKSFNRGEC | 317 |

(continued)

| Antibody clone B9 | | |
|---|---|---|
| | Amino acid sequence (N→C) / Nucleic acid sequence (5'→3') | SEQ ID NO |
| heavy chain | QVQLVESGAEVKKPGSSVKVSCKASGGTFSSYAISWVRQA PGQGLEWMGGIIPIFGTANYAQKFQGRVTITADESTSTAYM ELSSLRSEDTAVYYCARGEIAVAQNWDYYGMDVWGQGTLV TVSSASTKGPSVFPLAPCSRSTSESTAALGCLVKDYFPEPV TVSWNSGALTSGVHTFPAVLQSSGLYSLSSVVTVPSSSLG TKTYTCNVDHKPSNTKVDKRVESKYGPPCPPCPAPEFLGG PSVFLFPPKPKDTLMISRTPEVTCVVVDVSQEDPEVQFNW YVDGVEVHNAKTKPREEQFNSTYRVVSVLTVLHQDWLNG KEYKCKVSNKGLPSSIEKTISKAKGQPREPQVYTLPPSQEE MTKNQVSLTCLVKGFYPSDIAVEWESNGQPENNYKTTPPV LDSDGSFFLYSRLTVDKSRWQEGNVFSCSVMHEALHNHY TQKSLSLSLGK | 318 |

[Table 13]

| Antibody clone G11 | | |
|---|---|---|
| | Amino acid sequence (N→C) / Nucleic acid sequence (5'→3') | SEQ ID NO |
| CDR-L 1 | TGTSSDVGGYNYVS | 55 |
| CDR-L2 | DVSKRPS | 56 |
| CDR-L3 | SSYSSSSTLVV | 57 |
| CDR-H1 | SYWIG | 58 |
| CDR-H2 | IIYPGDSDTRYSPSFQG | 59 |
| CDR-H3 | QYYDGGYYMDV | 60 |
| light chain variable region | QSALTQPRSVSGSPGQSVTISCTGTSSDVGGYNYVSWYQ QHPGKAPKLMIYDVSKRPSGVPDRFSGSKSGNTASLTISGL QAEDEADYYCSSYSSSSTLVVFGGGTKLTVLGQPAAA | 239 |
| light chain variable region coding gene | CAGTCTGCGCTGACTCAGCCTCGCTCAGTGTCCGGGTC TCCTGGACAGTCAGTCACCATCTCCTGCACTGGAACCAG CAGTGATGTTGGTGGTTATAACTATGTCTCCTGGTACCA ACAGCACCCAGGCAAAGCCCCCAAACTCATGATTTATGA TGTCAGTAAGCGGCCCTCAGGGGTCCCTGATCGCTTCT CTGGCTCCAAGTCTGGCAACACGGCCTCCCTGACAATCT CTGGGCTCCAGGCTGAGGACGAGGCTGATTATTACTGC AGCTCATATTCAAGCAGCAGCACTCTCGTGGTTTTCGGC GGAGGGACCAAGCTGACCGTCCTAGGTCAGCCCGCGG CCGCA | 279 |

(continued)

| Antibody clone G11 | | |
|---|---|---|
| | Amino acid sequence (N→C) / Nucleic acid sequence (5'→3') | SEQ ID NO |
| heavy chain variable region | QVQLVQSGAEVKKPGESLKISCKGSGYSFTSYWIGWVRQ MPGKGLEWMGIIYPGDSDTRYSPSFQGQVTISADKSISTAY LQWSSLKASDTAMYYCASQYYDGGYYMDVWGQGTLVTVS S | 240 |
| heavy chain variable region coding gene | CAGGTCCAGCTGGTACAGTCTGGAGCAGAGGTGAAAAA GCCGGGGGAGTCTCTGAAGATCTCCTGTAAGGGTTCTG GATACAGCTTTACCAGCTACTGGATCGGCTGGGTGCGC CAGATGCCCGGGAAAGGCCTGGAGTGGATGGGGATCAT CTATCCTGGTGACTCTGATACCAGATACAGCCCGTCCTT CCAAGGCCAGGTCACCATCTCAGCCGACAAGTCCATCA GCACCGCCTACCTGCAGTGGAGCAGCCTGAAGGCCTCG GACACCGCCATGTATTACTGTGCGAGTCAATATTACGAT GGGGGTTACTACATGGACGTCTGGGGCCAGGGAACCCT GGTCACCGTCTCGAGT | 280 |
| light chain (Lambd a) | QSALTQPRSVSGSPGQSVTISCTGTSSDVGGYNYVSWYQ QHPGKAPKLMIYDVSKRPSGVPDRFSGSKSGNTASLTISGL QAEDEADYYCSSYSSSSTLVVFGGGTKLTVLGQPAAAPSV TLFPPSSEELQANKATLVCLISDFYPGAVTVAWKADSSPVK AGVETTTPSKQSNNKYAASSYLSLTPEQWKSHRSYSCQVT HEGSTVEKTVAPTECS | 319 |
| heavy chain | QVQLVQSGAEVKKPGESLKISCKGSGYSFTSYWIGWVRQ MPGKGLEWMGIIYPGDSDTRYSPSFQGQVTISADKSISTAY LQWSSLKASDTAMYYCASQYYDGGYYMDVWGQGTLVTVS SASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKS LSLSLGK | 320 |

[Table 14]

| Antibody clone G6 | | |
|---|---|---|
| | Amino acid sequence (N→C) / Nucleic acid sequence (5'→3') | SEQ ID NO |
| CDR-L 1 | QGDSLRRYYAT | 61 |
| CDR-L2 | GQNYRPS | 62 |

(continued)

| Antibody clone G6 | | |
|---|---|---|
| | Amino acid sequence (N→C) / Nucleic acid sequence (5'→3') | SEQ ID NO |
| CDR-L3 | NSRDSSGNHVV | 63 |
| CDR-H1 | SYYMH | 64 |
| CDR-H2 | GIIPIFGTANYAQKFQG | 65 |
| CDR-H3 | GWGYSSSFDY | 66 |
| light chain variable region | SYELTQDPAVSVALGQTVTITCQGDSLRRYYATWYQQKPG QAPVLVIYGQNYRPSGIPDRFSGSNSGTTASLTITGAQAED EADYYCNSRDSSGNHVVFGGGTKLTVLGQPAAA | 241 |
| light chain variable region coding gene | TCCTATGAGCTGACTCAGGACCCTGCTGTGTCTGTGGCC TTGGGACAGACAGTCACGATCACATGCCAAGGAGACAG CCTCAGAAGGTATTATGCAACCTGGTACCAGCAGAAGCC AGGACAGGCCCCTGTCCTTGTCATCTATGGTCAAAACTA CCGGCCCTCGGGGATCCCAGACCGATTCTCTGGCTCCA ACTCAGGAACCACAGCTTCCTTGACCATCACTGGGGCTC AGGCGGAAGATGAGGCTGACTATTACTGTAACTCCCGGG ACAGCAGTGGTAACCATGTGGTATTCGGCGGAGGGACC AAGCTGACCGTCCTAGGTCAGCCCGCGGCCGCA | 281 |
| heavy chain variable region | EVQLVESGAEVKKPGASVKVSCKASGYTFTSYYMHWVRQ APGQGLEWMGGIIPIFGTANYAQKFQGRVTITADESTSTAY MELSSLRSEDTAVYYCARGWGYSSSFDYWGQGTTVTVSS | 242 |
| heavy chain variable region coding gene | GAGGTGCAGCTGGTGGAGTCTGGGGCTGAGGTGAAGAA GCCTGGGGCCTCAGTGAAGGTTTCCTGCAAGGCATCTG GATACACCTTCACCAGCTACTATATGCACTGGGTGCGACA GGCCCCTGGACAAGGGCTTGAGTGGATGGGAGGGATCA TCCCTATCTTTGGTACAGCAAACTACGCACAGAAGTTCCA GGGCAGAGTCACGATTACCGCGGACGAATCCACGAGCA CAGCCTACATGGAGCTGAGCAGCCTGAGATCTGAGGAC ACGGCCGTGTACTACTGTGCGAGAGGGTGGGGGTATAG CAGCTCGTTTGACTACTGGGGGCAAGGGACCACGGTCA CCGTCTCGAGT | 282 |
| light chain (Lambd a) | SYELTQDPAVSVALGQTVTITCQGDSLRRYYATWYQQKPG QAPVLVIYGQNYRPSGIPDRFSGSNSGTTASLTITGAQAED EADYYCNSRDSSGNHVVFGGGTKLTVLGQPAAAPSVTLFP PSSEELQANKATLVCLISDFYPGAVTVAWKADSSPVKAGVE TTTPSKQSNNKYAASSYLSLTPEQWKSHRSYSCQVTHEGS TVEKTVAPTECS | 321 |

(continued)

| Antibody clone G6 | | |
|---|---|---|
| | Amino acid sequence (N→C) / Nucleic acid sequence (5'→3') | SEQ ID NO |
| heavy chain | EVQLVESGAEVKKPGASVKVSCKASGYTFTSYYMHWVRQ APGQGLEWMGGIIPIFGTANYAQKFQGRVTITADESTSTAY MELSSLRSEDTAVYYCARGWGYSSSFDYWGQGTTVTVSS ASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKS LSLSLGK | 322 |

[Table 15]

| Antibody clone F11 | | |
|---|---|---|
| | Amino acid sequence (N→C) / Nucleic acid sequence (5'→3') | SEQ ID NO |
| CDR-L 1 | SGSSSNIGTNTVN | 67 |
| CDR-L2 | SNDQRPS | 68 |
| CDR-L3 | ETWDDSLKGPV | 69 |
| CDR-H1 | SYAMS | 70 |
| CDR-H2 | TISGSGDSTYYADSVKG | 71 |
| CDR-H3 | EWELGDAFDI | 72 |
| light chain variable region | QSVLTQPPSTSGTPGQTFSIFCSGSSSNIGTNTVNWYQQL PGTAPKLLIYSNDQRPSGVPDRFSGSKSGTSASLAISGLQS EDEADYYCETWDDSLKGPVFGGGTKVTVLGQPAAA | 243 |
| light chain variable region coding gene | CAGTCTGTGCTGACTCAGCCACCCTCAACGTCTGGGAC CCCCGGGCAGACGTTCTCCATTTTTTGTTCTGGAAGCAG TTCGAACATCGGAACTAATACTGTTAATTGGTACCAGCAG CTCCCAGGAACGGCCCCCAAACTCCTCATCTATAGTAATG ATCAGCGGCCCTCAGGGGTCCCTGACCGATTCTCTGGC TCCAAGTCTGGCACCTCAGCCTCCCTGGCCATCAGTGG GCTCCAGTCTGAGGATGAGGCTGATTATTACTGTGAAACA TGGGATGACAGCCTGAAAGGCCCGGTGTTCGGCGGGG GGACCAAGGTCACCGTCCTAGGTCAGCCCGCGGCCGCA | 283 |
| heavy chain variable region | EVQLVESGGGLVQPGGSLKLSCAASGFTFSSYAMSWVRR APGKGLEWVSTISGSGDSTYYADSVKGRFTISRDNSKNTLY LQMNNLRAEDTAVYYCAREWELGDAFDIWGRGTLVTVSS | 244 |

(continued)

| Antibody clone F11 | | |
|---|---|---|
| | Amino acid sequence (N→C) / Nucleic acid sequence (5'→3') | SEQ ID NO |
| heavy chain variable region coding gene | GAGGTGCAGCTGGTGGAGTCTGGGGGAGGCTTGGTCC AGCCTGGGGGGTCCCTGAAACTCTCCTGTGCAGCGTCT GGATTCACCTTTAGCAGCTATGCCATGAGCTGGGTCCGC CGGGCTCCAGGGAAGGGGCTGGAGTGGGTCTCAACTAT TAGTGGTAGTGGTGATAGCACATACTACGCAGACTCCGT GAAGGGCCGGTTCACCATCTCCAGAGACAATTCCAAGAA CACGCTGTATCTGCAAATGAACAACCTGAGAGCCGAGGA CACGGCCGTATATTACTGTGCGAGAGAATGGGAACTAGG CGATGCTTTTGATATCTGGGGCCGTGGCACCCTGGTCAC CGTCTCGAGT | 284 |
| light chain (Lambd a) | QSVLTQPPSTSGTPGQTFSIFCSGSSSNIGTNTVNWYQQL PGTAPKLLIYSNDQRPSGVPDRFSGSKSGTSASLAISGLQS EDEADYYCETWDDSLKGPVFGGGTKVTVLGQPAAAPSVTL FPPSSEELQANKATLVCLISDFYPGAVTVAWKADSSPVKAG VETTTPSKQSNNKYAASSYLSLTPEQWKSHRSYSCQVTHE GSTVEKTVAPTECS | 323 |
| heavy chain | EVQLVESGGGLVQPGGSLKLSCAASGFTFSSYAMSWVRR APGKGLEWVSTISGSGDSTYYADSVKGRFTISRDNSKNTLY LQMNNLRAEDTAVYYCAREWELGDAFDIWGRGTLVTVSSA STKGPSVFPLAPCSRSTSESTAALGCLVKDYFPEPVTVSW NSGALTSGVHTFPAVLQSSGLYSLSSVVTVPSSSLGTKTYT CNVDHKPSNTKVDKRVESKYGPPCPPCPAPEFLGGPSVFL FPPKPKDTLMISRTPEVTCVVVDVSQEDPEVQFNWYVDGV EVHNAKTKPREEQFNSTYRVVSVLTVLHQDWLNGKEYKCK VSNKGLPSSIEKTISKAKGQPREPQVYTLPPSQEEMTKNQV SLTCLVKGFYPSDIAVEWESNGQPENNYKTTPPVLDSDGS FFLYSRLTVDKSRWQEGNVFSCSVMHEALHNHYTQKSLSL SLGK | 324 |

[Table 16]

| Antibody clone D3 | | |
|---|---|---|
| | Amino acid sequence (N→C) / Nucleic acid sequence (5'→3') | SEQ ID NO |
| CDR-L 1 | RASQSISSYLN | 73 |
| CDR-L2 | AASSLQS | 74 |
| CDR-L3 | QQSYSTRWT | 75 |
| CDR-H1 | SYAMS | 76 |
| CDR-H2 | AISGSGGSTYYADSVKG | 77 |

EP 4 071 172 A1

(continued)

| Antibody clone D3 | | |
|---|---|---|
| | Amino acid sequence (N→C) / Nucleic acid sequence (5'→3') | SEQ ID NO |
| CDR-H3 | DRGSYGYYYGMDV | 78 |
| light chain variable region | DIQMTQSPSSLSASVGDRVTITCRASQSISSYLNWYQQKPGKAPKLLIYAASSLQSGVPSRFSGSGSGTDFTLTISSLQPEDFATYYCQQSYSTRWTFGQGTKVEIKRTAAA | 245 |
| light chain variable region coding gene | GACATCCAGATGACCCAGTCTCCATCCTCCCTGTCTGCATCTGTAGGAGACAGAGTCACCATCACTTGCCGGGCAAGTCAGAGCATTAGCAGCTATTTAAATTGGTATCAGCAGAAACCAGGGAAAGCCCCTAAGCTCCTGATCTATGCTGCATCCAGTTTGCAAAGTGGGGTCCCATCAAGGTTCAGTGGCAGTGGATCTGGGACAGATTTCACTCTCACCATCAGCAGTCTGCAACCTGAAGATTTTGCAACTTACTACTGTCAACAGAGTTACAGTACCCGGTGGACGTTCGGCCAAGGGACCAAGGTGGAAATCAAACGTACCGCGGCCGCA | 285 |
| heavy chain variable region | EVQLLESGGGVVQPGRSLRLSCAASGSTFSSYAMSWVRQAPGKGLEWVSAISGSGGSTYYADSVKGRFTISRDNSKNTLYLQMNSLRAEDTAVYYCAKDRGSYGYYYGMDVWGQGTMVTVSS | 246 |
| heavy chain variable region coding gene | GAGGTGCAGCTGTTGGAGTCTGGGGGAGGCGTGGTCCAGCCTGGGAGGTCCCTGAGACTCTCCTGTGCAGCCTCTGGATCCACCTTTAGCAGCTATGCCATGAGCTGGGTCCGCCAGGCTCCAGGGAAGGGGCTGGAGTGGGTCTCAGCTATTAGTGGTAGTGGTGGTAGCACTACTACGCAGACTCCGTGAAGGGCCGGTTCACCATCTCCAGAGACAATTCCAAGAACACGCTGTATCTGCAAATGAACAGCCTGAGAGCCGAGGACACGGCCGTATATTACTGTGCGAAAGACAGAGGCAGCTATGGTTACTACTACGGTATGGACGTCTGGGGCCAAGGGACAATGGTCACCGTCTCGAGT | 286 |
| light chain (Kappa) | DIQMTQSPSSLSASVGDRVTITCRASQSISSYLNWYQQKPGKAPKLLIYAASSLQSGVPSRFSGSGSGTDFTLTISSLQPEDFATYYCQQSYSTRWTFGQGTKVEIKRTAAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS | 325 |

41

(continued)

| Antibody clone D3 | | |
|---|---|---|
| | Amino acid sequence (N→C) / Nucleic acid sequence (5'→3') | SEQ ID NO |
| heavy chain | EVQLLESGGGVVQPGRSLRLSCAASGSTFSSYAMSWVRQ APGKGLEWVSAISGSGGSTYYADSVKGRFTISRDNSKNTLY LQMNSLRAEDTAVYYCAKDRGSYGYYYGMDVWGQGTMV TVSSASTKGPSVFPLAPCSRSTSESTAALGCLVKDYFPEPV TVSWNSGALTSGVHTFPAVLQSSGLYSLSSVVTVPSSSLG TKTYTCNVDHKPSNTKVDKRVESKYGPPCPPCPAPEFLGG PSVFLFPPKPKDTLMISRTPEVTCVVVDVSQEDPEVQFNW YVDGVEVHNAKTKPREEQFNSTYRVVSVLTVLHQDWLNG KEYKCKVSNKGLPSSIEKTISKAKGQPREPQVYTLPPSQEE MTKNQVSLTCLVKGFYPSDIAVEWESNGQPENNYKTTPPV LDSDGSFFLYSRLTVDKSRWQEGNVFSCSVMHEALHNHY TQKSLSLSLGK | 326 |

[Table 17]

| Antibody clone B12 | | |
|---|---|---|
| | Amino acid sequence (N→C) / Nucleic acid sequence (5'→3') | SEQ ID NO |
| CDR-L 1 | RASQSISSYLN | 79 |
| CDR-L2 | AASSLQS | 80 |
| CDR-L3 | QQSYSTLRT | 81 |
| CDR-H1 | GYYMH | 82 |
| CDR-H2 | WINPNSGGTNYAQKFQG | 83 |
| CDR-H3 | AGASIVGATALDY | 84 |
| light chain variable region | DIQMTQSPSSLSASVGDRVTITCRASQSISSYLNWYQQKP GKAPKLLIYAASSLQSGVPSRFSGSGSGTDFTLTISSLQPED FATYYCQQSYSTLRTFGQGTKVEIKRTAAA | 247 |
| light chain variable region coding gene | GACATCCAGATGACCCAGTCTCCATCCTCCCTGTCTGCA TCTGTAGGAGACAGAGTCACCATCACTTGCCGGGCAAGT CAGAGCATTAGCAGCTATTTAAATTGGTATCAGCAGAAAC CAGGGAAAGCCCCTAAGCTCCTGATCTATGCTGCATCCA GTTTGCAAAGTGGGGTCCCATCAAGGTTCAGTGGCAGT GGATCTGGGACAGATTTCACTCTCACCATCAGCAGTCTG CAACCTGAAGATTTTGCAACTTACTACTGTCAACAGAGTT ACAGTACCCTCCGGACGTTCGGCCAAGGGACCAAGGTG GAGATCAAACGTACCGCGGCCGCA | 287 |

(continued)

| Antibody clone B12 | | |
|---|---|---|
| | Amino acid sequence (N→C) / Nucleic acid sequence (5'→3') | SEQ ID NO |
| heavy chain variable region | QVQLVQSGAEVKKPGASVKVSCKASGYTFTGYYMHWVRQ APGQGLEWMGWINPNSGGTNYAQKFQGRVTITADESTSTA YMELSSLRSEDTAVYYCTRAGASIVGATALDYWGQGTLVTV SS | 248 |
| heavy chain variable region coding gene | CAGGTCCAGCTGGTACAGTCTGGGGGCTGAGGTGAAGAA GCCTGGGGGCCTCAGTGAAGGTCTCCTGCAAGGCTTCTG GATACACCTTCACCGGCTACTATATGCACTGGGTGCGAC AGGCCCCTGGACAAGGGCTTGAGTGGATGGGATGGATC AACCCTAACAGTGGTGGCACAAACTACGCACAGAAGTTC CAGGGCAGAGTCACGATTACCGCGGACGAATCCACGAG CACAGCCTACATGGAGCTGAGCAGCCTGAGATCTGAGG ACACGGCCGTGTATTACTGTACGAGAGCCGGTGCTTCTA TAGTGGGAGCTACCGCGCTTGACTACTGGGGCCAGGGA ACCCTGGTCACCGTCTCGAGT | 288 |
| light chain (Kappa) | DIQMTQSPSSLSASVGDRVTITCRASQSISSYLNWYQQKP GKAPKLLIYAASSLQSGVPSRFSGSGSGTDFTLTISSLQPED FATYYCQQSYSTLRTFGQGTKVEIKRTAAAPSVFIFPPSDEQ LKSGTASVVCLLNNFYPREAKVQWKVDNALQSGNSQESVT EQDSKDSTYSLSSTLTLSKADYEKHKLYACEVTHQGLSSPV TKSFNRGEC | 327 |
| heavy chain | QVQLVQSGAEVKKPGASVKVSCKASGYTFTGYYMHWVRQ APGQGLEWMGWINPNSGGTNYAQKFQGRVTITADESTSTA YMELSSLRSEDTAVYYCTRAGASIVGATALDYWGQGTLVTV SSASTKGPSVFPLAPCSRSTSESTAALGCLVKDYFPEPVTV SWNSGALTSGVHTFPAVLQSSGLYSLSSVVTVPSSSLGTK TYTCNVDHKPSNTKVDKRVESKYGPPCPPCPAPEFLGGPS VFLFPPKPKDTLMISRTPEVTCVVVDVSQEDPEVQFNWYV DGVEVHNAKTKPREEQFNSTYRVVSVLTVLHQDWLNGKE YKCKVSNKGLPSSIEKTISKAKGQPREPQVYTLPPSQEEMT KNQVSLTCLVKGFYPSDIAVEWESNGQPENNYKTTPPVLD SDGSFFLYSRLTVDKSRWQEGNVFSCSVMHEALHNHYTQ KSLSLSLGK | 328 |

[Table 18]

| Antibody clone E4 | | |
|---|---|---|
| | Amino acid sequence (N→C) / Nucleic acid sequence (5'→3') | SEQ ID NO |
| CDR-L 1 | TRSSGSIASNYVQ | 85 |
| CDR-L2 | EDNQRPS | 86 |

(continued)

| Antibody clone E4 | | |
|---|---|---|
| | Amino acid sequence (N→C) / Nucleic acid sequence (5'→3') | SEQ ID NO |
| CDR-L3 | QSYDTGNRNYV | 87 |
| CDR-H1 | SYTIS | 88 |
| CDR-H2 | RIIPILGIANYAQKFQG | 89 |
| CDR-H3 | GPSLNYAGYFDN | 90 |
| light chain variable region | NFMLTQPHSVSESPGKTVTISCTRSSGSIASNYVQWYQQR PGSSPTTVIYEDNQRPSGVPDRFSGSIDSSSNSASLTISGL KTEDEADYYCQSYDTGNRNYVFGTGTQLTVLGQPAAA | 249 |
| light chain variable region coding gene | AATTTTATGCTGACTCAGCCCCACTCTGTGTCGGAGTCT CCGGGAAAGACGGTAACCATCTCCTGCACCCGCAGCAG TGGCAGCATTGCCAGCAACTATGTGCAGTGGTACCAGC AGCGCCCGGGCAGTTCCCCCACCACTGTGATCTATGAG GATAACCAAAGACCCTCTGGGGTCCCTGATCGGTTCTCT GGCTCCATCGACAGCTCCTCCAACTCTGCCTCCCTCACC ATCTCTGGACTGAAGACTGAGGACGAGGCTGACTACTAC TGTCAGTCTTATGATACCGGCAATCGGAATTATGTCTTC GGAACTGGGACCCAGCTCACCGTCCTAGGTCAGCCCGC GGCCGCA | 289 |
| heavy chain variable region | QVQLVQSGAEVKKPGSSVKVSCKASGGTFSSYTISWVRQA PGQGLEWMGRIIPILGIANYAQKFQGRVTMTRDMSTDTAY MELSSLTYDDTAVYFCVRGPSLNYAGYFDNWGQGTLVTVS S | 250 |
| heavy chain variable region coding gene | CAGGTGCAGCTGGTGCAATCTGGGGCTGAGGTGAAGAA GCCTGGGTCCTCGGTGAAGGTCTCCTGCAAGGCTTCTG GAGGCACCTTCAGCAGCTATACTATCAGCTGGGTGCGAC AGGCCCCTGGACAAGGGCTTGAGTGGATGGGAAGGATC ATCCCTATCCTTGGTATAGCAAACTACGCACAGAAGTTCC AGGGCAGAGTCACCATGACCAGGGACATGTCCACAGAC ACAGCCTACATGGAGTTGAGCAGCCTGACATATGATGAC ACGGCCGTATATTTTGTGTGAGAGGCCCTAGTCTTAATT ATGCCGGCTATTTTGACAACTGGGGCCAGGGCACCCTG GTCACCGTCTCGAGT | 290 |
| light chain (Lambd a) | NFMLTQPHSVSESPGKTVTISCTRSSGSIASNYVQWYQQR PGSSPTTVIYEDNQRPSGVPDRFSGSIDSSSNSASLTISGL KTEDEADYYCQSYDTGNRNYVFGTGTQLTVLGQPAAAPSV TLFPPSSEEIQANKATLVCLISDFYPGAVTVAWKADSSPVK AGVETTTPSKQSNNKYAASSYLSLTPEQWKSHRSYSCQVT HEGSTVEKTVAPTECS | 329 |

(continued)

| Antibody clone E4 | | |
|---|---|---|
| | Amino acid sequence (N→C) / Nucleic acid sequence (5'→3') | SEQ ID NO |
| heavy chain | QVQLVQSGAEVKKPGSSVKVSCKASGGTFSSYTISWVRQA PGQGLEWMGRIIPILGIANYAQKFQGRVTMTRDMSTDTAY MELSSLTYDDTAVYFCVRGPSLNYAGYFDNWGQGTLVTVS SASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKS LSLSLGK | 330 |

[Table 19]

| Antibody clone E12 | | |
|---|---|---|
| | Amino acid sequence (N→C) / Nucleic acid sequence (5'→3') | SEQ ID NO |
| CDR-L1 | QGDSLRSYYAS | 91 |
| CDR-L2 | GKEKRPS | 92 |
| CDR-L3 | NSRGSTTDYMV | 93 |
| CDR-H1 | SYAMH | 94 |
| CDR-H2 | VISYDGSNKYYADSVKG | 95 |
| CDR-H3 | ERGSGMDV | 96 |
| light chain variable region | SYELTQDPAVSVALGQTVRITCQGDSLRSYYASWYQQKSG QAPVLVIYGKEKRPSGIPDRFSGSSSGNTASLTITGARAEDE ADYYCNSRGSTTDYMVFGGGTQLTVLGQPAAA | 251 |
| light chain variable region coding gene | TCCTATGAGCTGACTCAGGACCCTGCTGTGTCTGTGGCC TTGGGACAGACAGTCAGGATCACATGCCAAGGAGACAG CCTCAGAAGCTATTATGCAAGCTGGTACCAGCAGAAGTC AGGACAGGCCCCTGTACTTGTCATCTATGGTAAAGAAAA GCGCCCCTCAGGGATCCCAGACCGATTCTCTGGCTCCA GCTCAGGAAACACAGCTTCCTTGACCATCACTGGGGCTC GGGCGGAAGATGAGGCTGACTATTACTGTAACTCCCGGG GCAGCACTACTGACTATATGGTGTTCGGCGGGGGGACCC AGCTCACCGTCCTAGGTCAGCCCGCGGCCGCA | 291 |
| heavy chain variable region | QVQLVESGGGLVQPGGSLRLSCAASGFTFSSYAMHWVRQ APGKGLEWVAVISYDGSNKYYADSVKGRFTISRDNSKNTLY LQMNSLRAEDTAVYYCARERGSGMDVWGQGTLVTVSS | 252 |

(continued)

| Antibody clone E12 | | |
|---|---|---|
| | Amino acid sequence (N→C) / Nucleic acid sequence (5'→3') | SEQ ID NO |
| heavy chain variable region coding gene | CAGGTGCAGCTGGTGGAGTCCGGGGGAGGCTTAGTTCA GCCTGGGGGGTCCCTGAGACTCTCCTGTGCAGCCTCTG GATTCACCTTCAGTAGCTATGCTATGCACTGGGTCCGCCA GGCTCCAGGCAAGGGGCTGGAGTGGGTGGCAGTTATAT CATATGATGGAAGCAATAAATACTACGCAGACTCCGTGAA GGGCCGATTCACCATCTCCAGAGACAATTCCAAGAACAC GCTGTATCTGCAAATGAACAGCCTGAGAGCTGAGGACAC GGCTGTGTATTACTGTGCGAGAGAACGGGGAAGTGGTAT GGACGTCTGGGGCCAAGGAACCCTGGTCACCGTCTCGA GT | 292 |
| light chain (Lambd a) | SYELTQDPAVSVALGQTVRITCQGDSLRSYYASWYQQKSG QAPVLVIYGKEKRPSGIPDRFSGSSSGNTASLTITGARAEDE ADYYCNSRGSTTDYMVFGGGTQLTVLGQPAAAPSVTLFPP SSEELQANKATLVCLISDFYPGAVTVAWKADSSPVKAGVET TTPSKQSNNKYAASSYLSLTPEQWKSHRSYSCQVTHEGST VEKTVAPTECS | 331 |
| heavy chain | QVQLVESGGGLVQPGGSLRLSCAASGFTFSSYAMHWVRQ APGKGLEWVAVISYDGSNKYYADSVKGRFTISRDNSKNTLY LQMNSLRAEDTAVYYCARERGSGMDVWGQGTLVTVSSAS TKGPSVFPLAPCSRSTSESTAALGCLVKDYFPEPVTVSWN SGALTSGVHTFPAVLQSSGLYSLSSVVTVPSSSLGTKTYTC NVDHKPSNTKVDKRVESKYGPPCPPCPAPEFLGGPSVFLF PPKPKDTLMISRTPEVTCVVVDVSQEDPEVQFNWYVDGVE VHNAKTKPREEQFNSTYRVVSVLTVLHQDWLNGKEYKCKV SNKGLPSSIEKTISKAKGQPREPQVYTLPPSQEEMTKNQVS LTCLVKGFYPSDIAVEWESNGQPENNYKTTPPVLDSDGSF FLYSRLTVDKSRWQEGNVFSCSVMHEALHNHYTQKSLSLS LGK | 332 |

[Table 20]

| Antibody clone D1 | | |
|---|---|---|
| | Amino acid sequence (N→C) / Nucleic acid sequence (5'→3') | SEQ ID NO |
| CDR-L 1 | KASQDIDDDMN | 97 |
| CDR-L2 | EASTLVP | 98 |
| CDR-L3 | LQHDKFPYT | 99 |
| CDR-H1 | SYGIS | 100 |
| CDR-H2 | WINPNSGGTNYAQKFQG | 101 |

(continued)

| Antibody clone D1 | | |
|---|---|---|
| | Amino acid sequence (N→C) / Nucleic acid sequence (5'→3') | SEQ ID NO |
| CDR-H3 | RGVDEGDY | 102 |
| light chain variable region | ETTLTQSPAFMSATPGDKVNISCKASQDIDDDMNWYQQKP GEAAISIIQEASTLVPGIPPRFSGSGYGTDFTLTINNIESEDAA YYFCLQHDKFPYTFGQGTKLEIKRTAAA | 253 |
| light chain variable region coding gene | GAAACGACACTCACGCAGTCTCCAGCATTCATGTCAGCG ACTCCAGGAGACAAAGTCAACATCTCCTGCAAAGCCAGC CAAGACATTGATGATGATATGAACTGGTACCAACAGAAA CCAGGAGAAGCTGCTATTTCCATTATTCAAGAAGCTAGT ACTCTCGTTCCTGGAATCCCACCTCGATTCAGTGGCAGC GGGTATGGAACAGATTTTACCCTCACAATTAATAACATAG AATCTGAGGATGCTGCATATTACTTCTGTCTACAACATGA TAAGTTCCCGTACACTTTTGGCCAGGGGACCAAGCTGGA GATCAAACGTACCGCGGCCGCA | 293 |
| heavy chain variable region | EVQLVQSGAEVKKPGASVKVSCKASGYTFTSYGISWVRQA PGQGLEWMGWINPNSGGTNYAQKFQGRVTMTRDTSISTA YMELSRLRSDDTAVYYCASRGVDEGDYWGQGTMVTVSS | 254 |
| heavy chain variable region coding gene | GAAGTGCAGCTGGTGCAGTCTGGAGCTGAGGTGAAGAA GCCTGGGGCCTCAGTGAAGGTCTCCTGCAAGGCTTCTG GTTACACCTTTACCAGCTATGGTATCAGCTGGGTGCGAC AGGCCCCTGGACAAGGGCTTGAGTGGATGGGATGGATC AACCCTAACAGTGGTGGCACAAACTATGCACAGAAGTTT CAGGGCAGGGTCACCATGACCAGGGACACGTCCATCAG CACAGCCTACATGGAGCTGAGCAGGCTGAGATCTGACG ACACGGCCGTGTATTACTGTGCGAGTCGGGGGGGTTGAT GAGGGGGACTACTGGGGCCAAGGGACAATGGTCACCGT CTCGAGT | 294 |
| light chain (Kappa) | ETTLTQSPAFMSATPGDKVNISCKASQDIDDDMNWYQQKP GEAAISIIQEASTLVPGIPPRFSGSGYGTDFTLTINNIESEDAA YYFCLQHDKFPYTFGQGTKLEIKRTAAAPSVFIFPPSDEQLK SGTASVVCLLNNFYPREAKVQWKVDNALQSGNSQESVTE QDSKDSTYSLSSTLTLSKADYEKHKLYACEVTHQGLSSPVT | 333 |
| | KSFNRGEC | |

(continued)

| Antibody clone D1 | | |
|---|---|---|
| | Amino acid sequence (N→C) / Nucleic acid sequence (5'→3') | SEQ ID NO |
| heavy chain | EVQLVQSGAEVKKPGASVKVSCKASGYTFTSYGISWVRQA PGQGLEWMGWINPNSGGTNYAQKFQGRVTMTRDTSISTA YMELSRLRSDDTAVYYCASRGVDEGDYWGQGTMVTVSSA STKGPSVFPLAPCSRSTSESTAALGCLVKDYFPEPVTVSW NSGALTSGVHTFPAVLQSSGLYSLSSVVTVPSSSLGTKTYT CNVDHKPSNTKVDKRVESKYGPPCPPCPAPEFLGGPSVFL FPPKPKDTLMISRTPEVTCVVVDVSQEDPEVQFNWYVDGV EVHNAKTKPREEQFNSTYRVVSVLTVLHQDWLNGKEYKCK VSNKGLPSSIEKTISKAKGQPREPQVYTLPPSQEEMTKNQV SLTCLVKGFYPSDIAVEWESNGQPENNYKTTPPVLDSDGS FFLYSRLTVDKSRWQEGNVFSCSVMHEALHNHYTQKSLSL SLGK | 334 |

[Table 21]

| Antibody clone E6 | | |
|---|---|---|
| | Amino acid sequence (N→C) / Nucleic acid sequence (5'→3') | SEQ ID NO |
| CDR-L 1 | TGSSGNIASNYVQ | 103 |
| CDR-L2 | RDDQRPS | 104 |
| CDR-L3 | QSYDSSSWV | 105 |
| CDR-H1 | TYDIT | 106 |
| CDR-H2 | WMNPNSGNSRSAQKFQG | 107 |
| CDR-H3 | GDYSGWLTATALDY | 108 |
| light chain variable region | NFMLTQPHSVSESPGKTVTLSCTGSSGNIASNYVQWYQHR PGSAPTTVIYRDDQRPSGVPDRFSGSIDSSSNSASLTISGL RPEDEADYYCQSYDSSSWVFGGGTKLTVLGQPAAA | 255 |
| light chain variable region coding gene | AATTTTATGCTGACTCAGCCCCACTCTGTGTCGGAGTCT CCGGGGAAGACGGTTACCCTCTCCTGCACCGGCAGCAG CGGCAACATTGCCAGTAACTATGTGCAGTGGTACCAGCA CCGCCCGGGCAGTGCCCCCACCACTGTGATCTACCGGG ATGACCAAAGACCCTCTGGAGTCCCTGATCGCTTCTCTG GCTCCATCGACAGTTCATCCAACTCTGCCTCCCTCACGA TCTCTGGACTGAGGCCTGAGGACGAGGCTGACTATTACT GTCAGTCTTATGATAGCAGCTCTTGGGTGTTCGGCGGAG GGACCAAGCTGACCGTCCTAGGTCAGCCCGCGGCCGCA | 295 |

(continued)

| Antibody clone E6 | | |
|---|---|---|
| | Amino acid sequence (N→C) / Nucleic acid sequence (5'→3') | SEQ ID NO |
| heavy chain variable region | QVQLVQSGAEVKKPGASVKVSCKASGYTFTTYDITWVRQA PGQGLEWMGWMNPNSGNSRSAQKFQGRVSMTSDSSIST AYMELSSLRSEDTAVYYCATGDYSGVVLTATALDYWGQGT LVTVSS | 256 |
| heavy chain variable region<br><br>coding gene | CAGGTCCAGCTTGTGCAGTCTGGAGCAGAGGTGAAGAA GCCTGGGGCCTCAGTGAAGGTCTCCTGCAAGGCTTCTG GATACACCTTCACCACTTATGATATCACCTGGGTGCGAC AGGCCCCTGGACAAGGCCTTGAGTGGATGGGATGGATG AACCCGAACAGTGGTAACTCACGCTCTGCACAGAAGTTC CAGGGCAGAGTCAGCATGACCAGTGACTCCTCCATAAG CACAGCCTACATGGAGCTGAGCAGCCTGAGATCTGAGG ACACGGCCGTGTATTACTGTGCAACAGGAGACTACTCG GGTGTGGTACTAACTGCAACAGCACTTGACTACTGGGG CCAGGGAACCCTGGTCACCGTCTCGAGT | 296 |
| light chain (Lambd a) | NFMLTQPHSVSESPGKTVTLSCTGSSGNIASNYVQWYQHR PGSAPTTVIYRDDQRPSGVPDRFSGSIDSSSNSASLTISGL RPEDEADYYCQSYDSSSWVFGGGTKLTVLGQPAAAPSVTL FPPSSEELQANKATLVCLISDFYPGAVTVAWKADSSPVKAG VETTTPSKQSNNKYAASSYLSLTPEQWKSHRSYSCQVTHE GSTVEKTVAPTECS | 335 |
| heavy chain | QVQLVQSGAEVKKPGASVKVSCKASGYTFTTYDITWVRQA PGQGLEWMGWMNPNSGNSRSAQKFQGRVSMTSDSSIST AYMELSSLRSEDTAVYYCATGDYSGVVLTATALDYWGQGT LVTVSSASTKGPSVFPLAPCSRSTSESTAALGCLVKDYFPE PVTVSWNSGALTSGVHTFPAVLQSSGLYSLSSVVTVPSSS LGTKTYTCNVDHKPSNTKVDKRVESKYGPPCPPCPAPEFL GGPSVFLFPPKPKDTLMISRTPEVTCVVVDVSQEDPEVQF NWYVDGVEVHNAKTKPREEQFNSTYRVVSVLTVLHQDWL NGKEYKCKVSNKGLPSSIEKTISKAKGQPREPQVYTLPPSQ EEMTKNQVSLTCLVKGFYPSDIAVEWESNGQPENNYKTTP PVLDSDGSFFLYSRLTVDKSRWQEGNVFSCSVMHEALHN HYTQKSLSLSLGK | 336 |

[Table 22]

| Antibody clone E9 | | |
|---|---|---|
| | Amino acid sequence (N→C) / Nucleic acid sequence (5'→3') | SEQ ID NO |
| CDR-L 1 | SGSSSNIGNNYVY | 109 |
| CDR-L2 | RNNQRPS | 110 |

(continued)

| Antibody clone E9 | | |
|---|---|---|
| | Amino acid sequence (N→C) / Nucleic acid sequence (5'→3') | SEQ ID NO |
| CDR-L3 | AAWDDSLSGWV | 111 |
| CDR-H1 | SYGMH | 112 |
| CDR-H2 | NIKQDGSEKYYVDSVKG | 113 |
| CDR-H3 | EDRIAAAGMRELDY | 114 |
| light chain variable region | QSELTQLPSASETPGQRVTISCSGSSSNIGNNYVYWYQQL PGTAPKLLIYRNNQRPSGVPDRFSGSKSGTSASLAISGLRS EDEADYYCAAWDDSLSGWVFGGGTKLTVLGQPAAA | 257 |
| light chain variable region coding gene | CAGTCTGAGCTGACTCAGCTACCCTCAGCGTCTGAGACC CCCGGGCAGAGGGTCACCATCTCTTGTTCTGGAAGCAG CTCCAACATCGGAAATAATTATGTATACTGGTACCAGCAAC TCCCCGGAACGGCCCCCAAACTCCTCATCTATAGGAATAA TCAGCGGCCCTCAGGGGTCCCTGACCGATTCTCTGGCT CCAAGTCTGGCACCTCAGCCTCCCTGGCCATCAGTGGG CTCCGGTCCGAGGATGAGGCTGATTATTACTGTGCAGCA TGGGATGACAGCCTGAGTGGTTGGGTGTTCGGCGGAGG GACCAAGCTGACCGTCCTAGGTCAGCCCGCGGCCGCA | 297 |
| heavy chain variable region | QVQLVESGGGLVQPGRSLRLSCAASGFTFSSYGMHWVRQ APGKGLEWVANIKQDGSEKYYVDSVKGRFTISRDNAKNTLY LQMNSLRAEDTAVYYCAREDRIAAAGMRELDYWGQGTLVT VSS | 258 |
| heavy chain variable region coding gene | CAGGTGCAGCTGGTGGAGTCTGGGGGAGGCTTGGTACA GCCTGGGAGGTCCCTGAGACTCTCCTGTGCAGCGTCTG GATTCACCTTCAGTAGCTATGGCATGCACTGGGTCCGCC AGGCTCCAGGGAAGGGGCTGGAGTGGGTGGCCAACATA AAGCAAGATGGAAGTGAGAAATACTATGTGGACTCTGTG AAGGGCCGATTCACCATCTCCAGAGACAACGCCAAGAAC ACGCTGTATCTCCAAATGAACAGCCTGAGAGCTGAGGAC ACGGCTGTGTATTACTGTGCGAGAGAGGACCGTATAGCA GCAGCTGGGATGCGGGAGTTGGACTACTGGGGCCAGG GCACCCTGGTCACCGTCTCGAGT | 298 |
| light chain (Lambd a) | QSELTQLPSASETPGQRVTISCSGSSSNIGNNYVYWYQQL PGTAPKLLIYRNNQRPSGVPDRFSGSKSGTSASLAISGLRS EDEADYYCAAWDDSLSGWVFGGGTKLTVLGQPAAAPSVT LFPPSSEELQANKATLVCLISDFYPGAVTVAWKADSSPVKA GVETTTPSKQSNNKYAASSYLSLTPEQWKSHRSYSCQVTH EGSTVEKTVAPTECS | 337 |

(continued)

| Antibody clone E9 | | |
|---|---|---|
| | Amino acid sequence (N→C) / Nucleic acid sequence (5'→3') | SEQ ID NO |
| heavy chain | QVQLVESGGGLVQPGRSLRLSCAASGFTFSSYGMHWVRQ APGKGLEWVANIKQDGSEKYYVDSVKGRFTISRDNAKNTLY LQMNSLRAEDTAVYYCAREDRIAAAGMRELDYWGQGTLVT VSSASTKGPSVFPLAPCSRSTSESTAALGCLVKDYFPEPVT VSWNSGALTSGVHTFPAVLQSSGLYSLSSVVTVPSSSLGT KTYTCNVDHKPSNTKVDKRVESKYGPPCPPCPAPEFLGGP SVFLFPPKPKDTLMISRTPEVTCVVVDVSQEDPEVQFNWY VDGVEVHNAKTKPREEQFNSTYRVVSVLTVLHQDWLNGK EYKCKVSNKGLPSSIEKTISKAKGQPREPQVYTLPPSQEEM TKNQVSLTCLVKGFYPSDIAVEWESNGQPENNYKTTPPVL DSDGSFFLYSRLTVDKSRWQEGNVFSCSVMHEALHNHYT QKSLSLSLGK | 338 |

[Table 23]

| Antibody clone A11 | | |
|---|---|---|
| | Amino acid sequence (N→C) / Nucleic acid sequence (5'→3') | SEQ ID NO |
| CDR-L 1 | RSSQSLLHSNGYNYLD | 115 |
| CDR-L2 | LGSNRAS | 116 |
| CDR-L3 | MQGTHWPPYT | 117 |
| CDR-H1 | SYAMT | 118 |
| CDR-H2 | GISSDGTTTTYADSVRG | 119 |
| CDR-H3 | DQLLGWDALNV | 120 |
| light chain variable region | DIVMTQSPLSLPVTPGEPASISCRSSQSLLHSNGYNYLDWY LQKPGQSPQLLIYLGSNRASGVPDRFSGSGSGTDFTLKISR VEAEDVGVYYCMQGTHWPPYTFGQGTKVEIKRTAAA | 259 |
| light chain variable region coding gene | GATATTGTGATGACCCAGTCTCCACTCTCCCTGCCCGTC ACCCCTGGAGAGCCGGCCTCCATCTCCTGCAGGTCTAG TCAGAGCCTCCTGCATAGTAATGGATACAACTATTTGGA TTGGTACCTGCAGAAGCCAGGGCAGTCTCCACAGCTCC TGATCTATTTGGGTTCTAACCGGGCCTCCGGGGTCCCTG ACAGGTTCAGTGGCAGTGGATCAGGCACAGATTTTACAC TGAAAATCAGCAGAGTGGAGGCTGAGGATGTTGGGGTT TATTACTGCATGCAAGGTACACACTGGCCTCCGTACACC TTTGGCCAGGGGACCAAGGTGGAGATCAAACGTACCGC GGCCGCA | 299 |

(continued)

| Antibody clone A11 | | |
|---|---|---|
| | Amino acid sequence (N→C) / Nucleic acid sequence (5'→3') | SEQ ID NO |
| heavy chain variable region | EVQLLESGGGLEQPGGFLRLSCAASGFSFTSYAMTWVRQ APGKGLEWVSGISSDGTTTTYADSVRGRFTISRDNAKNTVY LQMNSLRDEDTAVYYCARDQLLGWDALNVWGQGTMVTVS S | 260 |
| heavy chain variable region coding gene | GAGGTGCAGCTGTTGGAGTCTGGGGGAGGCTTGGAACA GCCTGGGGGGTTCCTGAGACTCTCCTGTGCAGCCTCTG GATTCTCCTTTACCAGCTACGCCATGACCTGGGTCCGCC AGGCTCCAGGGAAGGGGCTGGAGTGGGTCTCAGGTATT AGTAGTGATGGGACCACTACAACCTACGCGGACTCCGT GAGGGGCCGGTTCACCATCTCCAGAGACAACGCCAAGA ACACGGTGTATCTCCAAATGAACAGTCTGAGAGACGAGG ACACGGCTGTGTATTATTGTGCAAGAGATCAATTGTTGG GCTGGGATGCTCTGAATGTCTGGGGCCAAGGGACAATG GTCACCGTCTCGAGT | 300 |
| light chain (Kappa) | DIVMTQSPLSLPVTPGEPASISCRSSQSLLHSNGYNYLDWY LQKPGQSPQLLIYLGSNRASGVPDRFSGSGSGTDFTLKISR VEAEDVGVYYCMQGTHWPPYTFGQGTKVEIKRTAAAPSVF IFPPSDEQLKSGTASVVCLLNNFYPREAKVQWKVDNALQS GNSQESVTEQDSKDSTYSLSSTLTLSKADYEKHKLYACEVT HQGLSSPVTKSFNRGEC | 339 |
| heavy chain | EVQLLESGGGLEQPGGFLRLSCAASGFSFTSYAMTWVRQ APGKGLEWVSGISSDGTTTTYADSVRGRFTISRDNAKNTVY LQMNSLRDEDTAVYYCARDQLLGWDALNVWGQGTMVTVS SASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRVESKYGPPCPPCPAPEFLGGPSV FLFPPKPKDTLMISRTPEVTCVVVDVSQEDPEVQFNWYVD GVEVHNAKTKPREEQFNSTYRVVSVLTVLHQDWLNGKEYK CKVSNKGLPSSIEKTISKAKGQPREPQVYTLPPSQEEMTKN QVSLTCLVKGFYPSDIAVEWESNGQPENNYKTTPPVLDSD GSFFLYSRLTVDKSRWQEGNVFSCSVMHEALHNHYTQKS LSLSLGK | 340 |

**Example 2: Assay of *in vitro* biological activities of the selected antibodies**

**2.1. Natural killer cell (NK cell) surface binding assay**

In order to test whether or not 93 antibodies selected in Example 1.4 bind LILRB1 expressed on surface of immune cells, natural killer cell (NK cell) surface binding assay was performed. A human NK cell, KHYG-1 cell (JCRB) was cultured in RPMI 1640 medium (Gibco) supplemented with 10%(w/v) of FBS (Gibco) and 100 U/mL of interleukin-2 (Novartis). KHYG-1 cells were added to a U-bottom 96-well tissue culture plate (BD Falcon) at the amount of $5 \times 10^4$ cells/well. Each of the selected antibodies was added to the well to the final concentration of $50 \mu g/mL$ per well and incubated at 4°C for 1 hour.

In order to see the level of LILRB1-specific binding of the selected antibodies, a human IgG4 isotype control antibody (Biolegend) was treated in the same manner. After washing with FACS buffer, the cells were treated with an anti-human Fc-biotin antibody (life technologies) and incubated at 4°C for 1 hour. After washing with FACS buffer, the cells were treated with streptavidin PE (BD Pharmigen) and incubated at 4°C for 30 minutes. After washing with FACS buffer, the cells were resuspended and subjected to analysis using iQue screener (Sartorius).

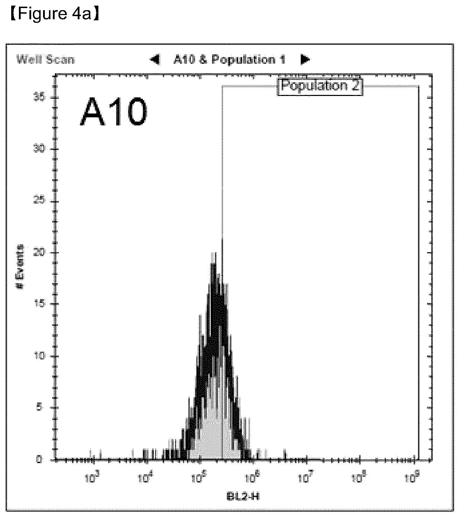

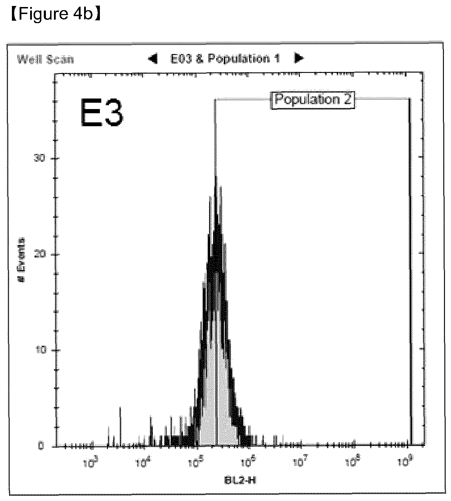

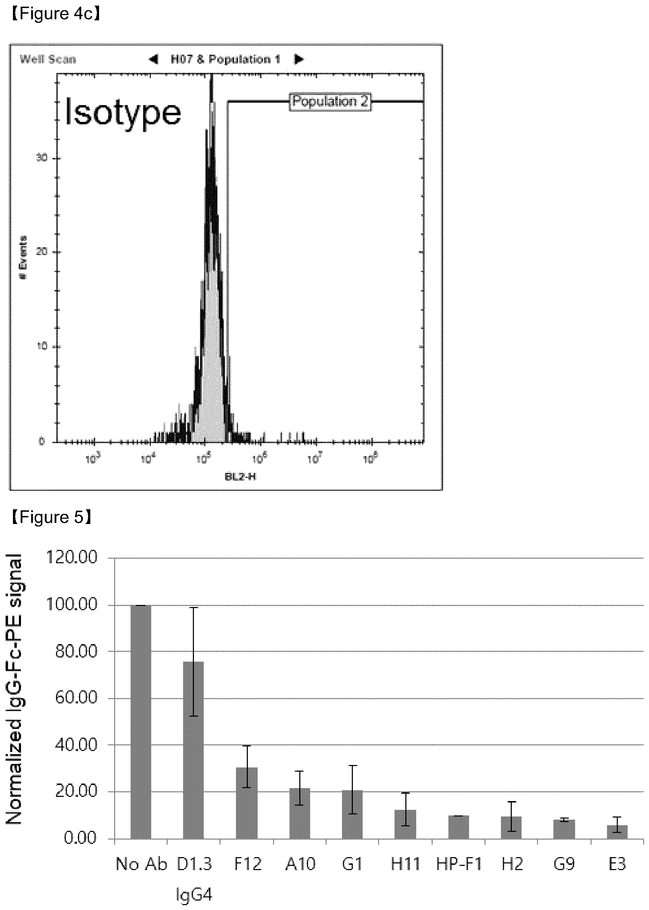

Among the obtained results, the results for antibodies A10, E3, E4, F12, G1, G9, G11, H2 and H11 are representatively compared with that of human IgG4 isotype (control), which are shown in Table 24. The flow cytometry diagrams for A10, E3 and human IgG4 isotype (control) are shown in Figs. 4a (A10), 4b (E3), and 4c (isotype IgG4), respectively:

[Table 24]

|  | Mean Fluorescence Intensity | % of population 2 |
|---|---|---|
| human IgG4 isotype control | 142917.2 | 2.95 |
| A10 | 222660.2 | 28.68 |
| E3 | 268702.2 | 40.22 |
| E4 | 272295.5 | 43.25 |
| F12 | 262012.7 | 38.02 |
| G1 | 321051.7 | 56.23 |
| G9 | 263079.3 | 41.32 |
| G11 | 262771.9 | 40.11 |
| H2 | 238570.9 | 29.00 |
| H11 | 244818.2 | 32.59 |

As shown in Table 24 and Figs. 4a~4c, the tested antibodies show higher level of binding to human NK cells (surface), compared to that of human IgG4 isotype control antibody.

**2.2. Analysis of inhibition of LILRB1 binding to HLA-G by the selected antibodies**

In order to test whether or not the antibodies selected in Example 1.5 exert an inhibitory effect on binding of LILRB1 to its ligand, HLA-G, the degree of blocking by the selected antibodies was analyzed.

For this purpose, JEG-3 cells (ATCC cat# HTB-36), which show high expression level of HLA-G, were used. JEG-3 cells were cultured in MEM medium (Gibco) supplemented with 10%(v/v) of FBS (Gibco) and 1 %(v/v) of pen-strep (Gibco). The JEG-3 cells were added to U-bottom 96-well tissue culture plate (BD Falcon) at the amount of $5\times10^4$ cells/well. The well plate was washed with 1X PBS buffer. Each of the antibodies selected in Example 1.5 (A10, E3, F12, G1, G9, H2 and H11) and LILRB1-Fc (RnD systems) were mixed in FACS buffer (1X PBS + 1% BSA + 1 mM EDTA) to the final concentrations of 10 μg/mL and 5 μg/mL, respectively. The cells were treated with 100 μL of the mixture solution per well and incubated on ice for 2 hours. An anti-LILRB1 antibody (clone HP-F1, Abcam) as a positive control and an anti-lysozyme IgG4 antibody (clone D1.3) as a negative control were treated in the same manner. After washing with FACS buffer twice, the cells were treated with PEanti-huIgG-Fc antibody (Biolegend, 10 μg/mL) and incubated on ice for one hour. After washing with FACS buffer twice, the cells were resuspended in 100 μL of the same buffer and subjected to analysis using iQue screener (Sartorius).

The obtained results are shown in Fig. 5. As shown in Fig. 5, all the tested antibodies A10, E3, F12, G1, G9, H2 and H11 effectively inhibit the binding of LILRB1-Fc to HLA-G-overexpressing cell line.

**2.3. Assay of cancer cell lysis by NK cells**

In order to test whether or not the selected antibodies increase the degree of cancer cell lysis by NK cells, the cell death rate of HLA-G-overexpressing HEK293 cell by NK cell KHYG-1 was analyzed. KHYG-1 cells (JCRB) were added to 96-well tissue culture plate (BD Falcon) at the amount of $2\times10^4$ cells/well ($4\times10^4$ cells/mL, total volume 50 μL). The cells were treated with each antibody (Table 25) to the final concentration of 20 μg/mL per well, and left at 37°C for one hour.

As a negative control, a human IgG4 isotype control antibody (Biolegend) was treated in the same manner.

HLA-G-overexpressing HEK293 cells (which were prepared by transduction of HEK293 cells (American Typo Culture Collection) with lentivirus constructed for expressing HLA-G) were stained with IncuCyte CytoLight Rapid Red Reagent (Sartorius) according to the manufacturer's protocol. After one hour, the HLA-G-overexpressing HEK293 cells were added to the plate at the amount of $1\times10^4$ cells/well ($2\times10^4$ cells/mL, total volume 50 $\mu$L). The plate was placed in IncuCyte S3(Sartorius) equipped in an incubator under the condition of 37°C and 5% $CO_2$, and images thereof were taken for 72 hours. Red area confluence indicating the density of live HLA-G-overexpressing HEK293 cells was measured, and cell viability was calculated. The obtained cell viabilities are shown in Table 25 (wherein the cell viabilities are shown as a relative value to that of control antibody (cell viability of IgG4 isotype-treated well = 1)):

$$\text{Relative cell viability (IgG4 Isotype} =1) = \frac{\text{Normalized red area confluence value of antibody}}{\text{Normalized red area confluence value of IgG4 Isotype}}$$

[Table 25]

| Antibody | Relative cell viability (IgG4 isotype=1) |
|---|---|
| human IgG4 Isotype control | 1.00 |
| A10 | 0.70 |
| B9 | 0.81 |
| D3 | 0.83 |
| E1 | 0.82 |
| E3 | 0.64 |
| F12 | 0.81 |
| G1 | 0.64 |
| G6 | 0.78 |
| G9 | 0.77 |
| G11 | 0.82 |
| H2 | 0.78 |
| H11 | 0.60 |

As shown in Table 25, all the tested antibodies including A10, B9, D3, E1, E3, F12, G1, G6, G9, G11, H2 and H11 increase cell death of HLA-G-overexpressing HEK293 cells by KHYG-1, compared to that of human IgG4 isotype control antibody.

**Example 3: Assay of *in vivo* biological activities of the selected antibodies**

Among the antibodies selected in Example 1.5, two antibodies (E3 and B3) were tested for their *in vivo* anti-cancer efficacies. For this purpose, it was tested whether or not administration of the two antibodies reduces tumor size where the tumor was generated by engrafting human colorectal carcinoma cells (Bioware Brite Cell Line HCT116 Red-Fluc colorectal carcinoma cells (PerkinElmer)) and THP-1 derived macrophages to the mice. As a negative control, human colon cancer xenograft mice prepared as above were treated with a human IgG1 isotype control antibody (BioXcell, Cat. No. BP0297). Hereinafter, the processes are described in detail:

Preparation of THP-1 derived macrophages

The THP-1 derived macrophages used above were prepared by differentiating THP-1 cells (ATCC) with 150 nM phorbol 12-myristate 13-acetate (PMA, Sigma), 20 ng/mL of interferon gamma (Peprotech) and 10 pg/mL of lipopolysaccharide (LPS, Sigma).

Measurement of anti-cancer efficacy in mouse model

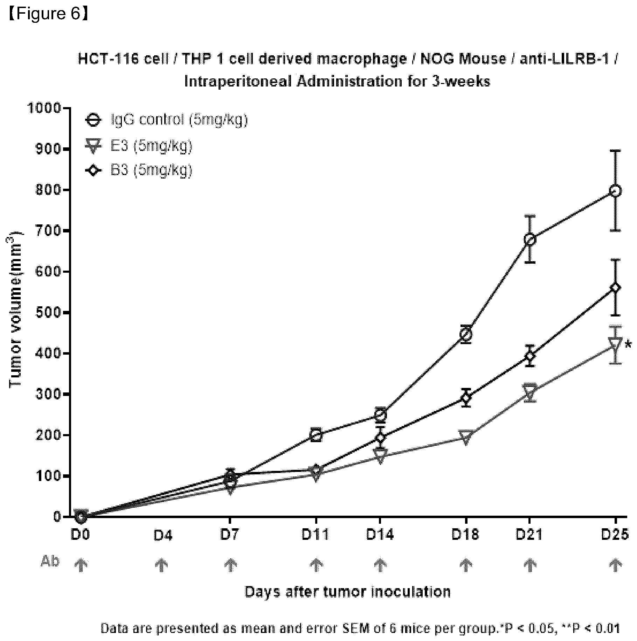

5-week old female CIEA NOG mice [NOG immunodeficient mouse] (Central Institute for Experimental Animals, Japan) were subcutaneously injected with a mixture of $3\times10^6$ cells of HCT116 Red-Fluc colorectal carcinoma cells, $3\times10^6$ cells of THP-1 derived macrophages and each of two test antibodies (E3 or B3 antibody; 20 μg per mouse). From the 4th day after tumor grafting, the antibody was administered to the mouse model at the dosage of 5 mg/kg by intraperitoneal injection twice a week. Then, the size ($mm^3$) of the grafted tumor was measured and shown in Fig. 6. As shown in Fig. 6, all the tested antibodies, particularly antibody E3, exhibit statistically significant effect of inhibiting tumor growth in mouse models grafted with HCT116 colon cancer cells and THP-1 derived macrophages.

**Example 4: Preparation of anti-LILRB1 antibody (E3.1)**

The nucleic acid sequence encoding the full-length heavy chain (SEQ ID NO: 302) of antibody E3, which was confirmed to have particularly significant effect in Example 3, was amplified by PCR. The nucleic acid sequence encoding the region from Ser1 to Leu110 of the light chain variable region (VL) (SEQ ID NO: 221) of antibody E3 was amplified by PCR and ligated to a nucleic acid sequence encoding the lambda constant region (Lambda CL.1, SEQ ID NO: 344) to amplify the nucleic acid sequence encoding lambda light chain by PCR. The amplified sequences were inserted into an expression vector (pTRIOZ-hIgG4, InvivoGen; alternatively, any one of vectors comprising CMV promoter or CMV/CHO beta-actin fusion promoter (KR10-1038126B1) and genes encoding human IgG4 heavy chain constant region and lambda light chain constant region, can be used), wherein the expression vector was designed for encoding a human IgG4 antibody. The DNA sequence of the expression vector was confirmed by sequencing.

An antibody (E3.1) was prepared using the constructed expression vector referring to Example 1.4, and the sequence of the antibody was analyzed referring to Example 1.6 and summarized in Table 26:

[Table 26]

| Antibody clone E3.1 | | |
|---|---|---|
| | amino acid sequence (N→C) / nucleic acid sequence (5'→3') | SEQ ID NO |
| CDR-L 1 | QGDSLRNFYAS | 1 |
| CDR-L2 | GKNNRPS | 2 |
| CDR-L3 | NSRDSSGSHLTGV | 3 |
| CDR-H1 | SYAMS | 4 |
| CDR-H2 | AISGSGGSTYYADSVKG | 5 |
| CDR-H3 | DTYYYGSGRSNAFDI | 6 |
| light chain variable region | SYELTQDPAVSVALGQTVRITCQGDSLRNFYASWYQQKS GQAPVLVMYGKNNRPSGIPDRFSGSTSGNTASLTITGAQ AEDEADYYCNSRDSSGSHLTGVFGGGTKVTVL | 345 |
| light chain variable region coding gene | TCCTATGAGCTGACTCAGGACCCTGCTGTGTCTGTGG CCTTGGGACAGACAGTCAGGATCACATGCCAGGGAGA CAGCCTCAGAAACTTTTATGCAAGCTGGTACCAGCAGA AGTCAGGACAGGCCCCAGTTCTTGTCATGTATGGTAAA AACAACCGGCCCTCAGGGATCCCAGACCGATTCTCTG GCTCCACCTCAGGAAACACAGCTTCCTTGACCATCACT GGGGCTCAGGCGGAAGATGAGGCTGACTATTACTGTA ACTCCCGGGACAGCAGTGGTAGCCATTTGACGGGCGT ATTCGGCGGAGGGACCAAGGTCACCGTCCTA | 346 |

(continued)

| Antibody clone E3.1 | | |
|---|---|---|
| | amino acid sequence (N→C) / nucleic acid sequence (5'→3') | SEQ ID NO |
| heavy chain variable region | QVQLVESGGGLVQPGGSLRLSCAASGFTFSSYAMSWVRQAPGKGLEWVSAISGSGGSTYYADSVKGRFTISRDNSKNTLYLQMISLRAEDTAVYYCARDTYYYGSGRSNAFDIWGQGTLVTVSS | 222 |
| heavy chain variable region coding gene | CAGGTGCAGCTGGTGGAGTCTGGGGGAGGCTTGGTACAGCCTGGGGGGTCCCTGAGACTCTCCTGTGCAGCCTCTGGATTCACCTTTAGCAGCTATGCCATGAGCTGGGTCCGCCAGGCTCCAGGGAAGGGGCTGGAGTGGGTCTCAGCTATTAGTGGTAGTGGTGGTAGCACATACTACGCAGACTCCGTGAAGGGCCGGTTCACCATCTCCAGAGACAATTCCAAGAATACGCTGTATCTGCAAATGATTAGCCTGAGAGCTGAGGACACGGCTGTGTATTACTGTGCGAGAGATACGTATTACTATGGTTCGGGGAGAAGTAATGCTTTTGATATATGGGGCCAGGGAACCCTGGTCACCGTCTCGAGT | 262 |
| light chain (Lambda ) | SYELTQDPAVSVALGQTVRITCQGDSLRNFYASWYQQKSGQAPVLVMYGKNNRPSGIPDRFSGSTSGNTASLTITGAQAEDEADYYCNSRDSSGSHLTGVFGGGTKVTVLGQPKANPTVTLFPPSSEELQANKATLVCLISDFYPGAVTVAWKADGSPVKAGVETTKPSKQSNNKYAASSYLSLTPEQWKSHRSYSCQVTHEGSTVEKTVAPTECS | 347 |
| heavy chain | QVQLVESGGGLVQPGGSLRLSCAASGFTFSSYAMSWVRQAPGKGLEWVSAISGSGGSTYYADSVKGRFTISRDNSKNTLYLQMISLRAEDTAVYYCARDTYYYGSGRSNAFDIWGQGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK | 302 |

## Example 5: Generation of Human LILR-overexpressing cell lines

The nucleic acid sequences encoding the full-length human LILR family proteins (Table 27) were amplified by PCR, and each of the amplified sequences was inserted into an expression vector (pTRIOZ-hIgG4, InvivoGen; alternatively, any one of vectors comprising CMV promoter or CMV/CHO beta-actin fusion promoter (KR10-1038126B1) and genes encoding human IgG4 heavy chain constant region and lambda light chain constant region, can be used). The DNA sequence of the expression vector was confirmed by sequencing. The constructed vector was transfected into CHO cells, to generate 11 stable cell lines overexpressing each LILR protein on its surface.

[Table 27]

| Protein | Genbank Accession No. | amino acid sequence (N→C) | SEQ ID NO | Antibody |
|---------|----------------------|---------------------------|-----------|----------|
| LILRB1 | AAH15731 | MTPILTVLICLGLSLGPRTHVQAGHLPK PTLWAEPGSVITQGSPVTLRCQGGQE TQEYRLYREKKTAPWITRIPQELVKKG QFPIPSITWEHAGRYRCYYGSDTAGR SESSDPLELVVTGAYIKPTLSAQPSPVV NSGGNVTLQCDSQVAFDGFILCKEGE DEHPQCLNSQPHARGSSRAIFSVGPV SPSRRWWYRCYAYDSNSPYEWSLPS DLLELLVLGVSKKPSLSVQPGPIVAPEE TLTLQCGSDAGYNRFVLYKDGERDFL QLAGAQPQAGLSQANFTLGPVSRSYG GQYRCYGAHNLSSEWSAPSDPLDILIA GQFYDRVSLSVQPGPTVASGENVTLL CQSQGWMQTFLLTKEGAADDPWRLR STYQSQKYQAEFPMGPVTSAHAGTYR CYGSQSSKPYLLTHPSDPLELVVSGPS GGPSSPTTGPTSTSGPEDQPLTPTGS DPQSGLGRHLGVVIGILVAVILLLLLLL LFLILRHRRQGKHWTSTQRKADFQHP AGAVGPEPTDRGLQWRSSPAADAQE ENLYAAVKHTQPEDGVEMDTRSPHDE DPQAVTYAEVKHSRPRREMASPPSPL SGEFLDTKDRQAEEDRQMDTEAAASE APQDVTYAQLHSLTLRRKATEPPPSQE GPSPAVPSIYATLAIH | 348 | Human LILRB1 antibody (ab1857 96, Abcam) |

(continued)

| Protein | Genbank Accession No. | amino acid sequence (N→C) | SEQ ID NO | Antibody |
|---------|----------------------|---------------------------|-----------|----------|
| LILRB2 | AAH36827 | MTPIVTVLICLGLSLGPRTHVQTGTIPK PTLWAEPDSVITQGSPVTLSCQGSLEA QEYRLYREKKSASWITRIRPELVKNGQ FHIPSITWEHTGRYGCQYYSRARWSE LSDPLVLVMTGAYPKPTLSAQPSPVVT SGGRVTLQCESQVAFGGFILCKEGED EHPQCLNSQPHARGSSRAIFSVGPVS PNRRWSHRCYGYDLNSPYVWSSPSD LLELLVPGVSKKPSLSVQPGPVVAPGE SLTLQCVSDVGYDRFVLYKEGERDLR QLPGRQPQAGLSQANFTLGPVSRSYG GQYRCYGAYNLSSEWSAPSDPLDILIT GQIHGTPFISVQPGPTVASGENVTLLC QSWRQFHTFLLTKAGAADAPLRLRSIH EYPKYQAEFPMSPVTSAHAGTYRCYG SLNSDPYLLSHPSEPLELVVSGPSMGS SPPPTGPISTPAGPEDQPLTPTGSDPQ SGLGRHLGVVIGILVAVVLLLLLLLLFLI LRHRRQGKHWTSTQRKADFQHPAGA VGPEPTDRGLQWRSSPAADAQEENLY AAVKDTQPEDGVEMDTRAAASEAPQD VTYAQLHSLTLRRKATEPPPSQEGEPP AEPSIYATLAIH | 349 | Human LILRB2/ CD85d/I LT4 antibody (MAB20 78, R&D System s) |
| LILRB3 | XP_00672 6377 | MTPALTALLCLGLSLGPRTRVQAGPFP KPTLWAEPGSVISWGSPVTIWCQGSL EAQEYRLDKEGSPEPLDRNNPLEPKN KARFSIPSMTEHHAGRYRCHYYSSAG WSEPSDPLELVMTGFYNKPTLSALPSP VVASGGNMTLRCGSQKGYHHFVLMK EGEHQLPRTLDSQQLHSGGFQALFPV GPVNPSHRWRFTCYYYYMNTPQVWS HPSDPLEILPSGVSRKPSLLTLQGPVLA PGQSLTLQCGSDVGYDRFVLYKEGER DFLQRPGQQPQAGLSQANFTLGPVSP SHGGQYRCYGAHNLSSEWSAPSDPL | 350 | Human LILRB3/ CD85a/I LT5 antibody (MAB18 06, R&D System s) |

(continued)

| Protein | Genbank Accession No. | amino acid sequence (N→C) | SEQ ID NO | Antibod y |
|---|---|---|---|---|
| | | NILMAGQIYDTVSLSAQPGPTVASGEN VTLLCQSWWQFDTFLLTKEGAAHPPL RLRSMYGAHKYQAEFPMSPVTSAHA GTYRCYGSYSSNPHLLSFPSEPLELM VSGHSGGSSLPPTGPPSTPGLGRYLE VLIGVSVAFVLLLFLLLFLLLRRQRHSK HRTSDQRKTDFQRPAGAAETEPKDRG LLRRSSPAADVQEENLYAAVKDTQSED RVELDSQSPHDEDPQAVTYAPVKHSS PRREMASPPSSLSGEFLDTKDRQVEE DRQMDTEAAASEASQDVTYAQLHSLT LRRKATEPPPSQEGEPPAEPSIYATLAI H | | |
| LILRB4 | NP_00126 5355 | MIPTFTALLCLGLSLGPRTHMQAGPLP KPTLWAEPGSVISWGNSVTIWCQGTL EAREYRLDKEESPAPWDRQNPLEPKN KARFSIPSMTEDYAGRYRCYYRSPVG WSQPSDPLELVMTGAYSKPTLSALPS PLVTSGKSVTLLCQSRSPMDTFLLIKE RAAHPLLHLRSEHGAQQHQAEFPMSP VTSVHGGTYRCFSSHGFSHYLLSHPS DPLELIVSGSLEGPRPSPTRSVSTAAG PEDQPLMPTGSVPHSGLRRHWEVLIG VLVVSILLLSLLLFLLLQHWRQGKHRTL AQRQADFQRPPGAAEPEPKDGGLQR RSSPAADVQGENFCAAVKNTQPEDGV EMDTRQSPHDEDPQAVTYAKVKHSRP RREMASPPSPLSGEFLDTKDRQAEED RQMDTEAAASEAPQDVTYARLHSFTL RQKATEPPPSQEGASPAEPSVYATLAI H | 351 | Human LILRB4/ CD85k/l LT3 antibody (MAB24 251, R&D System s) |

(continued)

| Protein | Genbank Accession No. | amino acid sequence (N→C) | SEQ ID NO | Antibod y |
|---------|----------------------|---------------------------|-----------|-----------|
| LILRB5 | NP_00683 1 | MTLTLSVLICLGLSVGPRTCVQAGTLP KPTLWAEPASVIARGKPVTLWCQGPLE TEEYRLDKEGLPWARKRQNPLEPGAK AKFHIPSTVYDSAGRYRCYYETPAGW SEPSDPLELVATGFYAEPTLLALPSPVV ASGGNVTLQCDTLDGLLTFVLVEEEQK LPRTLYSQKLPKGPSQALFPVGPVTPS CRWRFRCYYYYRKNPQVWSNPSDLL EILVPGVSRKPSLLIPQGSVVARGGSLT LQCRSDVGYDIFVLYKEGEHDLVQGS GQQPQAGLSQANFTLGPVSRSHGGQ YRCYGAHNLSPRWSAPSDPLDILIAGLI PDIPALSVQPGPKVASGENVTLLCQSW HQIDTFFLTKEGAAHPPLCLKSKYQSY RHQAEFSMSPVTSAQGGTYRCYSAIR SYPYLLSSPSYPQELVVSGPSGDPSLS PTGSTPTPGPEDQPLTPGLDPQSGL GRHLGVVTGVSVAFVLLLFLLLFLLLRH RHQSKHRTSAHFYRPAGAAGPEPKDQ GLQKRASPVADIQEEILNAAVKDTQPK DGVEMDARAAASEAPQDVTYAQLHSL TLRREATEPPPSQEREPPAEPSIYAPL AIH | 352 | Human LILRB5/ CD85c/ LIR-8 antibody (MAB30 65, R&D System s) |
| LILRA1 | NP_00685 4 | MTPIVTVLICLRLSLGPRTHVQAGTLPK PTLWAEPGSVITQGSPVTLWCQGILET QEYRLYREKKTAPWITRIPQEIVKKGQF PIPSITWEHTGRYRCFYGSHTAGWSE PSDPLELVVTGAYIKPTLSALPSPVVTS GGNVTLHCVSQVAFGSFILCKEGEDE HPQCLNSQPRTHGWSRAIFSVGPVSP SRRWSYRCYAYDSNSPHVWSLPSDLL ELLVLGVSKKPSLSVQPGPIVAPGESLT LQCVSDVSYDRFVLYKEGERDFLQLP GPQPQAGLSQANFTLGPVSRSYGGQ YRCSGAYNLSSEWSAPSDPLDILIAGQ FRGRPFISVHPGPTVASGENVTLLCQS WGPFHTFLLTKAGAADAPLRLRSIHEY PKYQAEFPMSPVTSAHSGTYRCYGSL SSNPYLLSHPSDSLELMVSGAAETLSP PQNKSDSKAGAANTLSPSQNKTASHP QDYTVENLIRMGIAGLVLVVLGILLFEA QHSQRSL | 353 | Human LILRA1/ LILRB1 antibody (MAB30 851, R&D System s) |

(continued)

| Protein | Genbank Accession No. | amino acid sequence (N→C) | SEQ ID NO | Antibod y |
|---------|----------------------|---------------------------|-----------|-----------|
| LILRA2 | AAH17412 | MTPILTVLICLGLSLGPRTHVQAGHLPK PTLWAEPGSVIIQGSPVTLRCQGSLQA EEYHLYRENKSASWVRRIQEPGKNGQ FPIPSITWEHAGRYHCQYYSHNHSSEY SDPLELVVTGAYSKPTLSALPSPVVTL GGNVTLQCVSQVAFDGFILCKEGEDE HPQRLNSHSHARGWSWAIFSVGPVSP SRRWSYRCYAYDSNSPYVWSLPSDLL ELLVPGVSKKPSLSVQPGPMVAPGESL TLQCVSDVGYDRFVLYKEGERDFLQR PGWQPQAGLSQANFTLGPVSPSHGG QYRCYSAHNLSSEWSAPSDPLDILITG QFYDRPSLSVQPVPTVAPGKNVTLLC QSRGQFHTFLLTKEGAGHPPLHLRSE HQAQQNQAEFRMGPVTSAHVGTYRC YSSLSSNPYLLSLPSDPLELVVSASLG QHPQDYTVENLIRMGVAGLVLVVLGILL FEAQHSQRSLQDAAGR | 354 | Human LILRA2/ CD85h/l LT1 antibody (MAB63 64, R&D System s) |
| LILRA3 | AAH28208 | MTSILTVLICLGLSLDPRTHVQAGPLPK PTLWAEPGSVITQGSPVTLRCQGSLET QEYHLYREKKTALWITRIPQELVKKGQF PILSITWEHAGRYCCIYGSHTVGLSES SDPLELVVTGAYSKPTLSALPSPVVTS GGNVTIQCDSQVAFDGFILCKEGEDEH PQCLNSHSHARGSSRAIFSVGPVSPS RRWSYRCYGYDSRAPYVWSLPSDLL GLLVPGVSKKPSLSVQPGPVVAPGEKL TFQCGSDAGYDRFVLYKEWGRDFLQ RPGRQPQAGLSQANFTLGPVSRSYG GQYTCSGAYNLSSEWSAPSDPLDILIT GQIRARPFLSVRPGPTVASGENVTLLC QSQGGMHTFLLTKEGAADSPLRLKSK RQSHKYQAEFPMSPVTSAHAGTYRCY GSLSSNPYLLTHPSDPLELVVSGAAET LSPPQNKSDSKAGE | 355 | Human LILRA3/ CD85e antibody (PA5-47349, Invitrog en) |

(continued)

| Protein | Genbank Accession No. | amino acid sequence (N→C) | SEQ ID NO | Antibody |
|---|---|---|---|---|
| LILRA4 | NP_036408 | MTLILTSLLFFGLSLGPRTRVQAENLPK PILWAEPGPVITWHNPVTIWCQGTLEA QGYRLDKEGNSMSRHILKTLESENKV KLSIPSMMWEHAGRYHCYYQSPAGW SEPSDPLELVVTAYSRPTLSALPSPVVT SGVNVTLRCASRLGLGRFTLIEEGDHR LSWTLNSHQHNHGKFQALFPMGPLTF SNRGTFRCYGYENNTPYVWSEPSDPL QLLVSGVSRKPSLLTLQGPVVTPGENL TLQCGSDVGYIRYTLYKEGADGLPQR PGRQPQAGLSQANFTLSPVSRSYGG QYRCYGAHNVSSEWSAPSDPLDILIAG QISDRPSLSVQPGPTVTSGEKVTLLCQ SWDPMFTFLLTKEGAAHPPLRLRSMY GAHKYQAEFPMSPVTSAHAGTYRCY GSRSSNPYLLSHPSEPLELVVSGATET LNPAQKKSDSKTAPHLQDYTVENLIRM GVAGLVLLFLGILLFEAQHSQRSPPRC SQEANSRKDNAPFRVVEPWEQI | 356 | CD85g (ILT7) antibody (16-5179-82, Invitrog en) |
| LILRA5 | NP_067073 | MAPWSHPSAQLQPVGGDAVSPALMV LLCLGLSLGPRTHVQAGNLSKATLWAE PGSVISRGNSVTIRCQGTLEAQEYRLV KEGSPEPWDTQNPLEPKNKARFSIPS MTEHHAGRYRCYYYSPAGWSEPSDP LELVVTGFYNKPTLSALPSPVVTSGEN VTLQCGSRLRFDRFILTEEGDHKLSWT LDSQLTPSGQFQALFPVGPVTPSHRW MLRCYGSRRHILQVWSEPSDLLEIPVS GAADNLSPSQNKSDSGTASHLQDYAV ENLIRMGMAGLILVVLGILIFQDWHSQR SPQAAAGR | 357 | Human LILRA5/ CD85f antibody (MAB67 54, R&D System s) |

(continued)

| Protein | Genbank Accession No. | amino acid sequence (N→C) | SEQ ID NO | Antibod y |
|---|---|---|---|---|
| LILRA6 | NP_00134 7096 | MTPALTALLCLGLSLGPRTRVQAGPFP KPTLWAEPGSVISWGSPVTIWCQGSL EAQEYQLDKEGSPEPLDRNNPLEPKN KARFSIPSMTQHHAGRYRCHYYSSAG WSEPSDPLELVMTGFYNKPTLSALPSP VVASGGNMTLRCGSQKGYHHFVLMK EGEHQLPRTLDSQQLHSGGFQALFPV GPVTPSHRWRFTCYYYYTNTPRVWS HPSDPLEILPSGVSRKPSLLTLQGPVLA PGQSLTLQCGSDVGYDRFVLYKEGER DFLQRPGQQPQAGLSQANFTLGPVSP SHGGQYRCYGAHNLSSEWSAPSDPL NILMAGQIYDTVSLSAQPGPTVASGEN VTLLCQSRGYFDTFLLTKEGAAHPPLR LRSMYGAHKYQAEFPMSPVTSAHAGT YRCYGSYSSNPHLLSFPSEPLELMVS GHSGGSSLPPTGPPSTPASHAKDYTV ENLIRMGMAGLVLVFLGILLFEAQHSQ RNPQDAAGR | 358 | Human LILRA6/ CD85b antibody (MAB86 56, R&D System s) |

**Example 6: Determination of EC$_{50}$ for binding of the selected antibodies to LILRB1 overexpressing cell surface**

In order to determine EC$_{50}$ values of the antibodies prepared in Examples 1 and 4 for binding to human LILRB1-overexpressing cell lines, a cell surface binding assay was performed. Representing the prepared antibodies, EC$_{50}$ values of E3.1 and H11 antibodies were measured. The CHO cells prepared in Example 5, which overexpress LILRB1 on surface, were added to U-bottom 96-well tissue culture plate (BD Falcon) at the amount of $1 \times 10^5$ cells/well. Threefold serial dilutions of E3.1 and H11 antibodies were prepared starting from the final concentrations of 600 ug/mL and 27 ug/mL, respectively. The cells were treated with each of the diluted antibodies and incubated at 4°C for 60 minutes. After washing with FACS buffer, the cells were treated with anti-human Fc-biotin antibody (Invitrogen) and incubated at 4°C for 30 minutes. After washing with FACS buffer, the cells were treated with streptavidin (BD Pharmigen) labeled with PE fluorescence and incubated at 4°C for 30 minutes. After washing with FACS buffer, the cells were resuspended and subjected to analysis using iQue screener (Sartorius). EC$_{50}$ values were calculated using nonlinear regression formula of GraphPad Prism software, and the obtained results are shown in Table 28:

[Table 28]

|  | E3.1 | H11 |
|---|---|---|
| EC$_{50}$ (nM) | 7.154 | 0.376 |

**Example 7: Assessment of cross-reactivity of the selected antibodies to Human LILR family-overexpressing cell lines**

In order to confirm whether or not the selected antibodies bind to human LILR family proteins other than LILRB1, a cell surface binding assay was performed. The CHO cells (prepared in Example 5) expressing each of various LILR family proteins on surface were added to U-bottom 96-well tissue culture plate (BD Falcon) at the amount of $1 \times 10^5$ cells/well. The cells in each well were treated with the selected antibody in the final concentration of 20 ug/mL and incubated at 4°C for 60 minutes. After washing with FACS buffer, the cells were treated with anti-human Fc-biotin antibody (Invitrogen) and incubated at 4°C for 30 minutes. After washing with FACS buffer, the cells were treated with streptavidin (BD Pharmigen) labeled with PE or FITC fluorescence and incubated at 4°C for 30 minutes. After washing with FACS buffer, the cells were resuspended and subjected to analysis using iQue screener (Sartorius). The cells treated with each LILR protein specific antibody (Table 27) were used as a positive control, and the cells treated with human IgG4 isotype control antibody (Biolegend) were used as a negative control.

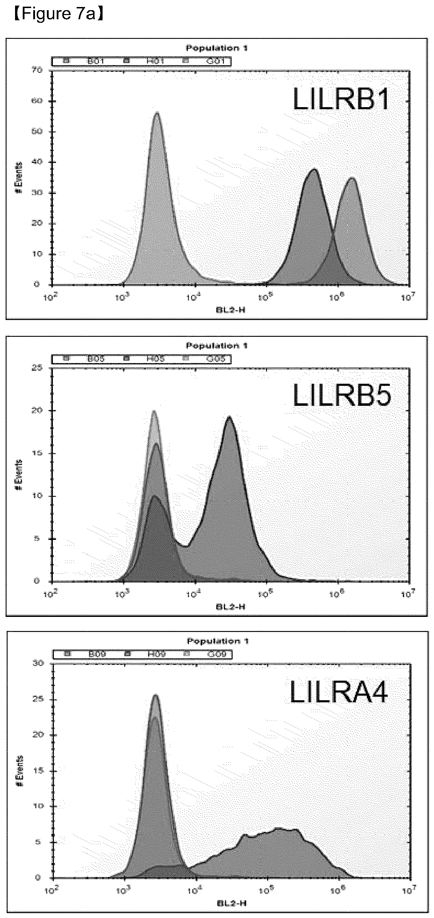

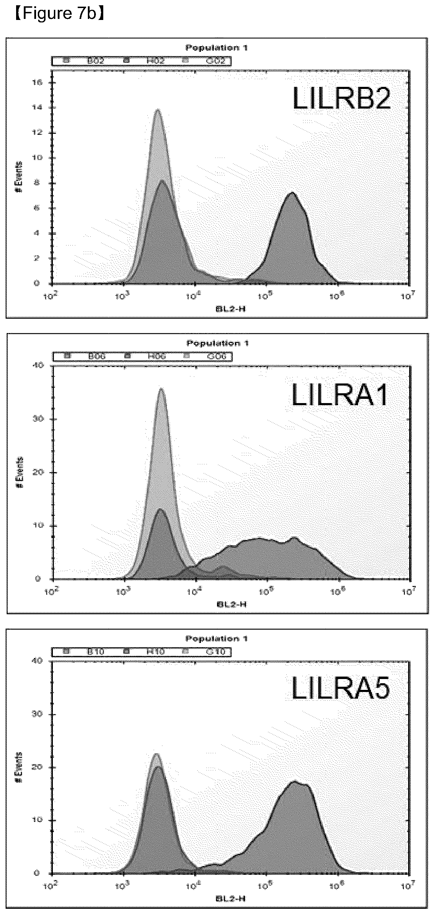

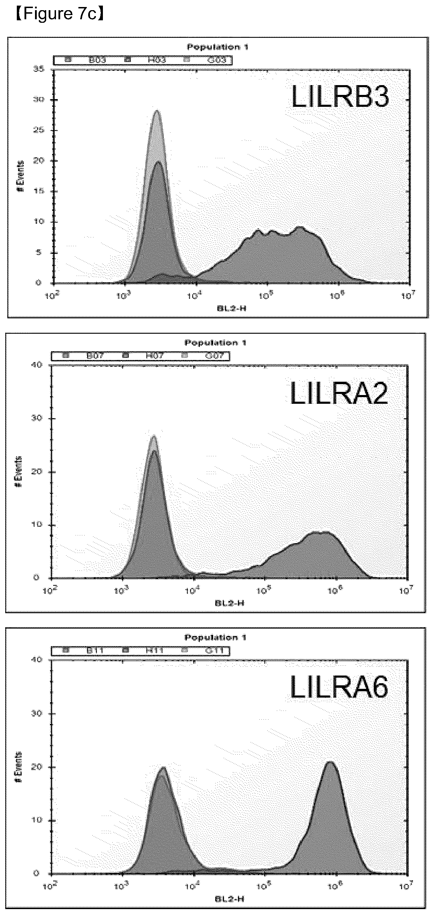

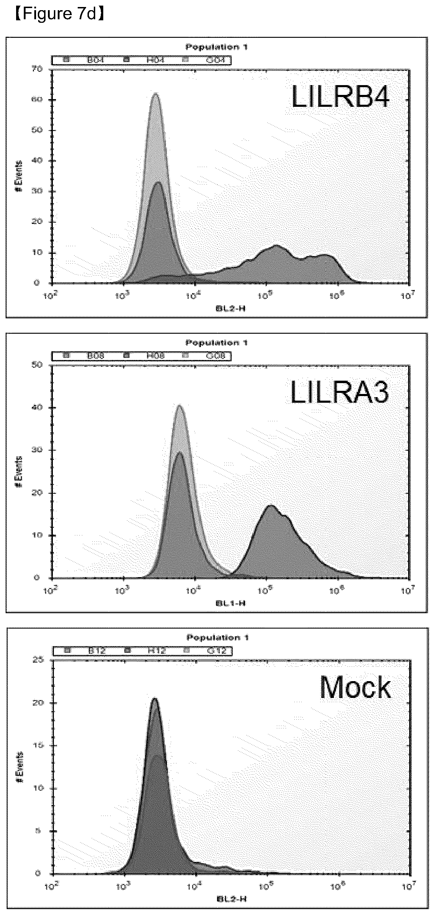

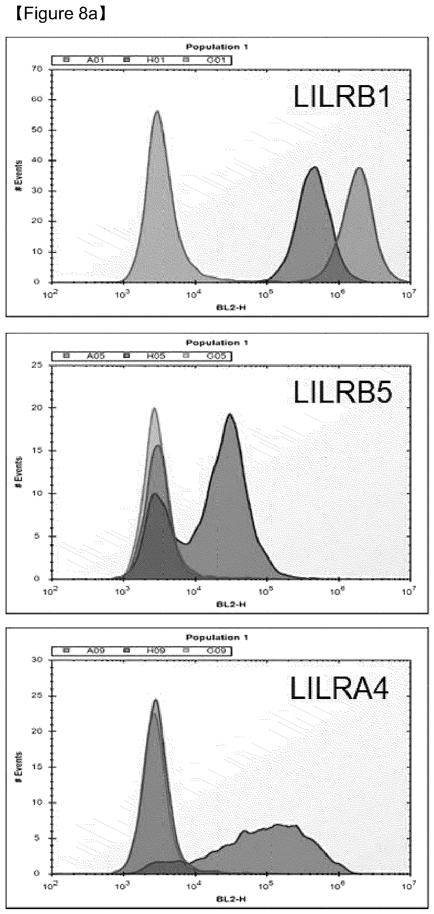

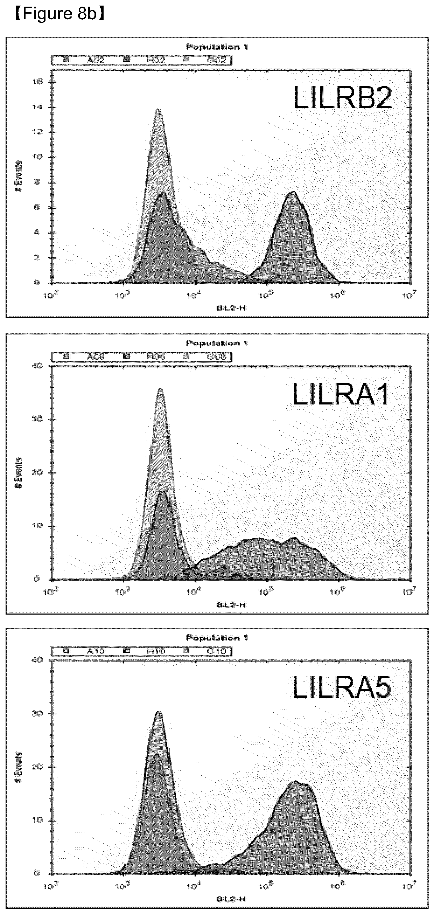

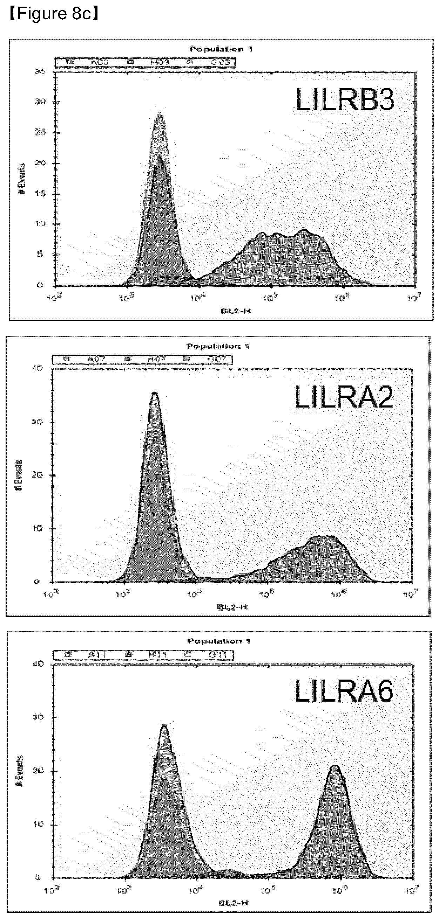

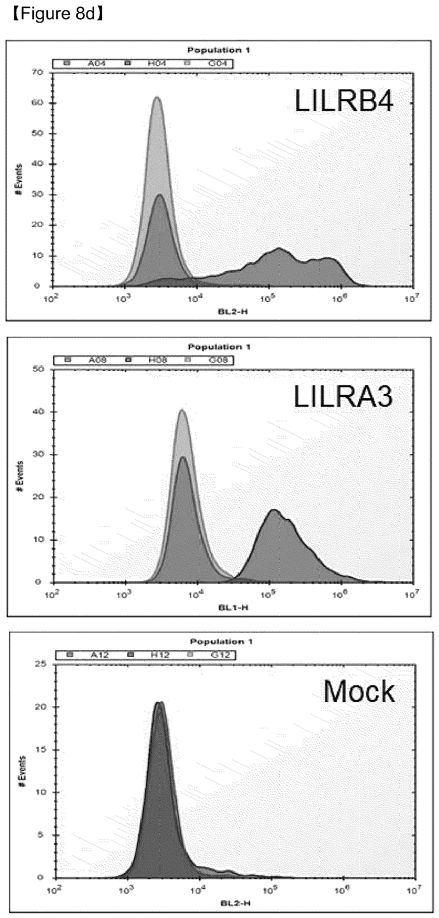

The results obtained for antibody E3.1 are shown in Figs. 7a to 7d (E3.1: red; LILR-specific antibody: blue; Isotype (hIgG4) control: gray), and the results obtained for antibody H11 are shown in Figs. 8a to 8d (H11: red; LILR-specific antibody: blue; Isotype (hIgG4) control: gray). As shown in Figs. 7a to 7d and Figs. 8a to 8d, the E3.1 and H11 antibodies do not bind at all or hardly bind to LILRs other than LILRB1. These results indicate that the antibodies provided by the examples have binding abilities specifically to LILRB1.

**Example 8: Measurement of release of granzyme B and perforin by enzyme-linked immune absorbent spot (ELISPOT) assay**

In order to confirm whether the E3.1 and H11 antibodies increase the level of cytotoxicity of NK cells, an enzyme-linked immune absorbent spot (ELISPOT) assay was performed. The level of cytotoxicity was determined by the release of cytotoxic granules, granzyme B and perforin, in NK cells.

$5 \times 10^3$ cells of LILRB1-expressing KHYG-1 cell lines (JCRB) and $5 \times 10^3$ cells of HLA-G-overexpressing K562 cells (which were prepared by transduction of K562 cells (American Type Culture Collection) with lentivirus constructed for expressing HLA-G) were co-cultured in U-bottom 96-well tissue culture plate. Each antibody(E3.1, H11 or human IgG4 isotype control antibody) was added thereto with final concentration of 50 ug/mL per each well and left incubated at 37°C for 30 minutes. The co-cultured cells were transferred onto 96 well plates (Immunospot, Cat. HGZBPFN-2M) (PVDF membrane) for ELISPOT, which were coated with antiperforin antibody and anti-granzyme B antibody, respectively, and further incubated at 37°C for 8 hours. The PVDF membranes were washed with a washing solution (0.05% tween 20 in PBS), then treated with anti-granzyme B-HRP and anti-perforin-biotin antibodies. Then, detection processes were performed according to the manufacturer's protocol. The PVDF membranes were dried at room temperature for 24 hours, and the number of spots for granzyme B and perforin were counted by ELISPOT analyzer (Immunospot).

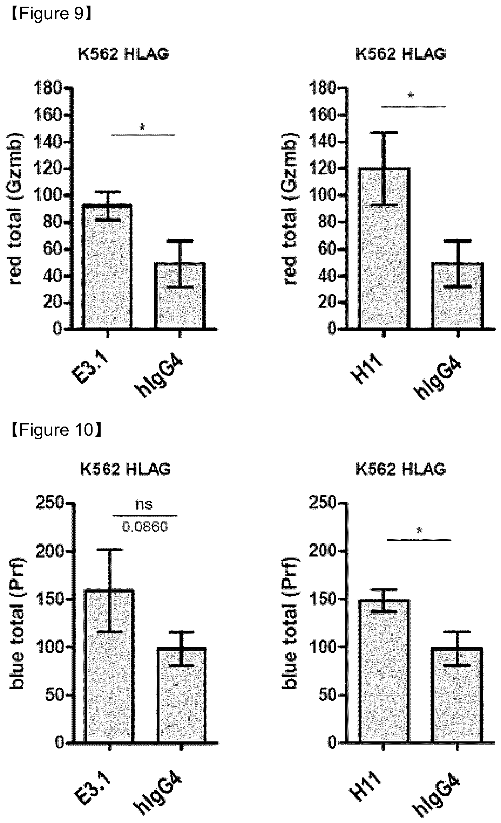

The results are shown in Fig 9 (granzyme b; Gzmb) and Fig. 10 (perforin; Prf), respectively (Y-axis indicates the total number of spots). As shown in Fig. 9 and Fig. 10, the release levels of both of granzyme B and perforin are significantly increased in E3.1 or H11 antibody-treated group, as compared with the human IgG4 isotype control antibody-treated group. Unpaired T-test was performed, and all experiments were performed three times under the same conditions for the reliability of the experiment, and the results are shown as average values.

**Example 9: preparation of Chimeric GHI/75 antibody**

In order to see if antibodies provided by the examples show higher efficacy compared to pre-existing antibodies, a chimeric GHI/75 antibody comprising a variable region of GHI/75 antibody (Biolegend, cat# 333721), which is a mousederived anti-human LILRB1 antibody, and a constant region of human antibody was prepared.

More specifically, the amino acid sequence of the GHI/75 antibody was analyzed through peptide mapping, and a vector, in which the nucleic acid sequence corresponding to the variable region (VH and VL domain) of a human IgG4 antibody was replaced by the nucleic acid sequence corresponding to the variable region (VH and VL domain) of mouse GHI/75 antibody, was prepared. In the vector, the region corresponding to upper hinge of human IgG4 was substituted with the nucleic acid sequence corresponding to the amino acid sequence (EPKSCDKTHT; SEQ ID NO: 359) of human IgG1 upper hinge. The vector was expressed as described in Example 1.4, and the obtained antibody was purified and used as a comparative antibody in examples below.

**Example 10: Measurement of inhibitory effect of the selected antibodies on LILRB1 signaling using IL-2 promoter luciferase assay**

In order to confirm whether the antibodies prepared in Examples 1 and 4 inhibit signaling by LILRB1, a luciferase reporter assay was performed. Among the antibodies prepared in Examples 1 and 4, the assay was performed representatively for E3.1 and H11 antibodies, and the chimeric GHI/75 antibody prepared in Example 9 was used for comparison. Jurkat cells expressing LILRB1 and interleukin 2 (IL-2) promoter luciferase (which were prepared by inserting IL-2 promoter luciferase vector (Promega) into Jurkat cell line (American Type Culture Collection) followed by transduction with lentivirus constructed for expressing LILRB1) and HLA-G-overexpressing K562 cells were used. Ninety six-well plates were coated with anti-CD3 antibody (Biolegend) by incubating with the antibody overnight at 4°C. On the next day, Jurkat cells expressing LILRB1 and IL-2 promoter luciferase were added to U-bottom 96-well plates at the amount of $1 \times 10^5$ cells/well, and the plates were treated with each antibody (E3.1, H11, chimeric GHI/75 or human IgG4 isotype (control)) to the final concentration of 20 ug/mL and incubated at 37°C for one hour. HLA-G-overexpressing K562 cells

($1\times10^5$ cells/well) were added to the plates and incubated at 37°C for 30 minutes. The obtained suspension was transferred to the plate coated with anti-CD3 antibody, and anti-CD28 antibody (Biolegend) was added thereto to the final concentration of 10 ug/mL. The plate was incubated at 37°C for 6 hours. Steady-Glo® solution (Promega) was added to each well, and the luminescence intensity was recorded using a luminometer (Envision, PerkinElmer).

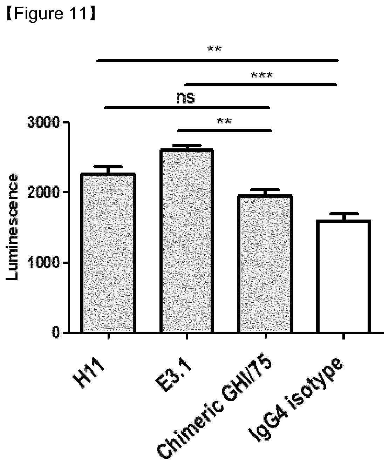

The results are shown in Fig. 11. As shown in Fig. 11, E3.1 and H11 antibodies provided in examples exhibit considerably increased LILRB1 signaling inhibitory activity compared to human IgG4 isotype control antibody and the chimeric GHI/75 control antibody.

**Example 11: Analysis of anti-cancer effect of selected antibodies in mouse model**

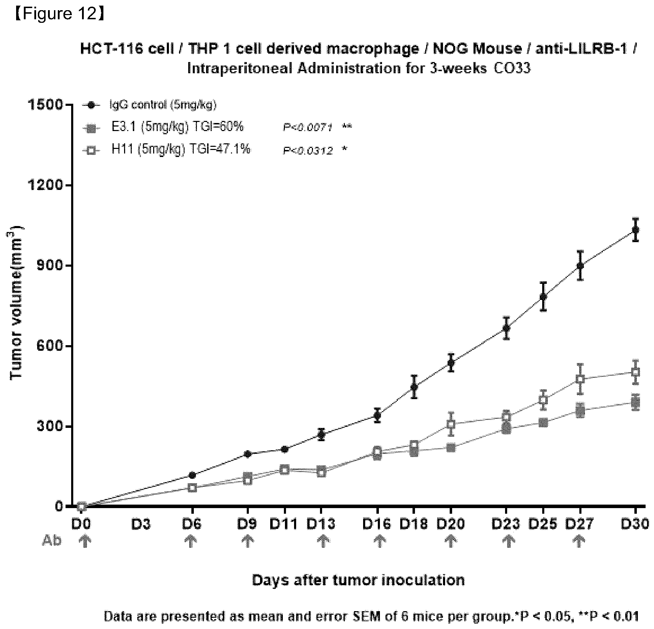

For analysis of anti-cancer effects of selected antibodies, referring to Example 3, a mixture of $3\times10^6$ cells of HCT116 Red-Fluc colorectal carcinoma cells, $3\times10^6$ cells of THP-1 derived macrophages and an antibody (20 $\mu$g/mouse) was subcutaneously injected to 5-week old female CIEA NOG mice (NOG immunodeficient mouse; Central Institute for Experimental Animals, Japan). The antibody used was E3.1 or H11 antibody, and human IgG4 isotype was used as a control antibody for comparison. From the 4th day after grafting tumor cells, the antibody was administered to the mouse model at the dosage of 5 mg/kg by intraperitoneal injection twice a week, and the tumor volume was measured and shown in Fig. 12. As shown in Fig. 12, E3.1 and H11 antibodies exhibit significant effect of inhibiting tumor growth in mouse models grafted with HCT116 colon cancer cells and THP-1 derived macrophages compared to the control antibody.

**Claims**

1. An anti-LILRB1 antibody or an antigen-binding fragment thereof, comprising complementarity determining regions (CDRs) as follows:

    a CDR-L1 comprising the amino acid sequence of SEQ ID NO: 1, 7, 13, 19, 25, 31, 37, 43, 49, 55, 61, 67, 73, 79, 85, 91, 97, 103, 109, or 115,
    a CDR-L2 comprising the amino acid sequence of SEQ ID NO: 2, 8, 14, 20, 26, 32, 38, 44, 50, 56, 62, 68, 74, 80, 86, 92, 98, 104, 110, or 116,
    a CDR-L3 comprising the amino acid sequence of SEQ ID NO: 3, 9, 15, 21, 27, 33, 39, 45, 51, 57, 63, 69, 75, 81, 87, 93, 99, 105, 111, or 117,
    a CDR-H1 comprising the amino acid sequence of SEQ ID NO: 4, 10, 16, 22, 28, 34, 40, 46, 52, 58, 64, 70, 76, 82, 88, 94, 100, 106, 112, or 118,
    a CDR-H2 comprising the amino acid sequence of SEQ ID NO: 5, 11, 17, 23, 29, 35, 41, 47, 53, 59, 65, 71, 77, 83, 89, 95, 101, 107, 113, or 119, and
    a CDR-H3 comprising the amino acid sequence of SEQ ID NO: 6, 12, 18, 24, 30, 36, 42, 48, 54, 60, 66, 72, 78, 84, 90, 96, 102, 108, 114, or 120,
    wherein the CDRs are defined based on Kabat numbering.

2. The anti-LILRB1 antibody or an antigen-binding fragment thereof of claim 1, comprising:

    (1) a CDR-L1 comprising the amino acid sequence of SEQ ID NO: 1, a CDR-L2 comprising the amino acid sequence of SEQ ID NO: 2, a CDR-L3 comprising the amino acid sequence of SEQ ID NO: 3, a CDR-H1 comprising the amino acid sequence of SEQ ID NO: 4, a CDR-H2 comprising the amino acid sequence of SEQ ID NO: 5, and a CDR-H3 comprising the amino acid sequence of SEQ ID NO: 6;
    (2) a CDR-L1 comprising the amino acid sequence of SEQ ID NO: 7, a CDR-L2 comprising the amino acid sequence of SEQ ID NO: 8, a CDR-L3 comprising the amino acid sequence of SEQ ID NO: 9, a CDR-H1 comprising the amino acid sequence of SEQ ID NO: 10, a CDR-H2 comprising the amino acid sequence of SEQ ID NO: 11, and a CDR-H3 comprising the amino acid sequence of SEQ ID NO: 12;
    (3) a CDR-L1 comprising the amino acid sequence of SEQ ID NO: 13, a CDR-L2 comprising the amino acid sequence of SEQ ID NO: 14, a CDR-L3 comprising the amino acid sequence of SEQ ID NO: 15, a CDR-H1 comprising the amino acid sequence of SEQ ID NO: 16, a CDR-H2 comprising the amino acid sequence of SEQ ID NO: 17, and a CDR-H3 comprising the amino acid sequence of SEQ ID NO: 18;
    (4) a CDR-L1 comprising the amino acid sequence of SEQ ID NO: 19, a CDR-L2 comprising the amino acid sequence of SEQ ID NO: 20, a CDR-L3 comprising the amino acid sequence of SEQ ID NO: 21, a CDR-H1 comprising the amino acid sequence of SEQ ID NO: 22, a CDR-H2 comprising the amino acid sequence of SEQ ID NO: 23, and a CDR-H3 comprising the amino acid sequence of SEQ ID NO: 24;

(5) a CDR-L1 comprising the amino acid sequence of SEQ ID NO: 25, a CDR-L2 comprising the amino acid sequence of SEQ ID NO: 26, a CDR-L3 comprising the amino acid sequence of SEQ ID NO: 27, a CDR-H1 comprising the amino acid sequence of SEQ ID NO: 28, a CDR-H2 comprising the amino acid sequence of SEQ ID NO: 29, and a CDR-H3 comprising the amino acid sequence of SEQ ID NO: 30;

(6) a CDR-L1 comprising the amino acid sequence of SEQ ID NO: 31, a CDR-L2 comprising the amino acid sequence of SEQ ID NO: 32, a CDR-L3 comprising the amino acid sequence of SEQ ID NO: 33, a CDR-H1 comprising the amino acid sequence of SEQ ID NO: 34, a CDR-H2 comprising the amino acid sequence of SEQ ID NO: 35, and a CDR-H3 comprising the amino acid sequence of SEQ ID NO: 36;

(7) a CDR-L1 comprising the amino acid sequence of SEQ ID NO: 37, a CDR-L2 comprising the amino acid sequence of SEQ ID NO: 38, a CDR-L3 comprising the amino acid sequence of SEQ ID NO: 39, a CDR-H1 comprising the amino acid sequence of SEQ ID NO: 40, a CDR-H2 comprising the amino acid sequence of SEQ ID NO: 41, and a CDR-H3 comprising the amino acid sequence of SEQ ID NO: 42;

(8) a CDR-L1 comprising the amino acid sequence of SEQ ID NO: 43, a CDR-L2 comprising the amino acid sequence of SEQ ID NO: 44, a CDR-L3 comprising the amino acid sequence of SEQ ID NO: 45, a CDR-H1 comprising the amino acid sequence of SEQ ID NO: 46, a CDR-H2 comprising the amino acid sequence of SEQ ID NO: 47, and a CDR-H3 comprising the amino acid sequence of SEQ ID NO: 48;

(9) a CDR-L1 comprising the amino acid sequence of SEQ ID NO: 49, a CDR-L2 comprising the amino acid sequence of SEQ ID NO: 50, a CDR-L3 comprising the amino acid sequence of SEQ ID NO: 51, a CDR-H1 comprising the amino acid sequence of SEQ ID NO: 52, a CDR-H2 comprising the amino acid sequence of SEQ ID NO: 53, and a CDR-H3 comprising the amino acid sequence of SEQ ID NO: 54;

(10) a CDR-L1 comprising the amino acid sequence of SEQ ID NO: 55, a CDR-L2 comprising the amino acid sequence of SEQ ID NO: 56, a CDR-L3 comprising the amino acid sequence of SEQ ID NO: 57, a CDR-H1 comprising the amino acid sequence of SEQ ID NO: 58, a CDR-H2 comprising the amino acid sequence of SEQ ID NO: 59, and a CDR-H3 comprising the amino acid sequence of SEQ ID NO: 60;

(11) a CDR-L1 comprising the amino acid sequence of SEQ ID NO: 61, a CDR-L2 comprising the amino acid sequence of SEQ ID NO: 62, a CDR-L3 comprising the amino acid sequence of SEQ ID NO: 63, a CDR-H1 comprising the amino acid sequence of SEQ ID NO: 64, a CDR-H2 comprising the amino acid sequence of SEQ ID NO: 65, and a CDR-H3 comprising the amino acid sequence of SEQ ID NO: 66;

(12) a CDR-L1 comprising the amino acid sequence of SEQ ID NO: 67, a CDR-L2 comprising the amino acid sequence of SEQ ID NO: 68, a CDR-L3 comprising the amino acid sequence of SEQ ID NO: 69, a CDR-H1 comprising the amino acid sequence of SEQ ID NO: 70, a CDR-H2 comprising the amino acid sequence of SEQ ID NO: 71, and a CDR-H3 comprising the amino acid sequence of SEQ ID NO: 72;

(13) a CDR-L1 comprising the amino acid sequence of SEQ ID NO: 73, a CDR-L2 comprising the amino acid sequence of SEQ ID NO: 74, a CDR-L3 comprising the amino acid sequence of SEQ ID NO: 75, a CDR-H1 comprising the amino acid sequence of SEQ ID NO: 76, a CDR-H2 comprising the amino acid sequence of SEQ ID NO: 77, and a CDR-H3 comprising the amino acid sequence of SEQ ID NO: 78;

(14) a CDR-L1 comprising the amino acid sequence of SEQ ID NO: 79, a CDR-L2 comprising the amino acid sequence of SEQ ID NO: 80, a CDR-L3 comprising the amino acid sequence of SEQ ID NO: 81, a CDR-H1 comprising the amino acid sequence of SEQ ID NO: 82, a CDR-H2 comprising the amino acid sequence of SEQ ID NO: 83, and a CDR-H3 comprising the amino acid sequence of SEQ ID NO: 84;

(15) a CDR-L1 comprising the amino acid sequence of SEQ ID NO: 85, a CDR-L2 comprising the amino acid sequence of SEQ ID NO: 86, a CDR-L3 comprising the amino acid sequence of SEQ ID NO: 87, a CDR-H1 comprising the amino acid sequence of SEQ ID NO: 88, a CDR-H2 comprising the amino acid sequence of SEQ ID NO: 89, and a CDR-H3 comprising the amino acid sequence of SEQ ID NO: 90;

(16) a CDR-L1 comprising the amino acid sequence of SEQ ID NO: 91, a CDR-L2 comprising the amino acid sequence of SEQ ID NO: 92, a CDR-L3 comprising the amino acid sequence of SEQ ID NO: 93, a CDR-H1 comprising the amino acid sequence of SEQ ID NO: 94, a CDR-H2 comprising the amino acid sequence of SEQ ID NO: 95, and a CDR-H3 comprising the amino acid sequence of SEQ ID NO: 96;

(17) a CDR-L1 comprising the amino acid sequence of SEQ ID NO: 97, a CDR-L2 comprising the amino acid sequence of SEQ ID NO: 98, a CDR-L3 comprising the amino acid sequence of SEQ ID NO: 99, a CDR-H1 comprising the amino acid sequence of SEQ ID NO: 100, a CDR-H2 comprising the amino acid sequence of SEQ ID NO: 101, and a CDR-H3 comprising the amino acid sequence of SEQ ID NO: 102;

(18) a CDR-L1 comprising the amino acid sequence of SEQ ID NO: 103, a CDR-L2 comprising the amino acid sequence of SEQ ID NO: 104, a CDR-L3 comprising the amino acid sequence of SEQ ID NO: 105, a CDR-H1 comprising the amino acid sequence of SEQ ID NO: 106, a CDR-H2 comprising the amino acid sequence of SEQ ID NO: 107, and a CDR-H3 comprising the amino acid sequence of SEQ ID NO: 108;

(19) a CDR-L1 comprising the amino acid sequence of SEQ ID NO: 109, a CDR-L2 comprising the amino acid sequence of SEQ ID NO: 110, a CDR-L3 comprising the amino acid sequence of SEQ ID NO: 111, a CDR-H1 comprising the amino acid sequence of SEQ ID NO: 112, a CDR-H2 comprising the amino acid sequence of SEQ ID NO: 113, and a CDR-H3 comprising the amino acid sequence of SEQ ID NO: 114; or

(20) a CDR-L1 comprising the amino acid sequence of SEQ ID NO: 115, a CDR-L2 comprising the amino acid sequence of SEQ ID NO: 116, a CDR-L3 comprising the amino acid sequence of SEQ ID NO: 117, a CDR-H1 comprising the amino acid sequence of SEQ ID NO: 118, a CDR-H2 comprising the amino acid sequence of SEQ ID NO: 119, and a CDR-H3 comprising the amino acid sequence of SEQ ID NO: 120.

3. The anti-LILRB1 antibody or an antigen-binding fragment thereof of claim 1, comprising:

a light chain variable region comprising the amino acid sequence of SEQ ID NO: 221, 223, 225, 227, 229, 231, 233, 235, 237, 239, 241, 243, 245, 247, 249, 251, 253, 255, 257, 259, or 345, and
a heavy chain variable region comprising the amino acid sequence of SEQ ID NO: 222, 224, 226, 228, 230, 232, 234, 236, 238, 240, 242, 244, 246, 248, 250, 252, 254, 256, 258, or 260.

4. The anti-LILRB1 antibody or an antigen-binding fragment thereof of claim 1, wherein the antibody is a human IgG1 or IgG4 antibody.

5. The anti-LILRB1 antibody or an antigen-binding fragment thereof of claim 1, wherein the antigen-binding fragment is scFv, (scFv)$_2$, Fab, Fab', F(ab')$_2$, a fusion polypeptide comprising scFv fused with an immunoglobulin Fc, or a fusion polypeptide comprising scFv fused with a constant region of a light chain.

6. A pharmaceutical composition for treating or preventing a cancer, comprising the anti-LILRB1 antibody or an antigen-binding fragment thereof of any one of claims 1 to 5, and a pharmaceutically acceptable carrier.

7. The pharmaceutical composition of claim 6, wherein the cancer is **characterized by** overexpression of MHC Class I.

8. A nucleic acid molecule encoding the antibody or an antigen-binding fragment thereof of any one of claims 1 to 5.

9. A recombinant vector comprising the nucleic acid molecule of claim 8.

10. A recombinant cell comprising the nucleic acid molecule of claim 8 or a recombinant vector comprising the nucleic acid molecule.

11. A method of preparing an anti-LILRB1 antibody or an antigen-binding fragment thereof, comprising culturing the recombinant cell of claim 10.

【Figure 1】

* 4-12% Gel, 1mm thick,
* Non-reducing

* 4-20% Gel, 1mm thick,
* Reducing

【Figure 2】

【Figure 3】

【Figure 4a】

【Figure 4b】

【Figure 4c】

【Figure 5】

【Figure 6】

HCT-116 cell / THP 1 cell derived macrophage / NOG Mouse / anti-LILRB-1 / Intraperitoneal Administration for 3-weeks

Data are presented as mean and error SEM of 6 mice per group. *P < 0.05, **P < 0.01

【Figure 7a】

【Figure 7b】

【Figure 7c】

【Figure 7d】

【Figure 8a】

【Figure 8b】

【Figure 8c】

【Figure 8d】

【Figure 9】

K562 HLAG

K562 HLAG

【Figure 10】

K562 HLAG

K562 HLAG

【Figure 11】

【Figure 12】

HCT-116 cell / THP 1 cell derived macrophage / NOG Mouse / anti-LILRB-1 / Intraperitoneal Administration for 3-weeks CO33

Data are presented as mean and error SEM of 6 mice per group.*$P < 0.05$, **$P < 0.01$

## INTERNATIONAL SEARCH REPORT

International application No.

**PCT/KR2020/018931**

| A. | CLASSIFICATION OF SUBJECT MATTER |
|---|---|

**C07K 16/28**(2006.01)i; **A61P 35/00**(2006.01)i; **A61K 39/00**(2006.01)i

According to International Patent Classification (IPC) or to both national classification and IPC

| B. | FIELDS SEARCHED |
|---|---|

Minimum documentation searched (classification system followed by classification symbols)

C07K 16/28(2006.01); A61K 35/15(2014.01); A61K 38/00(2006.01); A61K 38/18(2006.01); A61K 39/395(2006.01); A61P 11/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: LILRB1(Leukocyte immunoglobulin-like receptor subfamily B member 1), 항체 (antibody), 상보성 결정부위(CDR), 암(cancer)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2019-144052 A1 (ADANATE, INC.) 25 July 2019 (2019-07-25)<br>See abstract; and claims 1-23 and 61. | 1-11 |
| A | US 2015-0174203 A1 (ICAHN SCHOOL OF MEDICINE AT MOUNT SINAI et al.) 25 June 2015 (2015-06-25)<br>See claims 7-8 and 35-36; and paragraphs [0079] and [0088]-[0100]. | 1-11 |
| A | KIM, A. et al. LILRB1 Blockade Enhances Bispecific T Cell Engager Antibody-Induced Tumor Cell Killing by Effector CD8+ T Cells. The Journal of Immunology. electronic publication 28 June 2019, vol. 203, pp. 1076-1087.<br>See entire document. | 1-11 |
| A | US 2012-0315269 A1 (KLECHEVSKY, E. et al.) 13 December 2012 (2012-12-13)<br>See entire document. | 1-11 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 April 2021** | **02 April 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2020/018931**

**C.    DOCUMENTS CONSIDERED TO BE RELEVANT**

| Category* | Citation of document, with indication, where appropriate, of the relevant passages | Relevant to claim No. |
|---|---|---|
| A | VILLA-ALVAREZ, M. et al. Ig-Like Transcript 2 (ILT2) Blockade and Lenalidomide Restore NK Cell Function in Chronic Lymphocytic Leukemia. Frontiers in Immunology. 2018, vol. 9, thesis no. 2917, pp. 1-12. See entire document. | 1-11 |

Form PCT/ISA/210 (second sheet) (July 2019)

| **INTERNATIONAL SEARCH REPORT** | International application No. |
|---|---|
| | **PCT/KR2020/018931** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

    ☑    in the form of an Annex C/ST.25 text file.

    ☐    on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

    ☐    in the form of an Annex C/ST.25 text file (Rule 13*ter*.1(a)).

    ☐    on paper or in the form of an image file (Rule 13*ter*.1(b) and Administrative Instructions, Section 713).

2.  ☐    In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
Information on patent family members

International application No.

**PCT/KR2020/018931**

| Patent document cited in search report | | | Publication date (day/month/year) | Patent family member(s) | | | Publication date (day/month/year) |
|---|---|---|---|---|---|---|---|
| WO | 2019-144052 | A1 | 25 July 2019 | CN | 111867614 | A | 30 October 2020 |
| | | | | EP | 3740224 | A1 | 25 November 2020 |
| US | 2015-0174203 | A1 | 25 June 2015 | US | 2018-0177847 | A1 | 28 June 2018 |
| | | | | WO | 2013-181438 | A2 | 05 December 2013 |
| | | | | WO | 2013-181438 | A3 | 13 February 2014 |
| US | 2012-0315269 | A1 | 13 December 2012 | AR | 086287 | A1 | 04 December 2013 |
| | | | | TW | 201247700 | A | 01 December 2012 |
| | | | | WO | 2012-151578 | A1 | 08 November 2012 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190173414
- KR 1020200061907
- KR 101038126 B1

**Non-patent literature cited in the description**

- **KABAT, E.A. ; WU, T.T. ; PERRY, H. ; GOTTES-MAN, K. ; FOELLER, C.** Sequences of Proteins of Immunological Interest. 1991
- **STUDIER, F.W.** *Protein Expression and Purification,* 2005, vol. 41, 207-34